# EUROPÄISCHE PATENTSCHRIFT

(11) **EP 2 029 552 B1**
(45) Veröffentlichungstag und Bekanntmachung des Hinweises auf die Patenterteilung: **22.09.2010**
(21) Anmeldenummer: 07764565.3
(22) Anmeldetag: 16.05.2007
(51) Int. Cl.: C07D 239/48, C07D 401/12, A61K 31/506, A61P 35/00

(54) **SULFIMIDE ALS PROTEINKINASEINHIBITOREN**
SULFIMIDES AS PROTEIN KINASE INHIBITORS
SULFIMIDES COMME INHIBITEURS DE PROTÉINE KINASE

(30) Priorität: 08.06.2006 DE 102006027156; 19.06.2006 US 814525 P
(43) Veröffentlichungstag der Anmeldung: 04.03.2009
(73) Patentinhaber: Bayer Schering Pharma Aktiengesellschaft, 13353 Berlin (DE)
(72) Erfinder: LUECKING, Ulrich, 10245 Berlin (DE); SIEMEISTER, Gerd, 13503 Berlin (DE); BONIN VON, Arne, 16548 Glienicke-Nordbahn (DE); JAUTELAT, Rolf, 16548 Glienicke/Nordbahn (DE); AHSEN VON, Oliver, 10117 Berlin (DE); NGUYEN, Duy, 10179 Berlin (DE); DOECKE, Wolf-Dietrich, 10178 Berlin (DE)
(86) Internationale Anmeldenummer: PCT/EP2007/004913
(87) Internationale Veröffentlichungsnummer: WO 2007/140957

(56) Entgegenhaltungen:
- WO-A-2005/037800
- TOHRU UEDA AND JACK J FOX: "Nucleosides. XVII. Pyrimidinyl Amino Acids" J. MED. CHEM., Bd. 6, 1963, Seiten 697-700, XP002450119
- SIMON F CAMPELL AND RHONA M PLEWS: "2,4-Diamino-6,7,-dimethoxyquinazolines. 3. 2-(4-heterocyclylpiperazin-1-yl) derivatives as alpha1-adrenocepter antagonists and anthypertensive agents" J. MED. CHEM., Bd. 30, 1987, Seiten 1794-1798, XP002450120
- POLINA I SVIRSKAYA ET AL.: "Fluorinated heterocyclic compounds. 2. 2,4-difluoro and 4-amino-2-fluoropyrimidines, nucleoside base analogs" J. HETEROCYCL. CHEM., 1985, Seiten 149-153, XP002450121
- GIORGIO CARAVATTI ET AL.: "Structure based design of a non-peptidic antagonist of the SH2 domain of GRB2" BIOORG. MED. CHEM. LETT., Bd. 9, 1999, Seiten 1973-1978, XP002450122
- G.B. BARLIN: "Kinetics of reactions in heterocycles" J. CHEM. SOC. (B), 1967, Seiten 954-958, XP002450123
- DATABASE CA [Online] CHEMICAL ABSTRACTS SERVICE, COLUMBUS, OHIO, US; XP002450126 gefunden im STN Database accession no. 102:220513 & KONDRATENKO ET AL.: "S-trifluoromethyl-S-aryl-N-trifluoromethy lsulfonylsulfimides" ZHURNAL ORGANICHESKOI KHIMII, Bd. 20, Nr. 12, 1984, Seiten 2599-2604,
- KENNETH K ANDERSEN ET AL.: "Antimalarial sulfilimines and sulfoximines related to diaminodiphenyl sulfoxide and sulfone" J. MED. CHEM., Bd. 13, Nr. 4, 1970, Seiten 759-760, XP002450124

## Beschreibung

Die Erfindung betrifft Sulfimide als Proteinkinaseinhibitoren.

In eukaryontischen Zellen werden viele biologische Prozesse wie z.B. DNA-Replikation, Energiestoffwechsel, Zellwachstum oder -differenzierung durch reversible Phosphorylierung von Proteinen reguliert. Der Phosphorylierungsgrad eines Proteins hat unter anderem Einfluss auf die Funktion, Lokalisation oder Stabilität von Proteinen. Die Enzymfamilien der Proteinkinasen und Proteinphosphatasen sind verantwortlich für die Phosphorylierung bzw. Dephosphorylierung von Proteinen.

Über eine Inhibition von speziellen Proteinkinasen oder Proteinphosphatasen erhofft man sich in biologische Prozesse so eingreifen zu können, dass Krankheiten des menschlichen oder tierischen Körpers ursächlich oder symptomatisch behandelt werden können.

Im besonderen Interesse sind dabei Proteinkinasen, deren Inhibition die Behandlung von Krebs ermöglicht.

Folgende Proteinkinasefamilien kommen beispielsweise als Targets für inhibitorische Moleküle in Frage:
a) Zellzykluskinasen d.h. Kinasen, deren Aktivität den Ablauf des Zellteilungszyklus steuern. Zu den Zellzykluskinasen gehören im wesentlichen die Zyklin-abhängigen Kinasen (cyclin-dependent kinase: cdk), die polo-like Kinasen (Plk), und die Aurora Kinasen.
b) Rezeptortyrosinkinasen, die die Angiogenese regulieren (angiogene Rezeptortyrosinkinasen), wie beispielsweise die Rezeptortyrosinkinasen, die am Vascular Endothelial Growth Factor (VEGF) / VEGF-Rezeptor System, Fibroblast Growth Factor (FGF) / FGF-Rezeptor System, am Eph-Ligand / EphB4 System, und am Tie-Ligand / Tie System beteiligt sind,
c) Rezeptortyrosinkinasen, deren Aktivität zur Proliferation von Zellen beiträgt (proliferative Rezeptortyrosinkinasen), wie beispielsweise Rezeptortyrosinkinasen, die am Platelet-Derived Growth Factor (PDGF) Ligand /PDGF-Rezeptor System, c-Kit Ligand / c-Kit-Rezeptor System und am FMS-like Tyrosinkinase 3 (Flt-3) Ligand / Flt-3 System beteiligt sind,
d) Kontrollpunktkinasen (Checkpoint-Kinasen), die den geordneten Ablauf der Zellteilung überwachen, wie beispielsweise ATM und ATR, Chk1 und Chk2, Mps1, Bub1 und BubR1,
e) Kinasen, deren Aktivität die Zelle vor Apoptose schützen (anti-apoptotische Kinasen, Kinasen in sog. survival pathways, anti-apoptotische Kinasen), wie beispielsweise Akt/PKB, PDK1, IkappaB kinase (IKK), PIM 1, und integrin-linked kinase (ILK),
f) Kinasen, die für die Migration von Tumorzellen notwendig sind (migratorische Kinasen), wie beispielsweise focal adhesion kinase (FAK), und Rho kinase (ROCK).

Die Inhibierung einer oder mehrerer dieser Proteinkinasen eröffnet die Möglichkeit, das Tumorwachstum zu hemmen.

Dabei besteht vor allem ein Bedarf an Strukturen, die neben der Inhibierung von Zellzykluskinasen das Tumorwachstum durch die Inhibierung einer oder mehrerer weiterer Kinasen hemmen (Multi-target Tumor Growth Inhibitoren = MTGI). Bevorzugt ist vor allem die zusätzliche Inhibierung von Rezeptortyrosinkinasen, die die Angiogenese regulieren.

Den strukturell nahe liegenden Stand der Technik bilden die Strukturen folgender Patentanmeldungen:
WO 2002/096888 offenbart Anilino-Pyrimidinderivate als Inhibitoren der Zyklin-abhängigen Kinasen. Sulfimidsubstituenten sind für das Anilin nicht offenbart.
WO 2004/026881 offenbart makrozyklische Anilino-Pyrimidinderivate als Inhibitoren der Zyklin-abhängigen Kinasen. Ein für das Anilin möglicher Sulfimidsubstituent ist nicht offenbart.

WO 2005/037800 offenbart offene Anilino-Pyrimidinderivate als Inhibitoren der Zyklin-abhängigen Kinasen. Sulfimidsubstituenten sind für das Anilin nicht offenbart.

Allen diesen Strukturen des Standes der Technik ist gemeinsam, dass sie Zellzykluskinasen inhibieren.

Ausgehend von diesem Stand der Technik ist die Aufgabe der vorliegenden Erfindung, eine neue Klasse von Proteinkinaseinhibitoren bereitzustellen.

Im besonderen besteht die Aufgabe der vorliegenden Erfindung, Inhibitoren von Proteinkinasen bereitzustellen, über die ein Tumorwachstum gehemmt werden kann.

Vor allem besteht ein Bedarf an einer neuen Strukturklasse, die neben Zellzykluskinasen auch Rezeptortyrosinkinasen inhibiert, die die Angiogenese hemmen.

Die Aufgabe der vorliegenden Anmeldung wird gelöst durch Verbindungen der allgemeinen Formel (I), 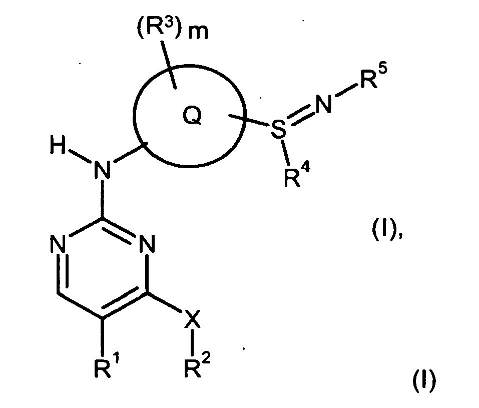 in der
- R¹: für
(i) Wasserstoff, Halogen, Cyano, Nitro, -NR⁸R⁹, -NR⁷-C(O)-R¹², -NR⁷-C(O)-OR¹², -NR⁷-C(O)-NR⁸R⁹, -NR⁷-SO₂-R¹², -CF₃ oder -OCF₃, oder
(ii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, C₁-C₆-Alkoxy, -CF₃ und/oder -OCF₃ ein- oder mehrfach, gleich oder verschieden substituierten C₁-C₆-Alkyl- C₂-C₆-Alkenyl-, C₁-C₆-Alkoxy- oder C₂-C₆-Alkinylrest, oder
(iii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, -CF₃, C₁-C₆-Alkoxy, -OCF₃ und/oder C₁-C₆-Alkyl ein- oder mehrfach, gleich oder verschieden substituierten Phenyl- oder monocyclischen Heteroarylring steht,
- R²: für
(i) Wasserstoff oder
(ii) einen C₁-C₁₀-Alkyl-, C₂-C₁₀-Alkenyl- oder C₂-C₁₀-Alkinylrest, einen C₃-C₇-Cycloalkyl-, Phenyl- oder Naphthylring, einen Heterocyclylring mit 3 bis 8 Ringatomen oder einen mono- oder bicyclischen Heteroarylring steht, jeweils gegebenenfalls ein- oder mehrfach, gleich oder verschieden substituiert mit
   a) Halogen, Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, -C(O)R⁶,-O(CO)-R¹², -SO₂NR⁸R⁹, -SO₂-R¹², -S(O)(NR⁸)R¹², -(N)S(O)R¹³R¹⁴, -CF₃, -OCF₃, -N[(CO)-(C₁-C₆-Alkyl)]₂ und/oder
   b) C₁-C₆-Alkoxy, C₁-C₆-Alkyl, C₂-C₆-Alkenyl, C₂-C₆-Alkinyl, C₃- C₈-Cycloalkyl, Phenyl, Naphthyl, Heterocyclyl mit 3 bis 8 Ringatomen und/oder einem monocyclischen oder bicyclischen Heteroaryl, jeweils gegebenenfalls selbst ein- oder mehrfach, gleich oder verschieden mit Halogen, Hydroxy, einem C₁-C₆-Alkyl, C₁-C₆-Alkoxy, -NR⁸R⁹, -C(O)OR¹⁶, -SO₂NR⁸R⁹, -CF₃ oder -OCF₃ substituiert,
- R³ für: (i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₆-Alkyl- und/oder C₁-C₆- Alkoxyrest, und/oder
(iii) einen gegebenenfalls mit Halogen, Hydroxy, C₁-C₆-Alkoxy, -CF₃, -OCF₃, -NR⁸R⁹ und/oder C₁-C₆-Alkyl ein- oder mehrfach, gleich oder verschieden substituierten C₃-C₇-Cycloalkylring steht,
- m: für 0-4 steht,
- R⁴: für einen C₁-C₆-Alkyl-, C₂-C₆-Alkenyl-, C₂-C₆-Alkinylrest, einen C₃-C₇- Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert,
oder
- R³ und R⁴: gemeinsam einen an Q annelierten 5 bis 7-gliedrigen Ring bilden, der gegebenenfalls ein- oder mehrfach, gleich oder verschieden mit Hydroxy, C₁-C₆-Alkyl, C₁-C₆-Alkoxy, Halogen oder -NR⁸R⁹ substituiert ist und gegebenenfalls zusätzlich zur Doppelbindung aus Q eine weitere Doppelbindung enthält, wenn der Ring 5-gliedrig ist
- R⁵: für
-SO₂-(CH₂)ₙ-R¹² mit n gleich 0 oder 1,
-C(O)R¹², -C(O)OR¹², -C(O)NR⁸R⁹, -C(S)OR¹², -C(S)NR⁸R⁹ oder
-R¹² steht,
oder
- R⁴ und R⁵: gemeinsam einen 5 bis 7-gliedrigen Ring der Formel 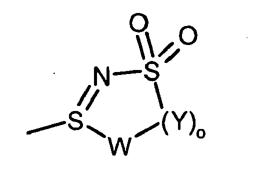 oder 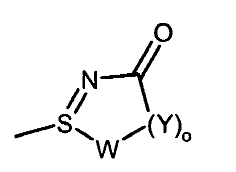 bilden,
in denen
W und Y jeweils unabhängig voneinander für eine gegebenenfalls ein- oder mehrfach, gleich oder verschieden mit Hydroxy, C₁-C₆- Alkyl, C₁-C₆-Alkoxy oder -NR⁸R⁹ substituierte
-CH₂-Gruppe stehen, wobei
der C₁-C₆-Alkyl- und/oder C₁-C₆-Alkoxysubstituent gegebenenfalls selbst ein oder mehrfach, gleich oder verschieden mit Hydroxy, C₁-C₆-Alkoxy oder -NR⁸R⁹ substituiert ist und/oder gegebenenfalls zusätzlich zur Imid-Doppelbindung 1 oder 2 weitere Doppelbindungen enthält
und
in denen
o 1-3 bedeutet
- X: für -O-, -S- oder -NR¹⁵- steht,
wobei,
R¹⁵ für
(i) Wasserstoff oder
(ii) einen C₁-C₆-Alkylrest, C₃-C₈-Cycloalkyl- oder Phenylring, einen Heterocyclylring mit 3 bis 8 Ringatomen oder einen monocyclischen Heteroarylring, oder
(iii) -C(O)-(C₁-C₆)-Alkyl, -C(O)-Phenyl, oder -C(O)-Benzyl steht, und (ii) und (iii) gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,
oder
wenn X für -NR¹⁵- steht, alternativ
X, R¹⁵ und R² gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 bis 3 Doppelbindungen enthält und/oder gegebenenfalls durch eine oder mehrere -C(O)-Gruppen unterbrochen ist,
- Q: für einen Phenyl ring steht,
- R⁶: für
(i) Wasserstoff oder Hydroxy, oder
(ii) einen C₁-C₆-Alkyl-, C₃-C₆-Alkenyl-, C₃-C₆-Alkinyl- oder C₁-C₆- Alkoxyrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆- Alkoxy und/oder -OCF₃ ein- oder mehrfach, gleich oder verschieden substituiert,
- R⁷: für Wasserstoff oder einen C₁-C₆-Alkylrest steht,
- R⁸ und R⁹: unabhängig voneinander für
(i) Wasserstoff und/oder
(ii) einen C₁-C₆-Alkylrest, C₂-C₆-Alkenylrest, C₃-C₈-Cycloalkyl- und/oder Phenylring, einen Heterocyclylring mit 3 bis 8 Ringatomen und/oder einen monocyclischen Heteroarylring, gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,
oder
- R⁸ und R⁹: gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, -NR¹⁰R¹¹, Cyano, Halogen, -CF₃, C₁-C₆-Alkoxy und/oder -OCF₃ ein- oder mehrfach, gleich oder verschieden substituiert sein kann,
- R¹⁰ und R¹¹: unabhängig voneinander für Wasserstoff oder einen C₁-C₆-Alkylrest stehen, der gegebenenfalls mit Hydroxy, Cyano, Halogen, -CF₃, C₁-C₆-Alkoxy und/oder -OCF₃ ein- oder mehrfach, gleich oder verschieden substituiert ist.
- R¹², R¹³ R¹⁴: unabhängig voneinander für -CF₃
oder
einen C₁-C₆-Alkyl, C₂-C₆-Alkenyl- und/oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht, die gegebenenfalls jeweils selbst mit Hydroxy, Nitro, -NR⁸R⁹, -NH-C(O)-C₁-C₆-Alkyl ,Cyano, Halogen, -CF₃, C₁-C₆-Alkyl, C₁-C₆- Alkoxy und/oder -OCF₃ ein- oder mehrfach, gleich oder verschieden substituiert sind,
- R¹⁶: für
(i) Wasserstoff oder
(ii) einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
sowie deren Salze, Diastereomere und Enantiomere.

Kein Dokument des Standes der Technik schlägt Sulfimidsubstituenten an Proteinkinasen inhibierende Anilino-Pyrimidinderivaten vor. Auch sind Sulfimidsubstituenten nicht für andere Strukturklassen offenbart, die Proteinkinasen inhibieren.

Der Erfindung liegen folgende Definitionen zu Grunde:

Cₙ-Alkyl:
Monovalenter, geradkettiger oder verzweigter, gesättigter Kohlenwasserstoffrest mit n Kohlenstoffatomen.

Ein C₁-C₆ Alkylrest umfasst unter anderem beispielsweise:
Methyl-, Ethyl-, Propyl-, Butyl-, Pentyl-,Hexyl-, *iso*-Propyl-, *iso*-Butyl-, *sec*-Butyl, *tert-*Butyl-, *iso*-Pentyl-, 2-Methylbutyl-, 1-Methylbutyl-, 1-Ethylpropyl-, 1,2-Dimethylpropyl, *neo*-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.

Bevorzugt ist ein Methyl-, Ethyl-, Propyl- oder Isopropylrest.

Cₙ-Alkenyl:
monovalenter, geradkettiger oder verzweigter Kohlenwasserstoffrest mit n Kohlenstoffatomen und mindestens einer Doppelbindung.

Ein C₂-C₁₀ Alkenylrest umfasst unter anderem beispielsweise:
Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-, (Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

CₙAlkinyl:
Monovalenter, geradkettiger oder verzweigter Kohlenwasserstoffrest mit n Kohlenstoffatomen und mindestens einer Dreifachbindung.

Ein C₂-C₁₀ Alkinylrest umfasst unter anderem beispielsweise:
Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-.

Bevorzugt ist ein Ethinyl-, Prop-1-inyl- oder Prop-2-inyl-rest.

Cₙ-Cycloalkyl:
Monovalenter, cyclischer Kohlenwasserstoffring mit n Kohlenstoffatomen.

C₃-C₇-Cyclolalkylring umfasst:
Cyclopropyl, Cyclobutyl, Cyclopentyl, Cyclohexyl- und Cycloheptyl.

Bevorzugt ist ein Cyclopropyl-, Cylopentyl- oder ein Cyclohexylring.

Cₙ-Alkoxy:
Geradkettiger oder verzweigter Cₙ-Alkyletherrest der Formel -OR mit R=Alkyl.

### Cₙ-Aryl

Cₙ-Aryl ist ein monovalentes, aromatisches Ringsystem ohne Heteroatom mit n Kohlenstoffatomen.

C₆-Aryl ist gleich Phenyl. C₁₀-Aryl ist gleich Naphthyl.

Bevorzugt ist Phenyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

Ein monocyclischer Heteroarylring gemäß der vorliegenden Erfindung hat 5 oder 6 Ringatome.

Heteroarylringe mit 5 Ringatomen umfassen beispielsweise die Ringe:
Thienyl, Thiazolyl, Furanyl, Pyrrolyl, Oxazolyl, Imidazolyl, Pyrazolyl, Isoxazolyl, Isothiazolyl, Oxadiazolyl, Triazolyl, Tetrazolyl- und Thiadiazolyl.

Heteroarylringe mit 6 Ringatomen umfassen beispielsweise die Ringe:
Pyridyl, Pyridazinyl, Pyrimidinyl, Pyrazinyl und Triazinyl.

Ein bicyclischer Heteroarylring gemäß der vorliegenden Erfindung hat 9 oder 10 Ringatome.

Heteroarylringe mit 9 Ringatomen umfassen beispielsweise die Ringe:
Phthalidyl-, Thiophthalidyl-, Indolyl-, Isoindolyl-, Indazolyl-, Benzothiazolyl-, Indolonyl-, Isoindolonyl-, Benzofuranyl, Benzothienyl, Benzimidazolyl, Benzoxazolyl, Azocinyl, indolizinyl, Purinyl.

Heteroarylringe mit 10 Ringatomen umfassen beispielsweise die Ringe:
Isoquinolinyl-, Quinolinyl-, Benzoxazinonyl-, Phthalazinonyl, Quinolonyl-, Isoquinolonyl-, Quinazolinyl-, Quinoxalinyl-, Cinnolinyl-, Phthalazinyl-, 1,7- or 1,8-Naphthyridinyl-, Quinolinyl-, Isoquinolinyl-, Quinazolinyl- oder Quinoxalinyl-Monocyclische Heteroarylringe mit 5 oder 6 Ringatomen sind bevorzugt.

### Heterocyclyl

Heterocyclyl im Sinne der Erfindung ist ein vollständig hydriertes Heteroaryl (vollständig hydriertes Heteroaryl = gesättigtes Heterocyclyl) d.h. ein nicht aromatisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein. Heterocyclylring mit 3 Ringatomen umfasst beispielsweise:
Aziridinyl.
Heterocyclylring mit 4 Ringatomen umfasst beispielsweise:
Azetidinyl, Oxetanyl.
Heterocyclylringe mit 5 Ringatomen umfassen beispielsweise die Ringe:
Pyrrolidinyl, Imidazolidinyl, Pyrazolidinyl und Tetrahydrofuranyl.
Heterocyclylringe mit 6 Ringatomen umfassen beispielsweise die Ringe:
Piperidinyl, Piperazinyl, Morpholinyl, Tetrahydropyranyl und Thiomorpholinyl
Heterocyclylring mit 7 Ringatomen umfasst beispielsweise:
Azepanyl, Oxepanyl, [1,3]-Diazepanyl, [1,4]-Diazepanyl.
Heterocyclylring mit 8 Ringatomen umfasst beispielsweise:
Oxocanyl, Azocanyl

### Halogen

Die Bezeichnung Halogen umfasst Fluor, Chlor, Brom und lod.

Bevorzugt ist Brom.

Eine bevorzugte Untergruppe sind Verbindungen der allgemeinen Formel (I), in der
- R¹: für Halogen, -CF₃, -OCF₃, C₁-C₄-Alkyl oder Nitro steht,
- R²: für einen C₁-C₁₀-Alkyl-, C₂-C₁₀-Alkenyl- oder C₂-C₁₀-Alkinylrest, einen C₃-C₇-Cycloalkyl-, Phenyl- oder einen mono- oder bicyclischen Heteroarylring oder einen Heterocyclylring mit 3 bis 7 Ringatomen steht,
jeweils gegebenenfalls ein oder mehrfach, gleich oder verschieden substituiert mit Hydroxy, -NR⁸R⁹, -NR⁷-C(O)-R¹² und/oder einem C₁- C₄-Alkylrest, der gegebenenfalls selbst ein- oder mehrfach substituiert ist mit Hydroxy
- R³: für
(i) Hydroxy, Halogen, Cyano, Nitro, -CF₃, -OCF₃, , -NR⁸R⁹, -NR⁷- C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₃-Alkyl- und/oder C₁-C₃- Alkoxyrest steht,
- m: für 0 oder 1 steht,
- R⁴: für einen C₁-C₅-Alkylrest, einen C₃-C₆-Cycloalkyl- oder einen Phenylring steht,
jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert
oder
- R³ und R⁴: gemeinsam einen an Q annelierten 5-gliedrigen Ring bilden, der gegebenenfalls zusätzlich zur Doppelbindung aus Q eine weitere Doppelbindung enthält.
- R⁵: für -SO₂-(CH₂)ₙ-R¹² mit n gleich 0 oder 1 steht,
wobei R¹² für CF₃
oder einen C₁-C₄-Alkylrest, einen C₃-C₆-Cycloalkyl- oder Phenylring oder einen Heterocyclylring mit 3 bis 6 Ringatomen oder einen monocyclischen Heteroarylring steht,
gegebenenfalls jeweils selbst mit Hydroxy, Nitro, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert,
oder
- R⁴ und R⁵: gemeinsam einen 5-gliedrigen Ring der Formel (1) bilden, 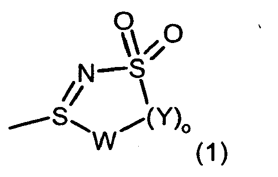 in der W und Y jeweils für eine -CH₂-Gruppe stehen und in der o 1 bedeutet.
- X: für -O-, -S- oder -NR¹⁵- steht,
wobei,
R¹⁵ für
(i) Wasserstoff oder
(ii) einen C₁-C₆-Alkylrest, C₃-C₈-Cycloalkyl- oder Phenylring, einen Heterocyclylring mit 3 bis 8 Ringatomen oder einen monocyclischen Heteroarylring, oder
(iii) -C(O)-(C₁-C₆)-Alkyl, -C(O)-Phenyl, oder -C(O)-Benzyl steht, und (ii) und (iii) gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,
   oder
   wenn X für -NR¹⁵- steht, alternativ
   X, R¹⁵ und R² gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 bis 3 Doppelbindungen enthält und/oder gegebenenfalls durch eine oder mehrere -C(O)- Gruppen unterbrochen ist,
- Q: für einen Phenyl ring steht,
- R⁶: für einen C₂-C₅-Alkyl-, C₄-C₆-Alkenyl-, C₄-C₆-Alkinyl- oder C₂-C₅- Alkoxyrest, einen C₄-C₆-Cycloalkyl- oder Phenylring, einen Heterocyclylring mit 3 bis 5 Ringatomen oder einen monocyclischen Heteroarylring steht,
jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert
- R⁷: für Wasserstoff oder einen C₁-C₆-Alkylrest steht,
- R⁸ und R⁹: jeweils unabhängig voneinander für Wasserstoff und/oder einen C₁-C₄- Alkylrest, C₃-C₆-Cycloalkyl- und/oder Phenylring,und/oder einen monocyclischen Heteroarylring,
jeweils gegebenenfalls mit Hydroxy, -NR¹⁰R¹¹ oder C₁-C₆-Alkoxy ein- oder mehrfach, gleich oder verschieden substituiert, oder
- R⁸ und R⁹: gemeinsam mit dem Stickstoffatom einen 5- bis 7-gliedrigen Ring bilden, der gegebenenfalls zusätzlich zum Stickstoffatom 1 weiteres Heteroatom enthält und der mit Hydroxy ein- oder mehrfach substituiert sein kann,
- R¹⁰ und R¹¹: unabhängig voneinander für Wasserstoff oder einen C₁-C₆-Alkylrest stehen, der gegebenenfalls mit Hydroxy ein- oder mehrfach, gleich oder verschieden substituiert ist.
- R¹²: für einen C₁-C₆-Alkyl, C₂-C₆-Alkenyl- oder C₂-C₆-Alkinylrest, einen C₃- C₇-Cycloalkyl- oder Phenylring, einen Heterocyclylring mit 3 bis 8 Ringatomen oder einen monocyclischen Heteroarylring steht,
jeweils gegebenenfalls selbst mit Hydroxy, Halogen, Nitro, -NR⁸R⁹, C₁-C₆-Alkyl ,und/oder C₁-C₆-Alkoxy ein- oder mehrfach, gleich oder verschieden substituiert,
- R¹³ und R¹⁴: unabhängig voneinander für einen C₁-C₆-Alkylrest stehen, und
- R¹⁶: für einen C₁-C₆-Alkylrest, einen C₃-C₇-Cycloalkyl- oder Phenylring, einen Heterocyclylring mit 3 bis 8 Ringatomen oder einen monocyclischen Heteroarylring steht,
sowie deren Salze, Diastereomere und Enantiomere.

Von der bevorzugten Untergruppe sind Verbindungen von besonderem Interesse, in denen in Forme(I)
- R⁴: für einen C₁-C₅-Alkylrest oder einen C₃-C₆-Cycloalkylring steht, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert
oder
- R³ und R⁴: gemeinsam einen an Q annelierten 5-gliedrigen Ring bilden, der gegebenenfalls zusätzlich zur Doppelbindung aus Q eine weitere Doppelbindung enthält.
- R⁵: für -SO₂R¹² steht,
wobei R¹² für einen C₁-C₄-Alkylrest, einen C₃-C₆-Cycloalkyl- oder Phenylring oder einen Heterocyclylring mit 3 bis 6 Ringatomen oder einen monocyclischen Heteroarylring steht,
gegebenenfalls jeweils selbst mit Hydroxy, Nitro, -NR⁸R⁹, Cyano, Halogen, -CF₃, C₁-C₆-Alkyl , C₁-C₆-Alkoxy und/oder -OCF₃ ein- oder mehrfach, gleich oder verschieden substituiert,
oder
- R⁴ und R⁵: gemeinsam einen 5-gliedrigen Ring der Formel (1) bilden, 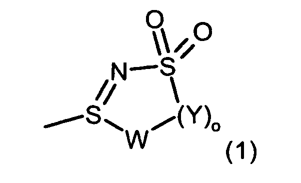 in der W und Y jeweils für eine -CH₂-Gruppe stehen und in der o 1 bedeutet,
sowie deren Salze, Diastereomere und Enantiomere.

Eine mehr bevorzugte Untergruppe sind Verbindungen der allgemeinen Formel (I), in der
- R¹: für Wasserstoff, Halogen oder -CF₃
- R²: für einen C₁-C₁₀-Alkyrest-, einen C₃-C₇-Cycloalkyl- oder Phenylring steht, jeweils gegebenenfalls ein- oder mehrfach, gleich oder verschieden substituiert mit Hydroxy oder -NH-C(O)-C₁-C₆-Alkyl
- m: für 0 steht,
- R⁴: für einen C₁-C₆-Alkylrest steht,
- R⁵: für -SO₂-(CH₂)ₙ-R¹² mit n gleich 0 oder 1 steht,
- X: für -NH- steht,
- Q: für einen Phenylring steht,
- R¹²: für -CF₃
oder
für einen C₁-C₆-Alkylrest, einen Phenyl- oder einen monocyclischen Heteroarylring steht, die gegebenenfalls jeweils selbst mit Nitro, Halogen, -CF₃, C₁-C₆-Alkyl , -NH-C(O)-C₁-C₆-Alkyl , C₁-C₆-Alkoxy und/oder -OCF₃ ein- oder mehrfach, gleich oder verschieden substituiert sind,
sowie deren Salze, Diastereomere und Enantiomere.

In der allgemeinen Formel (I) steht Q für einen Phenyl ring.
Bevorzugt steht Q für einen Phenyl- oder einen monocyclischen Heteroarylring Mehr bevorzugt steht Q für einen Phenyl- oder einen monocyclischen Heteroarylring mit 6 Ringatomen, insbesondere für einen Pyridylring.
Besonders bevorzugt steht Q für einen Phenylring.

In der allgemeinen Formel (I) kann R¹ stehen für:
(i) Wasserstoff, Halogen, Cyano, Nitro, -NR⁸R⁹, -NR⁷-C(O)-R¹², -NR⁷-C(O)-OR¹², -NR⁷-C(O)-NR⁸R⁹, -NR⁷-SO₂-R¹², -CF₃ oder -OCF₃, oder
(ii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl-, C₁-C₆-Alkoxy- oder C₂-C₆-Alkinylrest, oder
(iii) einen gegebenenfalls mit Hydroxy, -NR⁸R⁹, -NR⁷-C(O)₋R¹², -NR⁷-C(O)-OR¹², -NR⁷-C(O)-NR⁸R⁹, -NR⁷-SO₂-R¹², Cyano, Halogen, -CF₃, C₁-C₆-Alkoxy, -OCF₃ und/oder C₁-C₆-Alkyl ein- oder mehrfach, gleich oder verschieden substituierten Phenyl- oder monocyclischen Heteroarylring steht,

Bevorzugt steht R¹ für:
Halogen, -CF₃, -OCF₃, C₁-C₄-Alkyl oder Nitro.
Mehr bevorzugt steht R¹ für Halogen, -CF₃ oder C₁-C₂-Alkyl.
Noch mehr bevorzugt steht R¹ für Brom und CF₃.

In der allgemeinen Formel (I) kann R² stehen für:
(i) Wasserstoff oder
(ii) einen C₁-C₁₀-Alkyl-, C₂-C₁₀-Alkenyl- oder C₂-C₁₀-Alkinylrest, einen C₃-C₇-Cycloalkyl-, Phenyl- oder Naphthylring, einen Heterocyclylring mit 3 bis 8 Ringatomen oder einen mono- oder bicyclischen Heteroarylring steht, jeweils gegebenenfalls ein- oder mehrfach, gleich oder verschieden substituiert mit
   a) Halogen, Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, -C(O)R⁶, -O(CO)-R¹², -SO₂NR⁸R⁹, -SO₂-R¹², -S(O)(NR⁸)R¹², -(N)S(O)R¹³R¹⁴, -CF₃, -OCF₃, -N[(CO)-(C₁-C₆-Alkyl)]₂ und/oder
   b) C₁-C₆-Alkoxy, C₁-C₆-Alkyl, C₂-C₆-Alkenyl, C₂-C₆-Alkinyl, C₃-C₈-Cycloalkyl, Phenyl, Naphthyl, Heterocyclyl mit 3 bis 8 Ringatomen und/oder einem monocyclischen oder bicyclischen Heteroaryl, jeweils gegebenenfalls selbst ein- oder mehrfach, gleich oder verschieden mit Halogen, Hydroxy, einem C₁-C₆-Alkyl, C₁-C₆-Alkoxy, -NR⁸R⁹, -C(O)OR¹⁶, -SO₂NR⁸R⁹, -CF₃ oder -OCF₃ substituiert,

Bevorzugt steht R² für:
einen C₁-C₁₀-Alkyl-, C₂-C₁₀-Alkenyl- oder C₂-C₁₀-Alkinylrest, einen C₃-C₇-Cycloalkyl-, Phenyl- oder einen mono- oder bicyclischen Heteroarylring, einen Heterocyclylring mit 3 bis 7 Ringatomen, jeweils gegebenenfalls ein oder mehrfach, gleich oder verschieden substitutiert mit Hydroxy, -NR⁸R⁹, -NR⁷-C(O)-R¹² und/oder einem C₁-C₄-Alkylrest, der gegebenenfalls selbst ein- oder mehrfach substituiert ist mit Hydroxy.

Mehr bevorzugt steht R² für:
einen C₂-C₆-Alkyl-, C₂-C₈-Alkenyl- oder C₂-C₈-Alkinylrest, einen C₃-C₆-Cycloalkyl-, Phenylring, einen monocyclischen Heteroarylring mit 6 Ringatomen, einen
Heterocyclylring mit 5 bis 7 Ringatomen, jeweils gegebenenfalls ein oder mehrfach, gleich oder verschieden substitutiert mit Hydroxy, -NR⁸R⁹, -NR⁷-C(O)-R¹² und/oder einem C₁-C₄-Alkylrest, der gegebenenfalls selbst ein- oder mehrfach substituiert ist mit Hydroxy.

Besonders bevorzugt steht R² für:
einen C₁-C₆-Alkylrest, einen C₃-C₇-Cycloalkyl- oder Phenylring, jeweils gegebenenfalls ein oder mehrfach, gleich oder verschieden substituiert mit Hydroxy und/oder -NH-C(O)-C₁-C₆-Alkyl.

In der allgemeinen Formel (I) kann X stehen für:
-O-, -S- oder -NR¹⁵-,
wobei,
R¹⁵ steht für
   (i) Wasserstoff oder
   (ii) einen C₁-C₆-Alkylrest, C₃-C₈-Cycloalkyl- oder Phenylring, einen Heterocyclylring mit 3 bis 8 Ringatomen oder einen monocyclischen Heteroarylring, oder
   (iii) -C(O)-(C₁-C₆)-Alkyl, -C(O)-Phenyl, oder -C(O)-Benzyl,
      wobei (ii) und (iii) gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,
oder
wenn X für -NR¹⁵- steht, alternativ
   - X, R¹⁵ und R²: gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 bis 3 Doppelbindungen enthält und/oder gegebenenfalls durch eine oder mehrere -C(O)-Gruppen unterbrochen ist.

Bevorzugt steht X für :
-O-, -S- oder -NR¹⁵-, wobei
R¹⁵ für Wasserstoff oder einen C₁-C₆-Alkylrest, C₃-C₈-Cycloalkyl- oder einen Heterocyclylring mit 3 bis 8 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder -OCF₃,
oder
wenn X für -NR¹⁵- steht,
   - X, R¹⁵ und R²: bevorzugt alternativ gemeinsam einen 3 bis 6 gliedrigen Ring bilden, der gegebenenfalls zusätzlich zum Stickstoffatom ein weiteres Heteroatom enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 oder 2 Doppelbindungen enthält und/oder durch eine -C(O)-Gruppe unterbrochen ist.
Mehr bevorzugt steht X für -NR¹⁵-
wobei,
R¹⁵ für Wasserstoff oder einen C₃-C₆-Alkylrest, C₃-C₇-Cycloalkyl- oder einen Heterocyclylring mit 3 bis 6 Ringatomen steht, jeweils gegebenenfalls ein- oder mehrfach, gleich oder verschieden substituiert mit Hydroxy, -NR¹⁰R¹¹, Cyano, Halogen, -CF₃, C₁-C₆-Alkoxy und/oder -OCF₃,
oder
wenn X für -NR¹⁵- steht,
   - X, R¹⁵ und R²: mehr bevorzugt alternativ gemeinsam einen 5 oder 6 gliedrigen Ring, der gegebenenfalls zusätzlich zum Stickstoffatom ein weiteres Heteroatom enthält und der gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist.

Besonders bevorzugt steht X für -NR¹⁵-, wobei R¹⁵ für Wasserstoff steht.

In der allgemeinen Formel (I) kann R³ stehen für:
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₆-Alkyl- und/oder C₁-C₆-Alkoxyrest, und/oder
(iii) einen gegebenenfalls mit Halogen, Hydroxy, C₁-C₆-Alkoxy, -CF₃,-OCF_{3,}-NR⁸R⁹ und/oder C₁-C₆-Alkyl ein- oder mehrfach, gleich oder verschieden substituierten C₃-C₇-Cycloalkylring

Bevorzugt steht R³ für:
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹²,
-NR⁷-C(O)-OR¹², -NR⁷-C(O)-NR⁸R⁹, -NR⁷-SO₂-R¹², und/oder (ii) einen gegebenenfalls mit Halogen, Hydroxy, C₁-C₆-Alkoxy,
-CF₃, -OCF₃ oder -NR⁸R⁹ ein- oder mehrfach, gleich oder verschieden substituierten C₁-C₅-Alkyl- und/oder C₁-C₅-Alkoxyrest,

Mehr bevorzugt steht R³ für
(i) Hydroxy, Halogen, Cyano, Nitro, -CF₃, -OCF₃, , -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₃-Alkyl- und/oder C₁-C₃-Alkoxyrest

Noch mehr bevorzugt steht R³ für:
(i) Hydroxy, Halogen, Cyano, Nitro, -CF₃, -OCF₃,-NR⁸R⁹ und/oder
(ii) einen C₁-C₃-Alkyl- und/oder C₁-C₃-Alkoxyrest

Besonders bevorzugt steht R³ für:
Hydroxy, Fluor, Chlor, Brom, Cyano, Nitro, -CF₃, Methyl oder Methoxy.

In der allgemeinen Formel (I) kann m stehen für:
0-4, bevorzugt für 0 oder 1 , mehr bevorzugt für 0.

In der allgemeinen Formel (I) kann R⁴ stehen für:
einen C₁-C₆-Alkyl-, C₂-C₆-Alkenyl-, C₂-C₆-Alkinylrest, einen C₃-C₇-Cycloalkyl- oder
Phenylring, einen Heterocyclylring mit 3 bis 8 Ringatomen oder einen monocyclischen Heteroarylring,
jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert,
oder
R³ und R⁴ bilden gemeinsam einen an Q annelierten 5 bis 7-gliedrigen Ring, der gegebenenfalls ein- oder mehrfach, gleich oder verschieden mit Hydroxy, C₁-C₆-Alkyl, C₁-C₆-Alkoxy, Halogen oder -NR⁸R⁹ substituiert ist und gegebenenfalls zusätzlich zur Doppelbindung aus Q eine weitere Doppelbindung enthält, wenn der Ring 5-gliedrig ist.

Bevorzugt steht R⁴ für:
einen C₁-C₆-Alkyl-, C₂-C₆-Alkenyl-, C₂-C₆-Alkinylrest, einen C₃-C₇-Cycloalkyl- oder Phenylring, einen Heterocyclylring mit 3 bis 8 Ringatomen oder einen monocyclischen Heteroarylring,
jeweils gegebenenfalls selbst mit Hydroxy, -NR⁸R⁹, Halogen, ein- oder mehrfach, gleich oder verschieden substituiert,
oder
R³ und R⁴
bilden bevorzugt gemeinsam einen an Q annelierten 5 bis 7-gliedrigen Ring, der gegebenenfalls ein- oder mehrfach, gleich oder verschieden mit Hydroxy, Halogen oder -NR⁸R⁹ substituiert ist und gegebenenfalls eine Doppelbindung enthält, wenn der Ring 5-gliedrig ist.

Noch mehr bevorzugt steht R⁴ für einen C₁-C₅-Alkylrest, einen C₃-C₆-Cycloalkyl- oder einen Phenylring, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert
oder
R³ und R⁴ bilden bevorzugt gemeinsam einen an Q annelierten 5-gliedrigen Ring, der gegebenenfalls zusätzlich zur Doppelbindung aus Q eine weitere Doppelbindung enthält.

Besonders bevorzugt steht R⁴ für einen C₁-C₄-Alkylrest, einen C₃-C₅-Cycloalkyl- oder einen Phenylring, jeweils gegebenenfalls selbst mit Hydroxy, -NR⁸R⁹ oder Halogen ein- oder mehrfach, gleich oder verschieden substituiert.

Ganz besonders bevorzugt steht R⁴ für einen C₁-C₃-Alkylrest.

In der allgemeinen Formel (I) kann R⁵ stehen für:
-SO₂-(CH₂)ₙ-R¹² mit n gleich 0 oder 1, -C(O)R¹²,
-C(O)OR¹², -C(O)NR⁸R⁹, -C(S)OR¹², -C(S)NR⁸R⁹ oder-R¹²
   - wobei R¹²: für -CF₃ oder für einen C₁-C₆-Alkyl-, C₂-C₆-Alkenyl- und/oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht, gegebenenfalls jeweils selbst mit Hydroxy, Nitro, -NR⁸R⁹, -NH-C(O)-C₁-C₆-Alkyl, Cyano, Halogen, -CF₃, C₁-C₆-Alkyl, C₁-C₆- Alkoxy und/oder -OCF₃ ein- oder mehrfach, gleich oder verschieden substituiert.

Bevorzugt steht R⁵ für :
-SO₂R¹², -C(O)R¹², -C(O)OR¹², -C(O)NR⁸R⁹, -C(S)OR¹², -C(S)NR⁸R⁹ oder
-R¹²
wobei
   - R¹²: für einen C₁-C₆-Alkyl, C₂-C₆-Alkenyl- und/oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht, gegebenenfalls jeweils selbst mit Hydroxy, Nitro, -NR⁸R⁹, Cyano, Halogen, -CF₃, C₁-C₆-Alkyl , C₁-C₆-Alkoxy und/oder -OCF₃ ein- oder mehrfach, gleich oder verschieden substituiert,
oder
R⁴ und R⁵
bilden gemeinsam einen 5 bis 7-gliedrigen Ring der Formel (1) 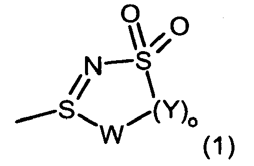
oder der Formel (2) 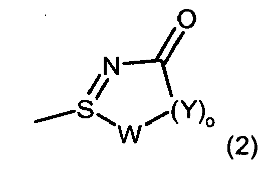
in denen
W und Y jeweils unabhängig voneinander für eine gegebenenfalls ein- oder mehrfach, gleich oder verschieden mit Hydroxy, C₁-C₆-Alkyl, C₁-C₆-Alkoxy oder -NR⁸R⁹ substituierte -CH₂-Gruppe stehen,
wobei
der C₁-C₆-Alkyl- und/oder C₁-C₆-Alkoxysubstituent gegebenenfalls selbst ein oder mehrfach, gleich oder verschieden mit Hydroxy, C₁-C₆-Alkoxy oder -NR⁸R⁹ substituiert ist und/oder gegebenenfalls zusätzlich zur Imid-Doppelbindung 1 oder 2 weitere Doppelbindungen enthält
und
in denen o 1-3 bedeutet

Auch bevorzugt steht R⁵ für -SO₂R¹² oder -C(O)R¹²,
- wobei R¹²: für einen C₁-C₅-Alkyl, C₂-C₅-Alkenyl- und/oder C₂-C₅-Alkinylrest, einen C₃-C₇-Cycloalkyl- oder Phenylring , einen Heterocyclylring mit 3 bis 6 Ringatomen und/oder einen monocyclischen Heteroarylring steht,
gegebenenfalls jeweils selbst mit Hydroxy, Nitro, -NR⁸R⁹, Cyano, Halogen, -CF₃, C₁-C₆-Alkyl , C₁-C₆-Alkoxy und/oder -OCF₃ ein- oder mehrfach, gleich oder verschieden substituiert,
oder
R⁴ und R⁵
bilden bevorzugt gemeinsam einen 5 bis 7-gliedrigen Ring der Formel (1) 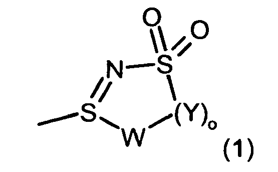
oder der Formel (2) 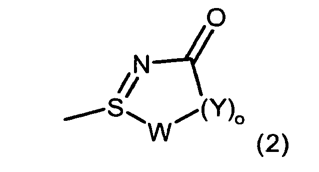
in denen
W und Y jeweils unabhängig voneinander für eine gegebenenfalls ein- oder mehrfach, gleich oder verschieden mit Hydroxy, C₁-C₆-Alkyl, C₁-C₆-Alkoxy oder -NR⁸R⁹ substituierte -CH₂-Gruppe stehen
und
in denen
o 1-3 bedeutet

Mehr bevorzugt steht R⁵ für -SO₂-(CH₂)ₙ-R¹² mit n gleich 0 oder 1
- wobei R¹²: für -CF₃
oder
für einen C₁-C₆-Alkylrest , einen Phenyl- oder einen monocyclischen Heteroarylring steht, die gegebenenfalls jeweils selbst mit Nitro, -NH-C(O)-C₁-C₆-Alkyl , Halogen, C₁-C₆-Alkyl, C₁-C₆-Alkoxy und/oder -OCF₃ ein- oder mehrfach, gleich oder verschieden substituiert sind.

Ebenso mehr bevorzugt steht R⁵ für -SO₂R¹²,
- wobei R¹²: für -CF₃
oder
für einen C₁-C₄-Alkylrest, einen C₃-C₆-Cycloalkyl- oder Phenylring oder einen Heterocyclylring mit 3 bis 6 Ringatomen oder einen monocyclischen Heteroarylring , gegebenenfalls jeweils selbst mit Hydroxy, Nitro, -NR⁸R⁹, Cyano, Halogen, -CF₃, C₁-C₆-Alkyl , C₁-C₆- Alkoxy und/oder -OCF₃ ein- oder mehrfach, gleich oder verschieden substituiert,
oder
R⁴ und R⁵
bilden bevorzugt gemeinsam einen 5-gliedrigen Ring der Formel (1) 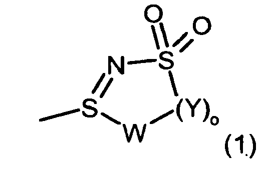
in der W und Y jeweils für eine -CH₂-Gruppe stehen und in der o 1 bedeutet.

Besonders bevorzugt steht R⁵ für -SO₂-(CH₂)ₙ-R¹² mit n gleich 0 oder 1
- wobei R¹²: für -CF₃
oder
für einen C₁-C₆-Alkylrest oder einen Phenyl-, Pyridyl-, Thienyl- oder Thiadiazolylring steht, die gegebenenfalls jeweils selbst mit Nitro, -NH-C(O)-C₁-C₆-Alkyl , Halogen, C₁-C₆-Alkyl, C₁-C₆-Alkoxy und/oder -OCF₃ ein- oder mehrfach, gleich oder verschieden substituiert sind.

Ebenso besonders bevorzugt steht R⁵ für -SO₂R¹², wobei
- R¹²: für einen C₁-C₆-Alkylrest, einen Phenyl- oder einen monocyclischen Heteroarylring steht, gegebenenfalls jeweils selbst mit Nitro, Halogen und/oder C₁-C₆-Alkyl ein- oder mehrfach, gleich oder verschieden substituiert.

In der allgemeinen Formel (I) kann R⁶ stehen für:
(i) Wasserstoff oder Hydroxy, oder
(ii) einen C₁-C₆-Alkyl-, C₃-C₆-Alkenyl-, C₃-C₆-Alkinyl- oder C₁-C₆-Alkoxyrest, einen C₃-C₇-Cycloalkyl- oder Phenylring, einen Heterocyclylring mit 3 bis 8 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Bevorzugt steht R⁶ für:
(i) Wasserstoff oder
(ii) einen C₁-C₄-Alkyl-, C₃-C₅-Alkenyl-, C₃-C₅-Alkinyl- oder C₁-C₅-Alkoxyrest, einen C₃-C₆-Cycloalkyl- oder Phenylring, einen Heterocyclylring mit 3 bis 6 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Mehr bevorzugt steht R⁶ für:
einen C₂-C₅-Alkyl₋, C₄-C₆-Alkenyl-, C₄-C₆-Alkinyl- oder C₂-C₅-Alkoxyrest, einen C₄-C₆-Cycloalkyl- oder Phenylring, einen Heterocyclylring mit 3 bis 5 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Besonders bevorzugt steht R⁶ für:
einen C₁-C₆-Alkyl-, einen C₁-C₆-Alkoxyrest oder einen C₃-C₇-Cycloalkylring, jeweils gegebenenfalls selbst mit Hydroxy, -NR⁸R⁹ und / oder C₁-C₆-Alkoxy ein- oder mehrfach, gleich oder verschieden substituiert.

In der allgemeinen Formel (I) kann R⁷ stehen für Wasserstoff oder einen C₁-C₆-Alkylrest.

In der allgemeinen Formel (I) können R⁸ und R⁹ unabhängig voneinander stehen für:
(i) Wasserstoff und/oder
(ii) einen C₁-C₆-Alkylrest, C₂-C₆-Alkenyl-, C₃-C₈-Cycloalkyl- und/oder Phenylring, einen Heterocyclylring mit 3 bis 8 Ringatomen und/oder einen monocyclischen Heteroarylring, jeweils gegebenenfalls mit Hydroxy, -NR¹⁰R¹¹, Cyano, Halogen, -CF₃, C₁-C₆-Alkoxy und/oder-OCF₃ ein- oder mehrfach, gleich oder verschieden substituiert,
oder
R⁸ und R⁹ bilden gemeinsam mit dem Stickstoffatom einen 5- bis 7-gliedrigen Ring, der gegebenenfalls zusätzlich zum Stickstoffatom 1 oder 2 weitere Heteroatome enthält und der mit Hydroxy, -NR¹⁰R¹¹, Cyano, Halogen, -CF₃, C₁-C₆-Alkoxy und/oder -OCF₃ ein- oder mehrfach, gleich oder verschieden substituiert sein kann.

Bevorzugt stehen R⁸ und R⁹ für:
(i) Wasserstoff und/oder
(ii) einen C₁-C₅-Alkyl-, C₂-C₅-Alkenylrest, einen C₃-C₇-Cycloalkyl- und/oder Phenylring und/oder einen monocyclischen Heteroarylring, jeweils gegebenenfalls mit Hydroxy, -NR¹⁰R¹¹ und/oder C₁-C₆-Alkoxy ein- oder mehrfach, gleich oder verschieden substituiert,
oder
R⁸ und R⁹ bilden gemeinsam mit dem Stickstoffatom einen 5- bis 7-gliedrigen Ring, der gegebenenfalls zusätzlich zum Stickstoffatom 1 weiteres Heteroatom enthält und der mit Hydroxy, -NR¹⁰R¹¹und/oder C₁-C₆-Alkoxy ein- oder mehrfach, gleich oder verschieden substituiert sein kann.

Mehr bevorzugt stehen R⁸ und R⁹ für:
(i) Wasserstoff und/oder
(ii) einen C₁-C₄-Alkylrest, C₃-C₆-Cycloalkyl- und/oder Phenylring,und/oder einen monocyclischen Heteroarylring, jeweils gegebenenfalls mit Hydroxy, -NR¹⁰R¹¹ oder C₁-C₆-Alkoxy ein- oder mehrfach, gleich oder verschieden substituiert,
oder
R⁸ und R⁹ bilden gemeinsam mit dem Stickstoffatom einen 5- bis 7-gliedrigen Ring, der gegebenenfalls zusätzlich zum Stickstoffatom 1 weiteres Heteroatom enthält und der mit Hydroxy ein- oder mehrfach substituiert sein kann.

Besonders bevorzugt stehen R⁸ und R⁹ für:
(i) Wasserstoff und/oder
(ii) einen C₁-C₆-Alkylrest, einen C₃-C₆-Cycloalkyl- und/oder Phenylring und/oder einen monocyclischen Heteroarylring,
oder
R⁸ und R⁹ bilden gemeinsam mit dem Stickstoffatom einen 5- oder 6-gliedrigen Ring, der gegebenenfalls zusätzlich zum Stickstoffatom 1 weiteres Heteroatom enthält.

In der allgemeinen Formel (I) können R¹⁰ und R¹¹ unabhängig voneinander stehen für Wasserstoff oder einen C₁-C₆-Alkylrest, der gegebenenfalls mit Hydroxy, Cyano, Halogen, -CF₃, C₁-C₆-Alkoxy und/oder -OCF₃ ein- oder mehrfach, gleich oder verschieden substituiert ist.

Bevorzugt können R¹⁰und R¹¹ unabhängig voneinander stehen für Wasserstoff oder einen C₁-C₆-Alkylrest, der gegebenenfalls mit Hydroxy, Halogen oder C₁-C₆-Alkoxy ein- oder mehrfach, gleich oder verschieden substituiert ist.

Mehr bevorzugt können R¹⁰ und R¹¹ unabhängig voneinander stehen für Wasserstoff oder einen C₁-C₆-Alkylrest, der gegebenenfalls mit Hydroxy ein- oder mehrfach, gleich oder verschieden substituiert ist.

Besonders bevorzugt können R¹⁰und R¹¹ unabhängig voneinander stehen für Wasserstoff oder eine Methylgruppe.

In der allgemeinen Formel (I) können R¹² , R¹³ , R¹⁴ unabhängig voneinander stehen für -CF₃ oder einen C₁-C₆-Alkyl-, C₂-C₆-Alkenyl- und/oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht,
die gegebenenfalls jeweils selbst mit Hydroxy, Nitro, -NR⁸R⁹, -NH-C(O)-C₁-C₆-Alkyl, Cyano, Halogen, -CF₃, C₁-C₆-Alkyl, C₁-C₆-Alkoxy und/oder -OCF₃ ein- oder mehrfach, gleich oder verschieden substituiert sind

Bevorzugt stehen R¹² , R¹³ , R¹⁴ unabhängig voneinander für einen C₁-C₆-Alkyl, C₂-C₆-Alkenyl- und/oder C₂-C₆-Alkinylrest, einen C₃-C₇-Cycloalkyl- und/oder Phenylring, einen Heterocyclylring mit 3 bis 8 Ringatomen und/oder einen monocyclischen Heteroarylring stehen,
jeweils gegebenenfalls selbst mit Hydroxy, Nitro, -NR⁸R⁹, Cyano, Halogen, -CF₃, C₁-C₆-Alkyl, C₁-C₆-Alkoxy und/oder -OCF₃ ein- oder mehrfach, gleich oder verschieden substituiert.

Bevorzugt steht R¹² für einen C₁-C₆-Alkyl, C₂-C₆-Alkenyl- oder C₂-C₆-Alkinylrest, einen C₃-C₇-Cycloalkyl- oder Phenylring , einen Heterocyclylring mit 3 bis 8 Ringatomen oder einen monocyclischen Heteroarylring,
jeweils gegebenenfalls selbst mit Hydroxy, Halogen, Nitro, -NR⁸R⁹, C₁-C₆-Alkyl und/oder C₁-C₆-Alkoxy ein- oder mehrfach, gleich oder verschieden substituiert.

Mehr bevorzugt steht R¹² für einen C₁-C₅-Alkyl, C₂-C₅-Alkenyl-, einen C₃-C₆-Cycloalkyl- oder Phenylring , einen Heterocyclylring mit 3 bis 6 Ringatomen oder einen monocyclischen Heteroarylring,
jeweils gegebenenfalls selbst mit Hydroxy, Halogen, Nitro, -NR⁸R⁹, C₁-C₆-Alkyl und/oder C₁-C₆-Alkoxy ein- oder mehrfach, gleich oder verschieden substituiert.

Besonders bevorzugt steht R¹² für -CF₃ oder für einen C₁-C₆-Alkylrest, einen Phenyl- oder einen monocyclischen Heteroarylring steht, die gegebenenfalls jeweils selbst mit Nitro, -NH-C(O)-C₁-C₆-Alkyl, Halogen, C₁-C₆-Alkyl, C₁-C₆-Alkoxy und/oder -OCF₃ ein- oder mehrfach, gleich oder verschieden substituiert sind.

Ebenso besonders bevorzugt steht R¹² für einen C₁-C₆-Alkylrest', einen Phenyl- oder monocyclischen Heteroarylring,
jeweils gegebenenfalls selbst mit Hydroxy, Halogen, Nitro oder C₁-C₆-Alkyl ein- oder mehrfach, gleich oder verschieden substituiert.

Bevorzugt stehen R¹³ und R¹⁴ unabhängig voneinander für einen C₁-C₆-Alkyl, C₂-C₆-Alkenyl- und/oder C₂-C₆-Alkinylrest, einen C₃-C₇-Cycloalkyl- und/oder Phenylring, einen Heterocyclylring mit 3 bis 8 Ringatomen und/oder einen monocyclischen Heteroarylring,
jeweils gegebenenfalls selbst mit Hydroxy,-NR⁸R⁹ und/oder C₁-C₆-Alkoxy ein- oder mehrfach, gleich oder verschieden substituiert.

Mehr bevorzugt stehen R¹³ und R¹⁴ unabhängig voneinander für einen C₁-C₅-Alkyl, C₂-C₅-Alkenyl- und/oder C₂-C₅-Alkinylrest, einen C₃-C₆-Cycloalkyl- und/oder Phenylring, einen Heterocyclylring mit 3 bis 6 Ringatomen und/oder einen monocyclischen Heteroarylring.

Besonders bevorzugt sehen R¹³ und R¹⁴ unabhängig voneinander für einen C₁-C₆-Alkylrest.

In der allgemeinen Formel (I) kann R¹⁶ stehen für:
(i) Wasserstoff oder
(ii) einen C₁-C₆-Alkyl-, C₃-C₆-Alkenyl-, C₃-C₆-Alkinylrest, einen C₃-C₇-Cycloalkyl-oder Phenylring, einen Heterocyclylring mit 3 bis 8 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Bevorzugt kann R¹⁶ stehen für:
einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert.

Mehr bevorzugt kann R¹⁶ stehen für:
einen C₁-C₆-Alkylrest, einen C₃-C₇-Cycloalkyl- oder Phenylring, einen Heterocyclylring mit 3 bis 8 Ringatomen oder einen monocyclischen Heteroarylring.

Besonders bevorzugt kann R¹⁶ stehen für einen C₁-C₆-Alkylrest.

Ebenfalls als von der vorliegenden Erfindung als erfasst anzusehen sind alle Verbindungen, die sich ergeben durch jede mögliche Kombination der oben genannten möglichen, bevorzugten und besonders bevorzugten Bedeutungen der Substituenten.

Besondere Ausführungsformen der Erfindung bestehen darüber hinaus in Verbindungen, die sich durch Kombination der direkt in den Beispielen offenbarten Bedeutungen für die Substituenten ergeben.

Die erfindungsgemäßen Verbindungen können mit einem Verfahren hergestellt werden, das folgende Schritte umfasst:
a) Umsetzung von 2-Chlor-pyrimidinen der Formel (IV) mit Nucleophilen der Formel (III) zu Verbindungen der Formel (II) 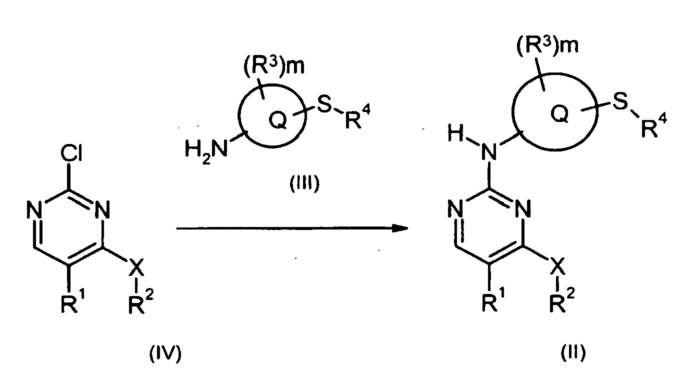
b) Iminierung der Thioether der Formel (II) unter Erhalt von Verbindungen der Formel (I) 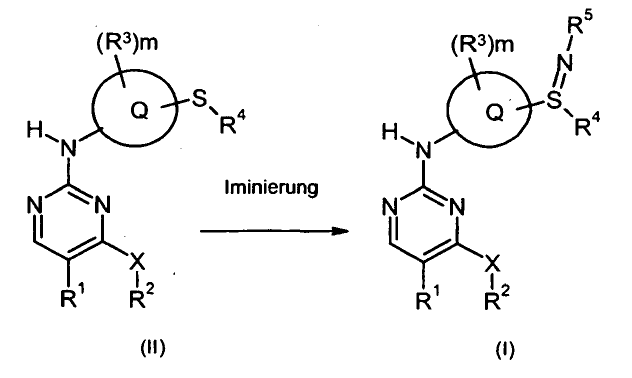
wobei Q, R¹, R², R³, R⁴, R⁵, X und m die in der allgemeinen Formel (I) gemäß der Ansprüche 1 bis 17 angegebenen Bedeutungen haben.

Die Intermediate der Formel (IV) können hergestellt werden durch Umsetzung von 2,4-Dichlor-pyrimidinen der Formel (VI) mit Nucleophilen der Formel (V), 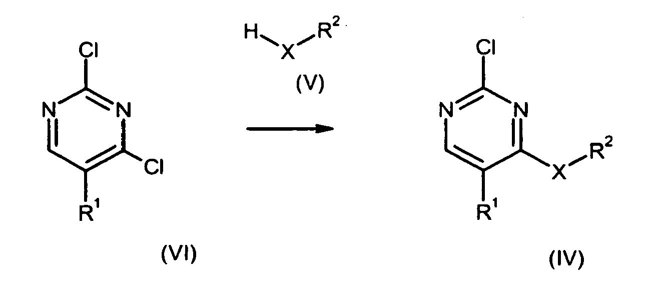 wobei R¹, R² und X die in der allgemeinen Formel (I) gemäß der Ansprüche 1 bis 20 angegebenen Bedeutungen haben.

Alternativ können die erfindungsgemäßen Verbindungen hergestellt werden durch Umsetzung von 2-Chlor-pyrimidinen der Formel (IV) mit Nucleophilen der Formel (VII), 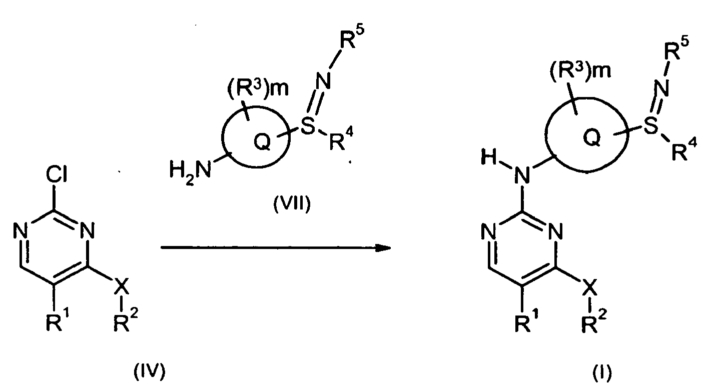 wobei Q, R¹, R², R³, R⁴, R⁵, X und m die in der allgemeinen Formel (I) gemäß der Ansprüche 1 bis 20 angegebenen Bedeutungen haben.

Die Intermediate der Formel (VII) können hergestellt werden mit einem Verfahren, das folgende Schritte umfasst:
a) Iminierung eines Thioether der Formel (IX) unter Erhalt von Sulfimiden der Formel (VIII) 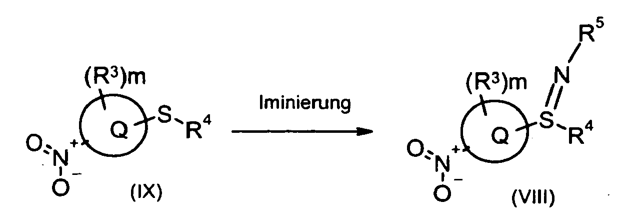
b) Reduktion der Nitro-Gruppe unter Erhalt der Intermediate der Formel (VII) 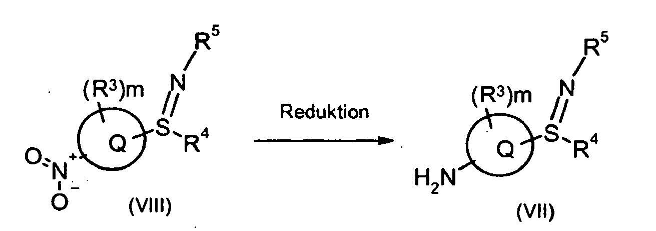 wobei Q, R³, R⁴ und R⁵ die in der allgemeinen Formel (I) gemäß der Ansprüche 1 bis 20 angegebenen Bedeutungen haben.

Alternativ können die Intermediate der Formel (VII) auch hergestellt werden mit einem Verfahren, das folgende Schritte umfasst:
a) Iminierung eines Thioether der Formel (X) unter Erhalt von Sulfimiden der Formel (XI) 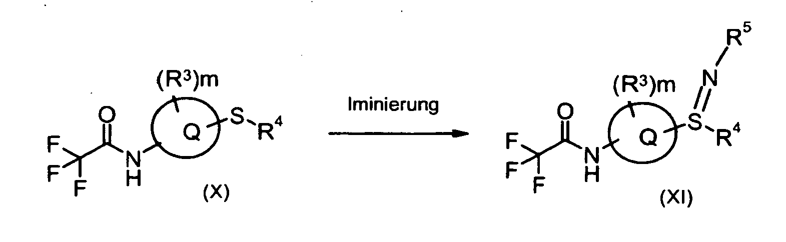
b) Abspaltung der Schutzgruppe unter Erhalt der Intermediate der Formel (VII) 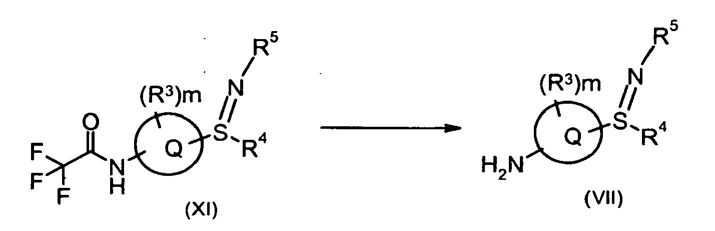
wobei Q, R³, R⁴ und R⁵ die in der allgemeinen Formel (I) gemäß der Ansprüche 1 bis 20 angegebenen Bedeutungen haben.

Der Anmeldung liegt folgende Gruppierung von Proteinkinasen zugrunde:
A. Zellzykluskinasen: a) CDKs, b) Plk, c) Aurora
B. angiogene Rezeptortyrosinkinasen: a) VEGF-R, b) Tie, c) FGF-R, d) EphB4
C. proliferative Rezeptortyrosinkinasen: a) PDGF-R, Flt-3, c-Kit
D. Kontrollpunktkinasen: a) ATM/ATR, b) Chk 1/2, c) TTK/hMps1, BubR1, Bub1
E. anti-apoptotische Kinasen a) AKT/PKB b) IKK c) PIM1, d) ILK
F. Migratorische Kinasen a) FAK, b) ROCK

### A. Zellzykluskinasen a) CDKs, b) Plk, c) Aurora

Der eukaryote Zellteilungszyklus stellt die Duplikation des Genoms und seine Verteilung auf die Tochterzellen sicher, indem er eine koordinierte und regulierte Abfolge von Ereignissen durchläuft. Der Zellzyklus wird in vier aufeinander folgende Phasen eingeteilt: die G1 Phase repräsentiert die Zeit vor der DNA-Replikation, in der die Zelle wächst und für externe Stimuli empfänglich ist. In der S Phase repliziert die Zelle ihre DNA, und in der G2 Phase bereitet sie sich auf den Eintritt in die Mitose vor. In der Mitose (M Phase) wird die replizierte DNA getrennt und die Zellteilung vollzogen.

Die Zyklin-abhängigen Kinasen (CDKs), eine Familie von Serin/Threonin-Kinasen, deren Mitglieder die Bindung eines Zyklins (Cyc) als regulatorische Untereinheit zu ihrer Aktivierung benötigen, treiben die Zelle durch den Zellzyklus. Unterschiedliche CDK/Cyc Paare sind in den verschiedenen Phasen des Zellzyklus aktiv. Für die grundlegende Funktion des Zellzyklus bedeutende CDK/Cyc Paare sind beispielsweise CDK4(6)/CycD, CDK2/CycE, CDK2/CycA, CDK1 /CycA und CDK1/CycB.

Der Eintritt in den Zellzyklus und das Durchlaufen des "Restriction Point", der die Unabhängigkeit einer Zelle von weiteren Wachstumssignalen für den Abschluss der begonnenen Zellteilung markiert, werden durch die Aktivität der CDK4(6)/CycD und CDK2/CycE Komplexe kontrolliert. Das wesentliche Substrat dieser CDK-Komplexe ist das Retinoblastoma-Protein (Rb), das Produkt des Retinoblastoma Tumorsuppressor Gens. Rb ist ein transkriptionelles Ko-Repressor Protein. Neben anderen noch weitgehend unverstandenen Mechanismen, bindet und inaktiviert Rb Transkriptionsfaktoren vom E2F-Typ, und bildet transkriptionelle Repressorkomplexe mit Histon-Deacetylasen (HDAC) (Zhang H.S. et al. (2000). Exit from G1 and S phase of the cell cycle is regulated by repressor complexes containing HDAC-RbhSWI/SNF and Rb-hSWI/SNF. Cell 101, 79-89). Durch die Phosphorylierung des Rb durch CDKs werden gebundene E2F Transkriptionsfaktoren freigesetzt und führen zu transkriptioneller Aktivierung von Genen, deren Produkte für die DNA Synthese und die Progression durch die S-Phase benötigt werden. Zusätzlich bewirkt die Rb-Phosphorylierung die Auflösung der Rb-HDAC Komplexe, wodurch weitere Gene aktiviert werden. Die Phosphorylierung von Rb durch CDKs ist mit dem Überschreiten des "Restriction Point" gleichzusetzen. Für die Progression durch die S-Phase und deren Abschluss ist die Aktivität der CDK2/CycE und CDK2/CycA Komplexe notwendig. Nach vollständiger Replikation der DNA steuert die CDK1 im Komplex mit CycA oder CycB das Durchlaufen der Phase G2 und den Eintritt der Zelle in die Mitose (Abb. 1). Beim Übergang von der G2 Phase in die Mitose trägt die Polo-like Kinase Plk1 zur Aktivierung der CDK1 bei. Während des Ablaufs der Mitose ist Plk1 weiterhin an der Maturierung der Zentrosomen, dem Aufbau des Spindelapparates, der Separierung der Chromosomen und der Trennung der Tochterzellen beteiligt.

Die Familie der Aurora Kinasen besteht im humanen Organismus aus drei Mitgliedern: Aurora-A, Aurora-B und Aurora-C. Die Aurora Kinasen regulieren wichtige Prozesse während der Zellteilung (Mitose).
Aurora-A ist an den Zentrosomen und den Spindelmikrotubuli lokalisiert, wo es verschiedene Substratproteine phosphoryliert, unter anderem das Kinesin Eg5, TACC, PP1. Die genauen Mechanismen der Genese des Spindelapparates und der Rolle von Aurora-A dabei sind allerdings noch weitgehend unklar.
Aurora-B ist Teil eines Multiprotein Komplexes, der an der Zentrosomenstruktur der Chromosomen lokalisiert ist und neben Aurora-B u.a. INCENP, Survivin und Borealin/Dasra B enthält (zusammenfassende Übersicht in: Vagnarelli & Earnshaw, Chromosomal passengers: the four-dimensional regulation of mitotic events. Chromosoma. 2004 Nov;113(5):211-22. Epub 2004 Sep 4). Die Kinaseaktivität von Aurora-B stellt sicher, dass vor der Teilung der Chromosomenpaare alle Verbindungen zum Mikrotubulin-Spindelapparat korrekt sind (sogenannter Spindel Checkpoint). Substrate von Aurora-B sind hier unter anderem Histon H3 und MCAK. Nach Trennung der Chromosomen ändert Aurora-B seine Lokalisation und kann während der letzten Mitosephase (Zytokinese) an der noch verbliebenen Verbindungsbrücke zwischen den beiden Tochterzellen gefunden werden. Durch Phosphorylierung seiner Substrate MgcRacGAP, Vimentin, Desmin, der leichten regulatorischen Kette von Myosin, und anderen, reguliert Aurora-B die Abschnürung der Tochterzellen.
Aurora-C ist von seiner Aminosäuresequenz, Lokalisation, Substratspezifität und Funktion Aurora-B sehr ähnlich (Li X et al. Direct association with inner centromere protein (INCENP) activates the novel chromosomal passenger protein, Aurora-C. J Biol Chem. 2004 Nov 5;279(45):47201-11. Epub 2004 Aug 16;, Chen et al. Overexpression of an Aurora-C kinase-deficient mutant disrupts the Aurora-B/INCENP complex and induces polyploidy. J Biomed Sci. 2005;12(2):297-310; Yan X et al. Aurora-C is directly associated with Survivin and required for cytokinesis. Genes to ells 2005 10, 617-626). Der Hauptunterschied zwischen Aurora-B und Aurora-C ist die starke Überexpression von Aurora-C im Hoden (Tseng TC et al. Protein kinase profile of sperm and eggs: cloning and characterization of two novel testis-specific protein kinases (AIE1, AIE2) related to yeast and fly chromosome segregation regulators. DNA Cell Biol. 1998 Oct;17(10):823-33.).
Die essentielle Funktion der Aurora Kinasen bei der Mitose macht sie zu interessanten Zielproteinen für die Entwicklung kleiner inhibitorischer Moleküle zur Behandlung von Krebs oder anderen Erkrankungen, die Störungen der Zellproliferation zur Ursache haben. Überzeugende experimentelle Daten deuten darauf hin, dass eine Inhibition der Aurora-Kinasen in vitro und in vivo das Fortschreiten zellulärer Proliferation verhindert und den programmierten Zelltod (Apoptose) auslöst. Dies konnte mittels (1) siRNA Technologie (Du & Hannon. Suppression of p160ROCK bypasses cell cycle arrest after Aurora-A/STK15 depletion. Proc Natl Acad Sci U S A. 2004 Jun 15;101(24):8975-80. Epub 2004 Jun 3; Sasai K et al. Aurora-C kinase is a novel chromosomal passenger protein that can complement Aurora-B kinase function in mitotic cells. Cell Motil Cytoskeleton. 2004 Dec;59(4):249-63) oder (2) Überexpression einer dominant negativen Aurora Kinase (Honda et al. Exploring the functional interactions between Aurora B, INCENP, and survivin in mitosis. Mol Biol Cell. 2003 Aug;14(8):3325-41. Epub 2003 May 29), sowie (3) mit kleinen chemischen Molekülen gezeigt werden, die spezifisch Aurora Kinasen inhibieren (Hauf S et al. The small molecule Hesperadin reveals a role for Aurora B in correcting kinetochore-microtubule attachment and in maintaining the spindle assembly checkpoint. J Cell Biol. 2003 Apr 28;161(2):281-94. Epub 2003 Apr 21.; Ditchfield C et al. Aurora B couples chromosome alignment with anaphase by targeting BubR1, Mad2, and Cenp-E to kinetochores. J Cell Biol. 2003 Apr 28;161(2):267-80.).
Die Inaktivierung von Aurora Kinasen führt dazu, dass (1) der mitotische Spindelapparat nicht oder fehlerhaft ausgebildet wird (vorwiegend bei Aurora-A Inhibition) und /oder dass (2) durch Blockierung des Spindel Checkpoints keine oder eine fehlerhafte Trennung der Schwesterchromatiden statt findet (vorwiegend bei Aurora-B/-C Inhibition) und / oder dass (3) die Trennung der Tochterzellen nicht vollzogen wird (vorwiegend bei Aurora-B/-C Inhibition). Diese Folgen (1-3) der Inaktivierung von Aurora Kinasen im einzelnen oder als Kombinationen führen schließlich zu Aneuploidie und / oder Polyploidie und letztlich sofort oder nach wiederholten Mitosen zu einem nicht lebensfähigen Zustand bzw. zum programmierten Zelltod der proliferierenden Zellen (mitotische Katastrophe). Spezifische Kinaseinhibitoren können den Zellzyklus in unterschiedlichen Stadien beeinflussen. So ist beispielsweise von einem CDK4 oder einem CDK2 Inhibitor eine Blockade des Zellzyklus in der G1 Phase bzw. im Übergang von der G1 Phase zu der S-Phase zu erwarten.

### B. Angiogene Rezeptortyrosinkinasen

Rezeptortyrosinkinasen und deren Liganden sind in entscheidender Weise bei einer Vielzahl von zellulären Prozessen beteiligt, die in der Regulation des Wachstums und der Differenzierung von Zellen involviert sind. Von besonderem Interesse sind hier das Vascular Endothelial Growth Factor (VEGF) / VEGF-Rezeptor System, das Fibroblast Growth Factor (FGF) / FGF Rezeptor System, das Eph-Ligand / Eph-Rezeptor System, und das Tie-Ligand / Tie-Rezeptor System. In pathologischen Situationen, die mit einer verstärkten Neubildung von Blutgefäßen (Neovaskularisierung) einher gehen, wie z.B. Tumorerkrankungen, wurde eine erhöhte Expression von angiogenen Wachstumsfaktoren und ihrer Rezeptoren gefunden. Inhibitoren des VEGF / VEGF-Rezeptor Systems, FGF / FGF-Rezeptor Systems (Rousseau et al., The tyrp1-Tag/tyrp1-FGFR1-DN bigenic mouse: a model for selective inhibition of tumor development, angiogenesis, and invasion into the neural tissue by blockade of fibroblast growth factor receptor activity. Cancer Res. 64, :2490, 2004), des EphB4 Systems (Kertesz et al., The soluble extracellular domain of EphB4 (sEphB4) antagonizes EphB4-EphrinB2 interaction, modulates angiogenesis and inhibits tumor growth. Blood. 2005 Dec 1; [Epub ahead of print]), sowie des Tie-Ligand / Tie Systems (Siemeister et al., Two independent mechanisms essential for tumor angiogenesis: inhibition of human melanoma xenograft growth by interfering with either the vascular endothelial growth factor receptor pathway or the Tie-2 pathway. Cancer Res. 59, 3185, 1999) können die Ausbildung eines Blutgefäßsystems in Tumoren inhibieren, damit den Tumor von der Sauerstoff- und Nährstoffversorgung abschneiden und somit das Tumorwachstum inhibieren.

### C. Proliferative Rezeptortyrosinkinasen

Rezeptortyrosinkinasen und deren Liganden sind in entscheidender Weise bei der Proliferation von Zellen beteiligt. Von besonderem Interesse sind hier das Platelet-Derived Growth Factor (PDGF) Ligand / PDGF-Rezeptor System, c-Kit Ligand / c-Kit Rezeptor System und das FMS-like Tyrosinkinase 3 (Flt-3) Ligand / Flt-3 System. In pathologischen Situationen, die mit einem verstärkten Wachstum von Zellen einher gehen, wie z.B. Tumorerkrankungen, wurde eine erhöhte Expression von proliferativen Wachstumsfaktoren und ihrer Rezeptoren oder die Kinase aktivierende Mutationen gefunden. Die Inhibition der Enzymaktivität dieser Rezeptortyrosinkinasen führt zu einer Reduktion des Tumorwachstums. Dies konnte z.B. durch Studien mit dem kleinen chemischen Molekül STI571/Glivec gezeigt werden, welches unter anderem PDGF-R und c-Kit inhibiert (zusammenfassende Übersichten in: Oestmann A., PDGF receptors - mediators of autocrine tumor growth and regulators of tumor vasculature and stroma, Cytokine Growth Factor Rev. 2004 Aug;15(4):275-86; Roskoski R., Signaling by Kit protein-tyrosine kinase - the stern cell factor receptor. Biochem Biophys Res Commun. 2005 Nov 11:337(1):1-13.; Markovic A. et al., FLT-3: a new focus in the understanding of acute leukemia. Int J Biochem Cell Biol. 2005 Jun;37(6):1168-72. Epub 2005 Jan 26.).

### D. Kontrollpunktkinasen

Unter Kontrollpunktkinasen (= Checkpoint-Kinasen) im Sinne der vorliegenden Anmeldung sind Zellzykluskinasen zu verstehen, die den geordneten Ablauf der Zellteilung überwachen, wie beispielsweise ATM und ATR, Chk1 und Chk2, Mps1, Bub1 und BubR1. Von besonderer Bedeutung sind der DNA-Schädigungs Checkpoint in der G2 Phase und der Spindel-Checkpoint während der Mitose.

Die Aktivierung der ATM, ATR, Chk1 und Chk2 Kinasen erfolgt nach DNA-Schädigung einer Zelle und führt zu einem Arrest des Zellzyklus in der G2 Phase durch Inaktivierung der CDK1. (Chen & Sanchez, Chk1 in the DNA damage response: conserved roles from yeasts to mammals. DNA Repair 3, 1025, 2004). Inaktivierung von Chk1 verursacht den Verlust des durch DNA Schädigung induzierten G2 Arrest, zur Zellzyklusprogression der Zelle in Anwesenheit geschädigter DNA, und führt schließlich zum Zelltod (Takai et al. Aberrant cell cycle checkpoint function and early embryonic death in Chk1(-/-) mice.Genes Dev. 2000 Jun 15;14(12):1439-47; Koniaras et al. Inhibition of Chk1-dependent G2 DNA damage checkpoint radiosensitizes p53 mutant human cells. Oncogene. 2001 Nov 8;20(51):7453-63.; Liu et al. Chk1 is an essential kinase that is regulated by Atr and required for the G(2)/M DNA damage checkpoint. Genes Dev. 2000 Jun 15;14(12):1448-59.). Die Inaktivierung von Chk1, Chk2 oder Chk1 und Chk2 verhindert den durch DNA-Schädigung verursachten G2 Arrest und macht proliferierende Krebszellen empfindlicher gegenüber DNA-schädigende Therapien wie z.B. Chemotherapie oder Radiotherapie. Chemotherapien, die zur DNA Schädigung führen, sind z.B. DNA-Strangbruch induzierende Substanzen, DNAalkylierende Substanzen, Topoisomerase Inhibitoren, Aurora Kinase Inhibitoren, Substanzen, die den Aufbau der mitotischen Spindel beeinflussen, hypoxischer Stress aufgrund limitierter Sauerstoffversorgung eines Tumors (z.B. induziert durch anti-angiogene Medikamente wie VEGF Kinase Inhibitoren).

Ein zweiter wesentlicher Checkpoint innerhalb der Zellzyklus kontrolliert den korrekten Aufbau und Anhaftung des Spindelapparates an die Chromosomen während der Mitose. An diesem sogenannten Spindel-Checkpoint sind die Kinasen TTK/hMps1, Bub1, und BubR1 beteiligt (zusammenfassende Übersicht in: Kops et al. On the road to cancer: aneuptoidy and the mitotic checkpoint. Nat Rev Cancer. 2005 Oct;5(10):773-85). Diese sind an Kinetochoren von noch nicht an den Spindelapparat angehefteter kondensierter Chromosomen lokalisiert und inhibieren den sogenannten anaphase-promoting complex/cyclosome (APC/C). Erst nach vollständiger und korrekter Anheftung des Spindelapparates an die Kinetochoren werden die Spindel-Checkpoint Kinasen Mps-1, Bub1, und BubR1 inaktiviert, wodurch APC/C aktiviert wird und es zur Trennung der gepaarten Chromosomen kommt. Eine Inhibition der Spindel-Checkpointkinasen führt zur der Trennung der gepaarten Chromosomen bevor alle Kinetochoren an den Spindelapparat angeheftet sind und in Folge zu chromosomalen Fehlverteilungen, die von den Zellen nicht toleriert werden und schließlich zum Zellzyklusstillstand oder zum Zelltod führen.

### E. Anti-apoptotische Kinasen

Verschiedene Mechanismen schützen eine Zelle gegenüber dem Zelltod während nicht optimaler Lebensbedingungen. In Tumorzellen führen diese Mechanismen zu einem Überlebensvorteil der Zellen in der wachsenden Tumormasse, die durch den Mangel an Sauerstoff, Glukose und weiteren Nährstoffen gekennzeichnet ist, ermöglichen ein Überleben von Tumorzellen ohne Anheftung an die extrazelluäre Matrix was zur Metastasierung führen kann, oder führen zu Resistenzen gegenüber Therapeutika. Wesentliche anti-apoptotische Signalwege umfassen den PDK1-AKT/PKB Signalweg (Altomare & Testa. Perturbations of the AKT signaling pathway in human cancer. Oncogene. 24, 7455, 2005), den NFkappaB Signalweg (Viatour et al. Phosphorylation of NFkB and IkB proteins: implications in cancer and inflammation), den PIM1 Signalweg (Hammerman et al. PIM and Akt oncogenes are independent regulators of hematopoietic cell growth and survival. Blood. 2005 105, 4477, 2005) und den integrin-linked kinase (ILK) Signalweg (Persad & Dedhar. The role of integrin-linked kinase (ILK) in cancer progression. Cancer Met. Rev. 22, 375, 2003). Durch die Inhibition der anti-apoptotischen Kinasen, wie beispielsweise AKT/PBK, PDK1, IkappaB Kinase (IKK), PIM1, oder ILK, werden die Tumorzellen gegenüber der Wirkung von Therapeutika oder gegenüber ungünstigen Lebensbedingungen in der Tumorumgebung sensitiviert. Tumorzellen werden nach Inhibition der anti-apoptotischen Kinasen empfindlicher auf durch Aurora-Inhibition verursachte Störungen der Mitose reagieren und vermehrt dem Zelltod unterliegen.

### F. Migratorische Kinasen

Invasives, Gewebe-infiltrierendes Tumorwachstum und Metastasierung setzt voraus, daß die Tumorzellen den Gewebeverband durch Migration verlassen können. Verschiedene zelluläre Mechanismen sind in die Regulation der Zellmigration involviert: Integrin-vermittelte Adhäsion an Proteine der extrazellulären Matrix reguliert über die Aktivität der focal adhesion kinase (FAK); Kontrolle des Zusammenbaus der kontraktilen Aktin-Filamente über den RhoA / Rho-Kinase (ROCK) Signalweg (zusammenfassende Übersicht in M.C. Frame, Newest findings on the oldest oncogene; how activated src does it. J. Cell Sci. 117, 989, 2004).

Die erfindungsgemäßen Verbindungen wirken zum Beispiel
- gegen Krebs, wie solide Tumore, Tumor- oder Metastasenwachstum, insbesondere:
   Ataxia-telangiectasia, Basalzellkarzinom, Blasenkarzinom, Gehirntumor, Brustkrebs, Cervix Karzinom, Tumoren des Zentralnervensystems, Kolorektalkarzinom, Endometriales Karzinom, Magenkarzinom, Gastrointestinales Karzinom, Kopf- und Halstumore, Akute lymphozytische Leukämie, Akute myelogene Leukämie, Chronische lymphozytische Leukämie, Chronische myelogene Leukämie, Haarzell Leukämie, Leberkarzinom, Lungentumor, Nicht-kleinzelliges Lungenkarzinom, Kleinzelliges Lungenkarzinom, B-Zell Lymphom, Hodgkin's Lymphom, Non-Hodgkin's Lymphom, T-Zell Lymphom, Melanom, Mesotheliom, Myelom, Myom, Tumore des Oesophagus, orale Tumore, Ovarialkarzinom, Pankreastumore, Prostatatumore, Nierenkarzinom, Sarkom, Kaposi's Sarkom, Leiomyosarkom, Hautkrebs, Plattenzellkarzinom, Hodenkrebs, Schilddrüsenkrebs, Bindegewebstumor des Magen-Darmgewebes, Bindegewebssarkom der Haut, Hypereosinophiles Syndrom, Mastzellenkrebs,
- bei kardiovaskulären Erkrankungen wie Stenosen, Arteriosklerosen und Restenosen, Stent-induzierte Restenose,
- bei Angiofibrom, Crohn-Krankheit, Endometriose, Hämangioma.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

### Herstellung der erfindungsgemäßen Verbindungen

### Verfahrensvariante 1

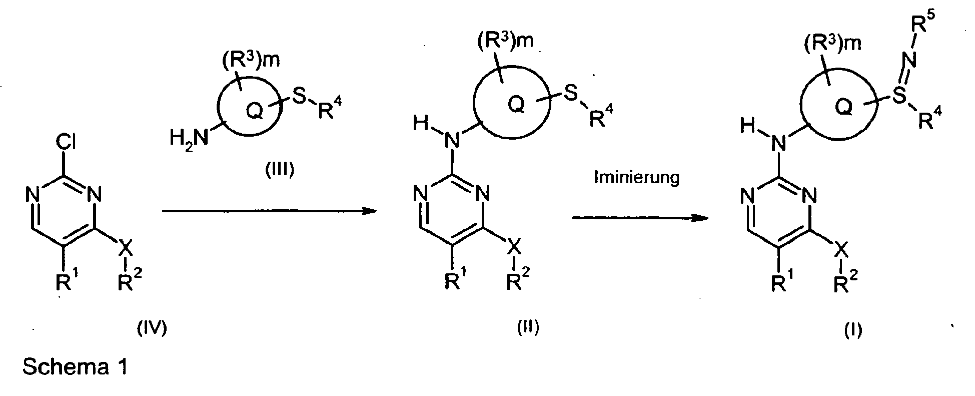

Die Substituenten Q, R¹, R², R³, R⁴, R⁵, X und m haben die in der allgemeinen Formel (I) angegebenen Bedeutungen.
2-Chlor-pyrimidine der Formel (IV) können mit Nukleophilen der Formel (III) zu Verbindungen der Formel (II) umgesetzt werden. Die erhaltenen Thioether der Formel (II) können anschließend durch Iminierung zu Produkten der Formel (I) umgesetzt werden (für Iminierungsmethoden siehe z.B : a) Johnson et al, J. Org. Chem. 1979, 44, 2065; b) Oae et al, Org. Chem. Sulfur 1977, 383; c) Kucsman et al, Phosphorous Sulfur 1977, 3, 9; d) Sharpless et al, J. Org. Chem. 2001, 66, 594; e) Katsuki et al, Tetrahedron Lett. 2001, 42, 7071; f) Bolm et al, Org. Lett. 2004, 6, 1305; g) Carreira et al, Helv. Chim. Acta 2002, 85, 3773; h) Bolm et al, Org. Lett. 2006, 8, 2349; i) Bolm et al, Org. Lett. 2005, 7, 4983; j) Bolm et al, Org. Lett. 2006, 8, 2349).

### Herstellung der Intermediate der Formel (IV) :

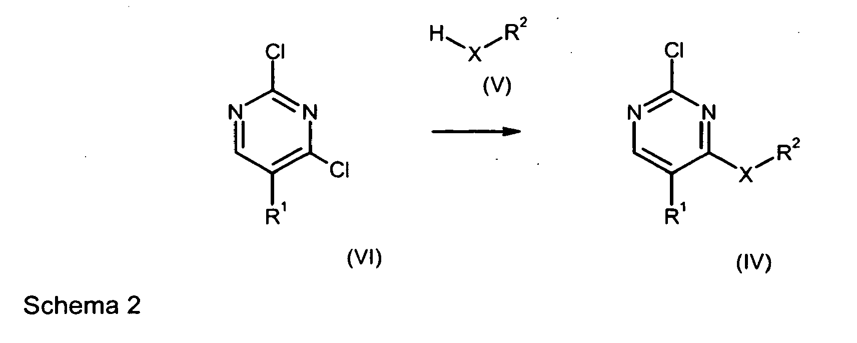

Die Substituenten R¹, R² und X haben die in der allgemeinen Formel (I) angegebenen Bedeutungen.
2,4-Dichlor-pyrimidine der Formel (VI) können mit Nukleophilen der Formel (V) zu Verbindungen der Formel (IV) umgesetzt werden (siehe z.B.: a) U. Lücking et al, WO 2005037800; b) J. Bryant et al, WO 2004048343; c) U. Lücking et al, WO 2003076437; d) T. Brumby et al, WO 2002096888).

### Verfahrensvariante 2

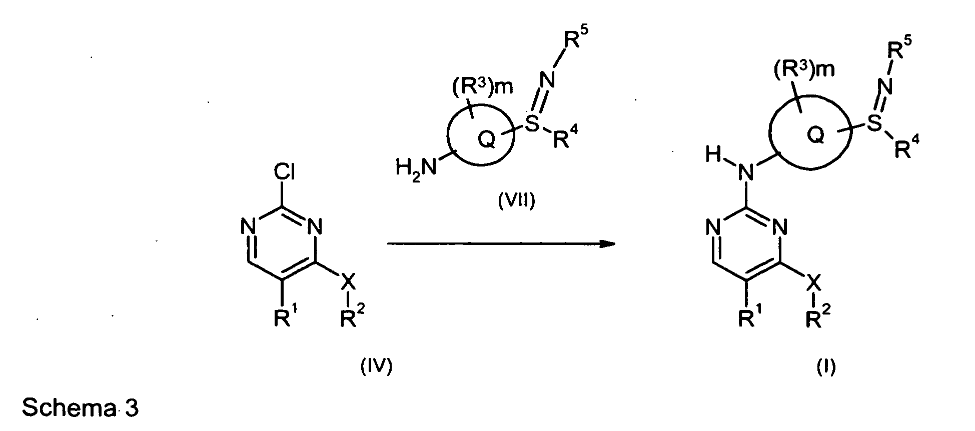

Die Substituenten Q, R¹, R², R³, R⁴, R⁵, X und m haben die in der allgemeinen Formel (I) angegebenen Bedeutungen.

2-Chlor-pyrimidine der Formel (IV) können mit Nukleophilen der Formel (VII) zu Produkten der Formel (I) umgesetzt werden. Geeignet ist beispielsweise die Verwendung von Isopropanol, Acetonitril oder 1-Butanol als Lösungsmittel und ggf. der Zusatz einer Säure wie beispielsweise Hydrogenchlorid.

### Herstellung der Intermediate der Formel (VII) :

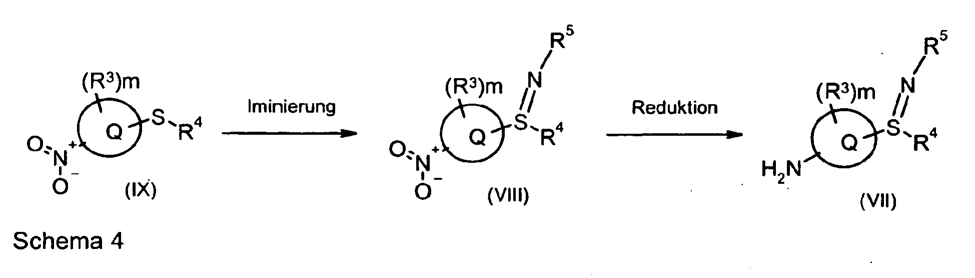

Die Substituenten Q, R³, R⁴, R⁵ und m haben die in der allgemeinen Formel (I) angegebenen Bedeutungen.
Thioether der Formel (IX) können zu Sulfimiden der Formel (VIII) umgesetzt werden (für Iminierungsmethoden siehe z. B. : a) Johnson et al, J. Org: Chem. 1979, 44, 2065; b) Oae et al, Org. Chem. Sulfur 1977, 383; c) Kucsman et al, Phosphorous Sulfur 1977, 3, 9; d) Sharpless et al, J. Org. Chem. 2001, 66, 594; e) Katsuki et al, Tetrahedron Lett. 2001, 42, 7071; f) Bolm et al, Org. Lett. 2004, 6, 1305; g) Carreira et al, Helv. Chim. Acta 2002, 85, 3773; h) Bolm et al, Org. Lett. 2006, 8, 2349; i) Bolm et al, Org. Lett. 2005, 7, 4983; Bolm et al, Org. Lett. 2006, 8, 2349)).
Für die anschließende Reduktion der Nitrogruppe stehen eine Reihe von Methoden zur Verfügung (siehe z.B.: R.C. Larock, Comprehensive Organic Transformations, VCH, New York, 1989, 411-415). Geeignet ist beispielsweise die beschriebene Hydrierung unter Verwendung von Raney Nickel oder die Verwendung von Titan(III)chiorid in THF.

Alternativ können die Intermediate der Formel (VII) auch über folgendes Verfahren hergestellt werden (Schema 5):

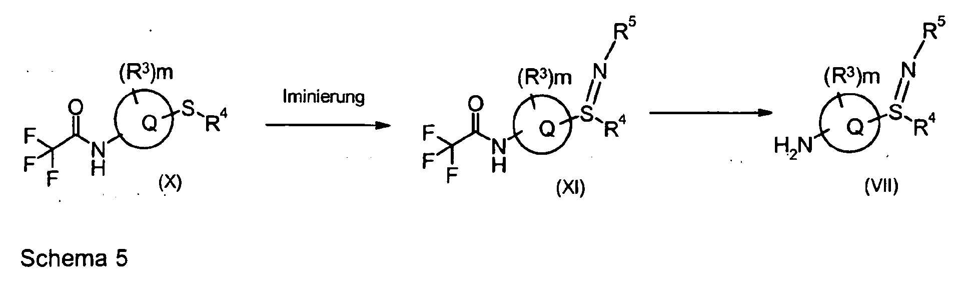

Die Substituenten Q, R³, R⁴, R⁵ und m haben die in der allgemeinen Formel (I) angegebenen Bedeutungen

Thioether der Formel (X) können zu Sulfimiden der Formel (XI) umgesetzt werden (für Iminierungsmethoden siehe z. B. : a) Johnson et al, J. Org: Chem. 1979, 44, 2065; b) Oae et al, Org. Chem. Sulfur 1977, 383; c) Kucsman et al, Phosphorous Sulfur 1977, 3, 9; d) Sharpless et al, J. Org. Chem. 2001, 66, 594; e) Katsuki et al, Tetrahedron Lett. 2001, 42, 7071; f) Bolm et al, Org. Lett. 2004, 6, 1305; g) Carreira et al, Helv. Chim. Acta 2002, 85, 3773; h) Bolm et al, Org. Lett. 2006, 8, 2349; i) Bolm et al, Org. Lett. 2005, 7, 4983; Bolm et al, Org. Lett. 2006, 8, 2349)). Anschließend erfolgt die Abspaltung der Schutzgruppe unter Bildung der Intermediate der Formel (VII). Besonders geeignet ist beispielsweise die beschriebene Verwendung von Kaliumcarbonat in Methanol.

### Verfahrensvariante 1 (Beispiele 1-22)

### Beispiel 1

### (RS)-S-(4-{[5-Brom-4-(isopropylamino)pyrimidin-2-yl]amino}phenyl)-S-methyl-N-(tolylsulfonyl)sulfimid

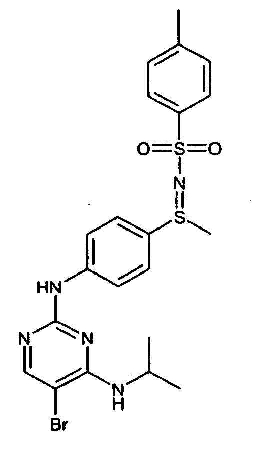

### 1a) Herstellung der Zwischenprodukte

### Verbindung 1.1

### (5-Brom-2-chlor-pyrimidin-4-yl)-isopropyl-amin

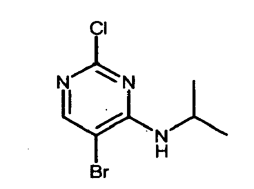

Eine Lösung von 4,87 g (21,4 mmol) 5-Brom-2,4-dichlor-pyrimidin in 23 ml Acetonitril wird bei 0°C mit 3,3 ml (23.8 mmol) Triethylamin und 2,0 ml (23,3 mmol) 2-Aminopropan versetzt. Das Reaktionsgemisch wird über Nacht bei Raumtemperatur gerührt und anschließend das Lösungsmittel abgezogen. Der erhaltene Rückstand wird chromatographisch (Dichlormethan / Ethanol 8:2) gereinigt. Man erhält 4,09 g (16,4 mmol; Ausbeute: 76 %) des Produktes.
¹H-NMR (DMSO): 8.21 (s, 1H), 7.30 (d, 1H), 4.25 (m, 1H), 1.15 (d, 6H).

### Verbindung 1.2

### 5-Bromo-N⁴-isopropyl-N²-(4-methylsulfanyl-phenyl)-pyrimidin-2,4-diamin

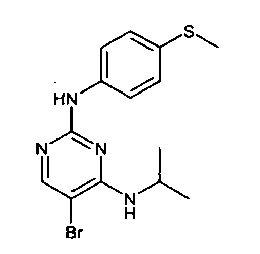

Eine Lösung von 4,08 g (16,3 mmol) (5-Brom-2-chlorpyrimidin-4-yl)-isopropyl-amin in 20 ml Acetonitril wird bei Raumtemperatur mit einer Lösung von 2 ml (16,3 mmol) 4-Methylsulfanyl-phenylamin in 10 ml Acetonitril versetzt. Der Ansatz wird mit 4,1 ml einer 4 molaren Lösung von Chlorwasserstoff in Dioxan und 4,1 ml Wasser versetzt und anschließend 16 Stunden unter Rückfluss gerührt. Nach dem Erkalten wird der gebildete Niederschlag abgesaugt, mit Wasser gewaschen und getrocknet. Man erhält 4,94 g (12,7 mmol; Ausbeute: 78%) des Produktes in Form des Hydrochlorides.
¹H-NMR (DMSO): 10.39 (s, 1H), 8.18 (s, 1H), 7.88 (br, 1H), 7.49 (m, 2H), 7.29 (m, 2H), 4.30 (m, 1H), 2.5 (s, 3H), 1.21 (d, 6H).
MS: 353 (ES).

### 1b) Herstellung des Endproduktes

Eine Suspension von 1,56 g (4,0 mmol) des Hydrochlorides von 5-Bromo-N⁴-isopropyl-N²-(4-methylsulfanyl-phenyl)-pyrimidin-2,4-diamin in 20 ml Acetonitril wird bei Raumtemperatur mit 1,36 g (4,8 mmol) Chloramin-T Trihydrat (Aldrich) versetzt. Der Ansatz wird 16 Stunden bei Raumtemperatur gerührt und anschließend mit Essigester verdünnt. Der Ansatz wird abgenutscht und der Filterkuchen mit Essigester nachgewaschen. Die vereinten organischen Phasen werden getrocknet (Na₂SO₄), filtriert und eingeengt. Der erhaltene Rückstand wird per HPLC gereinigt:
- Säule:: Purospher Star C18 5µ
- Länge x ID:: 125 x 25 mm
- Eluenten:: A: H₂O + 0,2% NH₃, B: MeCN
- Fluss:: 25 ml / min
- Gradient:: 50%A+50%B(1-)_50->64%B(10')->95%B(0,5')
- Detektor:: UV 254nm
- Temperatur:: Raumtemperatur
- RT in min:: 7,8-8,

Man erhält 289 mg (0,55 mmol; Ausbeute: 14 %) des Produktes.

¹H-NMR (DMSO): 9.64 (s, 1H), 8.08 (s, 1H), 7.88 (m, 2H), 7.59 (m, 2H), 7.51 (m, 2H), 7.19 (m, 2H), 6.58 (d, 1H), 4.30 (m, 1H), 2.87 (s, 3H), 2.28 (s, 3H), 1.22 (d, 6H). ¹³C-NMR (DMSO): 158.4 (s), 157.8 (s), 156.3 (d), 145.2 (s), 142.3 (s), 141.5 (s), 129.6 (d), 127.8 (d), 126.4 (s), 126.2 (d), 119.0 (d), 94.4 (s), 43.0 (d), 37.6 (q), 22.4 (q), 21.3 (q).
MS: 522 (ES+).

### Beispiel 2

### (RS)-S-(3-{[5-Brom-4-(isopropylamino)pyrimidin-2-yl]amino}phenyl)-S-methyl-N-(tolylsulfonyl)sulfimid

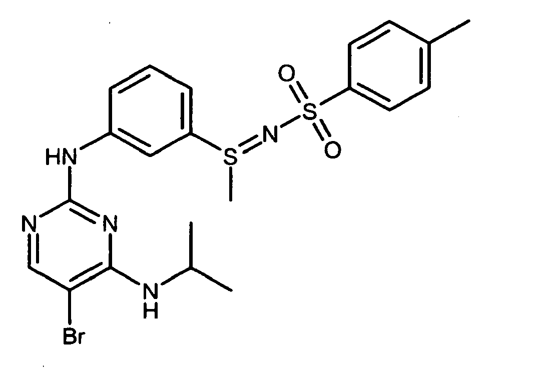

### 2a) Herstellung des Zwischenproduktes

### Verbindung 2.1)

### 5-Bromo-N⁴-isopropyl-N²-(3-methylsulfanyl-phenyl)-pyrimidin-2,4-diamin

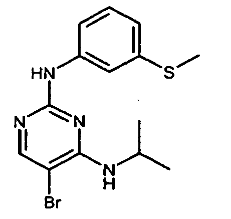

Eine Lösung von 0,152 g (0,61 mmol) (5-Brom-2-chlor-pyrimidin-4-yl)-isopropyl-amin und 0,077 g (0.55 mmol) 3-Methylsulfanyl-phenylamin in 2 ml Acetonitril wird mit 0,14 ml einer 4 molaren Lösung von Chlorwasserstoff in Dioxan und 0,17 ml Wasser versetzt und anschließend 24 Stunden bei 50°C gerührt. Nach dem Erkalten wird der Ansatz auf gesättigte Natriumhydrogencarbonat Lösung gegeben. Es wird mit Ethylacetat extrahiert. Die vereinigten organischen Phasen werden mit gesättigter Natriumchloridlösung gewaschen und über Natriumsulfat getrocknet. Nach Entfernen des Lösungsmittels und Umkristallisieren des Rückstands aus Ethylacetat/Hexan 9/1 erhält man 0.17 g (79% der Theorie) des Produktes.

¹H-NMR (300 MHz, DMSO): 9.20 (s, 1H), 7.99 (s, 1H), 7.77 (s, 1H), 7.37 (d, 1H), 7.14 (t, 1H), 6.75 (d, 1H), 6.47 (d, 1H), 4.34-4.27 (m, 1H), 2.40 (s, 3H), 1.21 (d, 6H).

### 2b) Herstellung des Endproduktes

Analog zum Beispiel 1 werden 0,15 g (0.42 mmol) 5-Bromo-N⁴-isopropyl-N²-(3-methylsulfanyl-phenyl)-pyrimidin-2,4-diamin mit 0,132 g (0,47 mmol) Chloramin-T Trihydrat in 3.0 ml Acetonitril umgesetzt (24 Stunden). Nach chromatographischer Reinigung (Kieselgel, Ethylacetat/Hexan mit Ethylacetat 0-100%, dann Ethylacetat/Methanol mit Methanol 5-10%) erhält man 0,14 g (63% der Theorie) des Produktes.

¹H-NMR (300 MHz, DMSO): 9.57 (s, 1H), 8.31 (s, 1H), 8.02 (s, 1H), 7.68 (d, 1H), 7.51 (d, 2H), 7.38 (t, 1H), 7.24 (d, 1H), 7.15 (d, 2H), 6.53 (d, 1H), 4.40-4.33 (m, 1H), 2.87 (s, 3H), 2.24 (s, 3H), 1.22-1.18 (m, 6H).

### Beispiel 3

### (RS)-S-(4-{[5-Brom-4-(cyclopropylamino)pyrimidin-2-yl]amino}phenyl)-S-methyl -N-(tolylsulfonyl)sulfimid

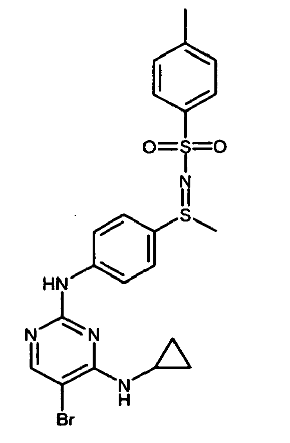

### 3a) Herstellung der Zwischenprodukte

### Verbindung 3.1

### (5-Brom-2-chlor-pyrimidin-4-yl)-cyclopropyl-amin

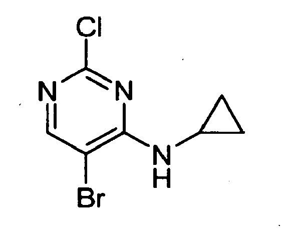

Analog zur Verbindung 1.1 wird eine Lösung von 1,0 g (4,39 mmol) 5-Brom-2,4-dichlor-pyrimidin in 15 ml Acetonitril mit 0,27 g (4,74 mmol) Cyclopropylamin in Gegenwart von 0,74 ml (5,3 mmol) Triethylamin umgesetzt. Nach chromatographischer Reinigung (Kieselgel, Ethylacetat/Hexan mit Ethylacetat 0-100%) erhält man 0,76 g (70% der Theorie) des Produktes.

¹H-NMR (300 MHz, DMSO): 8.21 (s, 1H), 7.70 (br, 1H), 2.81-2.74 (m, 1H), 0.74-0.59 (m, 4H).

### Verbindung 3.2

### 5-Bromo-N⁴-cyclopropyl-N²-(4-methylsulfanyl-phenyl)-pyrimidin-2,4-diamin

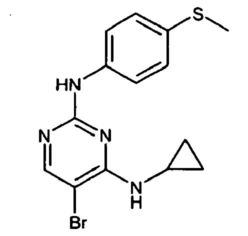

Analog zur Verbindung 2.1 werden 0,2 g (0.8 mmol) (5-Brom-2-chlor-pyrimidin-4-yl)-cyclopropyl-amin mit 0,102 g (0.73 mmol) 4-Methylsulfanyl-phenylamin in 3 ml Acetonitril in Gegenwart von 0,18 ml einer 4 molaren Lösung von Chlorwasserstoff in Dioxan sowie 0,23 ml Wasser umgesetzt. Man erhält 0,25 g (88% der Theorie) des Produktes.

¹H-NMR (400 MHz, DMSO): 9.28 (s, 1H), 7.97 (s, 1H), 7.80 (d, 2H), 7.16 (d, 2H), 7.01 (br, 1H), 2.78-2.75 (m, 1H), 2.38 (s, 3H), 0.78-0.76 (m, 2H), 0.62-0.59 (m, 2H).

### 3b) Herstellung des Endproduktes

Analog zum Beispiel 1 werden 0,21 g (0,6 mmol) 5-Bromo-N⁴-cyclopropyl-N²-(4-methylsulfanyl-phenyl)-pyrimidin-2,4-diamin mit 0,185 g (0,66 mmol) Chloramin-T Trihydrat in 5.0 ml Acetonitril umgesetzt (24 Stunden). Der Ansatz wird mit Ethylacetat verdünnt. Der entstandene Niederschlag wird abgesaugt und mit Ethylacetat gewaschen. Das Filtrat wird eingeengt und der Rückstand wird chromatographiert (Dichlormethan/Methanol mit Methanol 0-15%). Man erhält 0,23 g (74%) des Produktes.

¹H-NMR (400 MHz, DMSO): 9.75 (s, 1H), 8.04 (s, 1H), 8.01 (d, 2H), 7.59 (d, 2H), 7.52 (d, 2H), 7.20-7.18 (m, 3H), 2.87 (s, 3H), 2.79-2.74 (m, 1H), 2.27 (s, 3H), 0.80-0.75 (m, 2H), 0.66-0.62 (m, 2H).

### Beispiel 4

### (RS)-S-(3-{[5-Brom-4-(cyclopropylamino)pyrimidin-2-yl]amino}phenyl) -S-methyl-N-(tolylsulfonyl)sulfimid

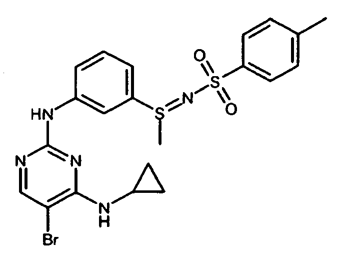

### 4a) Herstellung des Zwischenproduktes

### Verbindung 4.1 5-Bromo-N⁴-cyclopropyl-N²-(3-methylsulfanyl-phenyl)-pyrimidin-2,4-diamin

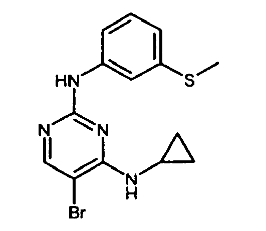

Analog zur Verbindung 2.1 werden 0,58 g (2,33 mmol) (5-Brom-2-chlor-pyrimidin-4-yl)-cyclopropyl-amin mit 0,296 g (2,12 mmol) 3-Methylsulfanyl-phenylamin in 7,0 ml Acetonitril in Gegenwart von 0,53 ml einer 4 molaren Lösung von Chlorwasserstoff in Dioxan sowie 0.67 ml Wasser umgesetzt. Man erhält 0,6 g (73% der Theorie) des Produktes.

¹H-NMR (300 MHz, DMSO): 9.28 (s, 1H), 7.99 (s, 1H), 7.94 (s, 1H), 7.51 (dd, 1H), 7.15 (t, 1H), 7.05 (d, 1H), 6.75 (d, 1H), 2.79-2.75 (m, 1H), 2.39 (s, 3H), 0.83-0.79 (m, 2H), 0.64-0.60 (m, 2H).

### 4b) Herstellung des Endproduktes

Analog zum Beispiel 1 werden 0,19 g (0.54 mmol) 5-Bromo-N⁴-cyclopropyl-N²-(3-methylsulfanyl-phenyl)-pyrimidin-2,4-diamin mit 0,168 g (0,59 mmol) Chloramin-T Trihydrat in 5,0 ml Acetonitril umgesetzt (24 Stunden). Nach chromatographischer Reinigung (Kieselgel, Ethylacetat/Hexan mit Ethylacetat 0-100%, dann Ethylacetat/Methanol mit Methanol 5-10%) erhält man 0,13 g (47% der Theorie) des Produktes.

¹H-NMR (400 MHz, DMSO): 9.65 (s, 1H), 8.48 (s, 1H), 8.03 (s, 1H), 7.78 (d, 1H), 7.50 (d, 2H), 7.40 (t, 1H), 7.25 (d, 1H), 7.16 (d, 2H), 7.12 (d, 1H), 2.94-2.92 (m, 1H), 2.86 (s, 3H), 2.25 (s, 3H), 0.82-0.79 (m, 2H), 0.67-0.55 (m, 2H).

### Beispiel 5

### (RS)-S-(4-{[4-(Isopropylamino)pyrimidin-2-yl]amino}phenyl)-S-methyl -N-(tolylsulfonyl)sulfimid

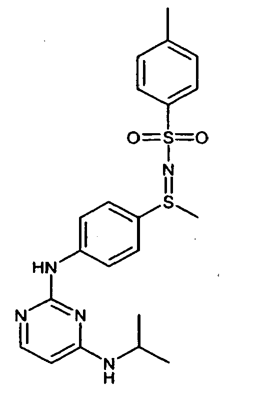

### 5a) Herstellung der Zwischenprodukte

### Verbindung 5.1

### (2-Chlor-pyrimidin-4-yl)-isopropyl-amin

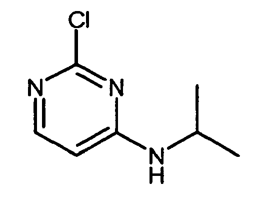

Analog zur Verbindung 1.1 wird eine Lösung von 0,347 g (2,33 mmol) 2,4-Dichlorpyrimidin in 15 ml Acetonitril mit 0,22 ml (2.52 mmol) Isopropylamin in Gegenwart von 0,39 ml (2,83 mmol) Triethylamin umgesetzt. Nach chromatographischer Reinigung (Kieselgel, Ethylacetat/Hexan mit Ethylacetat 0-100%) erhält man 0,26 g (65% der Theorie) des Produktes.

¹H-NMR (300 MHz, DMSO): 7.81 (br, 2H), 6.35 (br, 1H), 4.00 (br, 1H), 1.09 (d, 6H).

### Verbindung 5.2

### N⁴-isopropyl-N²-(4-methylsulfanyl-phenyl)-pyrimidin-2,4-diamin

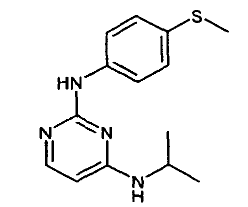

Analog zur Verbindung 2.1 werden 0,26 g (1,51 mmol) (2-Chlor-pyrimidin-4-yl)-islopropyl-amin mit 0,192 g (1,38 mmol) 4-Methylsulfanyl-phenylamin in 5 ml Acetonitril in Gegenwart von 0,34 ml einer 4 molaren Lösung von Chlorwasserstoff in Dioxan sowie 0,43 ml Wasser umgesetzt. Man erhält 0,31 g (75% der Theorie) des Produktes.

¹H-NMR (400 MHz, DMSO): 8.89 (s, 1H), 7.72-7.70 (m, 3H), 7.13 (d, 2H), 6.98 (br, 1H), 5.85 (d, 1H), 4.07 (br, 1H), 2.38 (s, 3H), 1.13 (d, 6H).

### 5b) Herstellung des Endproduktes

Analog zum Beispiel 1 werden 0,28 g (1,02 mmol) N⁴-Islopropyl-N²-(4-methylsulfanyl-phenyl)-pyrimidin-2,4-diamin mit 0,316 g (1,12 mmol) Chloramin-T Trihydrat in 9,0 ml Acetonitril umgesetzt (24 Stunden). Nach chromatographischer Reinigung (Kieselgel, Ethylacetat/Hexan mit Ethylacetat 0-100%, dann Ethylacetat/Methanol mit Methanol 5-10%) erhält man 0,26 g (58% der Theorie) des Produktes.

¹H-NMR (300 MHz, DMSO): 9.39 (s, 1H), 7.93 (d, 2H), 7.78 (d, 1H), 7.57-7.50 (m, 4H), 7.21-7.10 (m, 3H), 5.94 (d, 1H), 4.25-3.95 (br, 1H), 2.86 (s, 3H), 2.27 (s, 3H), 1.16-1.14 (m, 6H).

### Beispiel 6

### (RS)-S-{4-[(4-Amino-5-brompyrimidin-2-yl)amino]phenyl}-S-methyl -N-(tolylsulfonyl)sulfimid

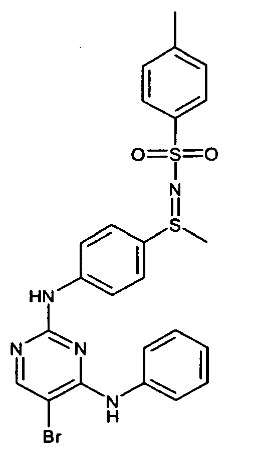

### 6a) Herstellung der Zwischenprodukte

### Verbindung 6.1

### (5-Brom-2-chlor-pyrimidin-4-yl)-phenyl-amin

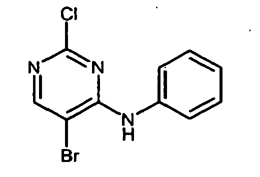

Analog zur Verbindung 1.1 wird eine Lösung von 0,3 g (1.32 mmol) 5-Brom-2,4-dichlor-pyrimidin in 5 ml Acetonitril mit 0,13 ml (1.42 mmol) Anilin in Gegenwart von 0,22 ml (1,6 mmol) Triethylamin umgesetzt. Nach chromatographischer Reinigung (Kieselgel, Ethylacetat/Hexan mit Methylacetat 0-100%) erhält man 0,289 g (77% der Theorie) des Produktes.

¹H-NMR (400 MHz, DMSO): 9.26 (s, 1H), 8.42 (s, 1H), 7.50 (d, 2H), 7.36 (t, 2H), 7.16 (t, 1H).

### Verbindung 6.2

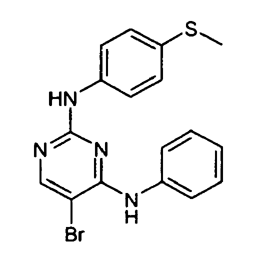

Analog zur Verbindung 2.1 werden 0,286 g (1,0 mmol) (5-Brom-2-chlor-pyrimidin-4-yl)-phenyl-amin mit 0,127 g (0,91 mmol) 4-Methylsulfanyl-phenylamin in 3,0 ml Acetonitril in Gegenwart von 0,23 ml einer 4 molaren Lösung von Chlorwasserstoff in Dioxan sowie 0,29 ml Wasser umgesetzt. Man erhält nach Umkristallisieren aus Ethylacetat 0,248 g (64% der Theorie) des Produktes.

¹H-NMR (400 MHz, DMSO): 9.33 (s, 1H), 8.59 (s, 1H), 8.18 (s, 1H), 7.59-7.51 (m, 4H), 7.34 (t, 2H), 7.14 (t, 1H), 7.05 (d, 2H), 2.37 (s, 3H).

### 6b) Herstellung des Endproduktes

Analog zum Beispiel 1 werden 0,232 g (0,6 mmol) 5-Bromo-N²-(4-methylsulfanylphenyl)-N⁴-phenyl-pyrimidin-2,4-diamin mit 0,186 g (0,66 mmol) Chloramin-T Trihydrat in 5,0 ml Acetonitril umgesetzt (24 Stunden). Nach chromatographischer Reinigung (Kieselgel, Ethylacetat/Hexan mit Ethylacetat 0-100%, dann Ethylacetat/Methanol mit Methanol 5-10%) erhält man 0,179 g (54% der Theorie) des Produktes.

¹H-NMR (400 MHz, DMSO): 9.76 (s, 1H), 8.76 (s, 1H), 8.22 (s, 1H), 7.71 (d, 2H), 7.54 (d, 2H), 7.50 (d, 2H), 7.45 (d, 2H), 7.37 (t, 2H),7.20-7.17 (m, 3H), 2.86 (s, 3H), 2.26 (s, 3H).

### Beispiel 7

### (RS)-S-[4-({5-Brom-4-[(2-hydroxyethyl)amino]pyrimidin-2-yl}amino)phenyl] -S-methyl-N-(tolylsutfonyl)sulfimid

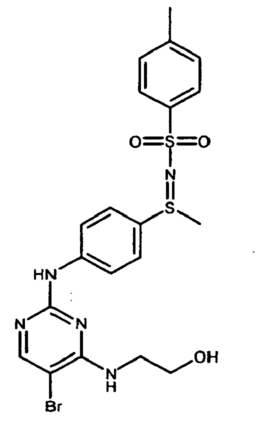

### 7a) Herstellung der Zwischenprodukte

### Verbindung 7.1

### 2-(5-Bromo-2-chloro-pyrimidin-4-ylamino)-ethanol

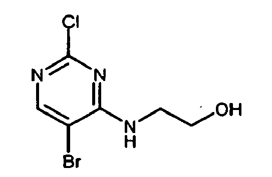

Analog zur Verbindung 1.1 wird eine Lösung von 0,34 g (1,49 mmol) 5-Brom-2,4-dichlor-pyrimidin in 5 ml Acetonitril mit 0,1 ml (1,61 mmol) Ethanolamin in Gegenwart von 0,25 ml (1,81 mmol) Triethylamin umgesetzt. Nach Umkristallisation aus Hexan/Ethylacetat 7/3 erhält man 0,28 g (74% der Theorie) des Produktes.

¹H-NMR (300 MHz, DMSO): 8.20 (s, 1H), 7.52 (t, 1H), 4.77 (t, 1H), 3.52-3.33 (m, 4H).

### Verbindung 7.2

### 2-[5-Bromo-2-(4-methylsulfanyl-phenylamino)-pyrimidin-4-ylamino]-ethanol

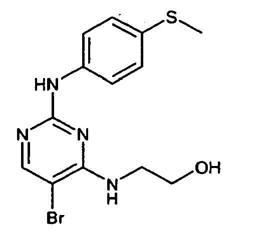

Analog zur Verbindung 2.1 werden 0,28 g (1,11 mmol) 2-(5-Bromo-2-chloro-pyrimidin-4-ylamino)-ethanol mit 0,14 g (1,01 mmol) 4-Methylsulfanyl-phenylamin in 4 ml Acetonitril in Gegenwart von 0,25 ml einer 4 molaren Lösung von Chlorwasserstoff in Dioxan sowie 0,32 ml Wasser umgesetzt. Man erhält 0,31 g (79% der Theorie) des Produktes.

¹H-NMR (400 MHz, DMSO): 9.21 (s, 1H), 7.97 (s, 1H), 7.65 (d, 2H), 7.15 (d, 2H), 6.74 (t, 1H), 4.75 (t, 1H), 3.57-3.53 (m, 2H), 3.47-3.42 (m, 2H), 2.38 (s, 3H).

### 7b) Herstellung des Endproduktes:

Analog zum Beispiel 1 werden 0,28 g (0,79 mmol) 2-[5-Bromo-2-(4-methylsulfanyl-phenylamino)-pyrimidin-4-ylamino]-ethanol mit 0,244 g (0,87 mmol) Chloramin-T Trihydrat in 7.0 ml Acetonitril umgesetzt (24 Stunden). Nach chromatographischer Reinigung (Kieselgel, Ethylacetat/Hexan mit Ethylacetat 0-100%, dann Ethylacetat/Methanol mit Methanol 5-20%) erhält man 0,25 g (61 % der Theorie) des Produktes.

¹H-NMR (400 MHz, DMSO): 9.67 (s, 1H), 8.05 (s, 1H), 7.87 (d, 2H), 7.59 (d, 2H), 7.52 (d, 2H), 7.20 (d, 2H), 6.90 (t, 1H),4.79 (t, 1H), 3.59-3.55 (m, 2H), 3.48-3.45 (m, 2H), 2.87 (s, 3H), 2.27 (s, 3H).

### Beispiel 8

### (RS)-S-{4-[(5-Brom-4-{[(R)-2-hydroxy-1-methylethyl]amino}pyrimidin-2-yl) amino]phenyl}-S-methyl-N-(tolylsulfonyl)sulfimid

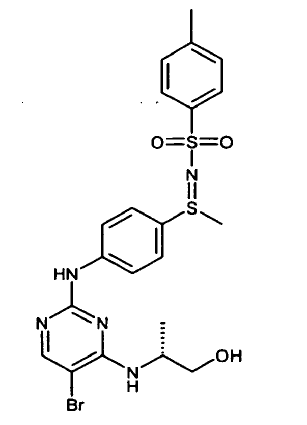

### 8a) Herstellung des Zwischenproduktes

### Verbindung 8.1

### (R) 2-[5-Bromo-2-(4-methylsulfanyl-phenylamino)-pyrimidin-4-ylamino]propan-1-ol

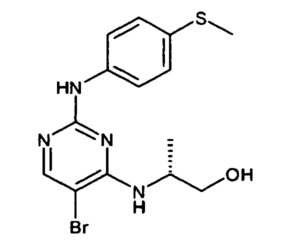

Analog zur Verbindung 2.1 werden 0,27 g (1,01 mmol) (R)-2-[(5-Brom-2-chlorpyrimidin-4-yl)amino]propan-1-ol (vgl. W02005037800) mit 0,128 g (0,92 mmol) 4-Methylsulfanyl-phenylamin in 4 ml Acetonitril in Gegenwart von 0,23 ml einer 4 molaren Lösung von Chlorwasserstoff in Dioxan sowie 0,29 ml Wasser umgesetzt. Man erhält 0,25 g (67% der Theorie) des Produktes.

¹H-NMR (300 MHz, DMSO):9.21 (s, 1H), 7.98 (s, 1H), 7.65 (d, 2H), 7.15 (d, 2H), 6.23 (d, 1H), 4.84 (t, 1H), 4.22-4.15 (m, 1H), 3.52-3.44 (m, 2H), 2.38 (s, 3H), 1.15 (d, 3H).

### 8b) Herstellung des Endproduktes:

Analog zum Beispiel 1 werden 0,24 g (0,65 mmol) (R) 2-[5-Bromo-2-(4-methylsulfanyl-phenylamino)-pyrimidin-4-ylamino]propan-1-ol mit 0,202 g (0,71 mmol) Chloramin-T Trihydrat in 6,0 ml Acetonitril umgesetzt (24 Stunden). Nach chromatographischer Reinigung (Ethylacetat/Methanol mit Methanol 10-25%) erhält man 0,247 g (71 % der Theorie) des Produktes.

¹H-NMR (400 MHz, DMSO): 9.67 (s, 1H), 8.06 (s, 1H), 7.86 (d, 2H), 7.59 (d, 2H), 7.52 (d, 2H), 7.20 (d, 2H), 6.39 (d, 1H), 4.87 (t, 1H), 4.23-4.17 (m, 1H), 3.52-3.43 (m, 2H), 2.87 (s, 3H), 2.27 (s, 3H), 1.17 (d, 3H).

### Beispiel 9

### (RS)-S{4-[(5-Brom-4{[(R)-2-hydroxy-1,2-dimethylpropyl]amino}pyrimidin-2-yl)amino]phenyl}-S-methyl-N-(tolylsulfonyl)sulfimid

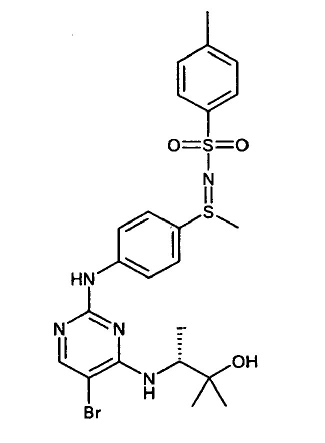

### 9a) Herstellung des Zwischenproduktes

### Verbindung 9.1

### (R)-3-[{5-Brom-2-(4-methylsulfanyl-phenylamino)-pyrimidin-4-yl)}amino]-2-methylbutan-2-ol

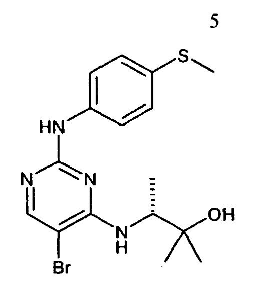

Analog zur Verbindung 2.1 werden 0,3g (1,02 mmol) (R)-3-[(5-Brom-2-chlorpyrimidin-4-yl)aminol-2-methylbutan-2-ol (vgl. WO2005037800) mit 0,129 g (0,93 mmol) 4-Methylsulfanyl-phenylamin in 3 ml Acetonitril in Gegenwart von 0,23 ml einer 4 molaren Lösung von Chlorwasserstoff in Dioxan sowie 0,29 ml Wasser umgesetzt. Nach chromatographischer Reinigung (Kieselgel, Ethylacetat/Hexan mit Ethylacetat 0-100%) erhält man 0,29 g (72% der Theorie) des Produktes.

¹H-NMR (300 MHz, DMSO): 9.22 (s, 1H), 8.00 (s, 1H), 7.64 (d, 2H), 7.15 (d, 2H), 5.95 (d, 1H), 4.78 (s, 1H), 4.07-3.98 (m, 1H), 2.39 (s, 3H), 1.16-1.08 (m, 9H).

### 9b) Herstellung des Endproduktes

Analog zum Beispiel 1 werden 0,28 g (0.7 mmol) (R)-3-[{5-Brom-2-(4-methylsulfanyl-phenylamino)-pyrimidin-4-yl)}amino]-2-methylbutan-2-ol mit 0,218 g (0.78 mmol) Chloramin-T Trihydrat in 6,0 ml Acetonitril umgesetzt (24 Stunden). Nach chromatographischer Reinigung (Kieselgel, Ethylacetat/Hexan mit Ethylacetat 0-100%, dann Ethylacetat/Methanol mit Methanol 5-10%) erhält man 0,21 g (53% der Theorie) des Produktes.

¹H-NMR (400 MHz, DMSO): 9.68 (s, 1H), 8.08 (s, 1H), 7.86 (d, 2H), 7.60 (d, 2H), 7.52 (d, 2H), 7.19 (d, 2H), 6.07 (d, 1H), 4.83 (s, 1H), 4.07-4.01 (m, 1H), 2.87 (s, 3H), 2.27 (s, 3H), 1.16-1.14 (m, 6H), 1.10 (s, 3H).

### Beispiel 10

### (RS)-S-{4-[(5-Brom-4-{[(R)-2-hydroxy-1,2-dimethylpropyl]amino}pyrimidin-2-yl)amino]phenyl}-S-methyl-N-[(5-methyl-2-pyridyl)sulfonyl]sulfimid

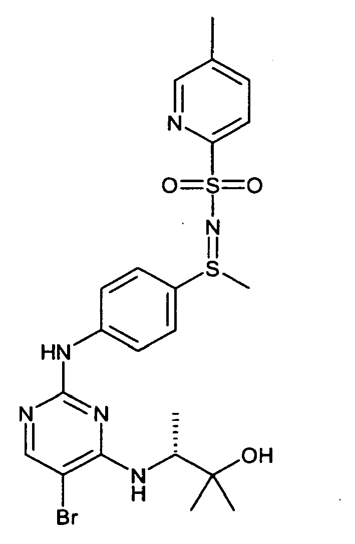

### 10a) Herstellung des Zwischenproduktes

Zwischenprodukt ist Verbindung 9.1

### 10b) Herstellung des Endproduktes

300 mg (0,76 mmol) (R)-3-[{5-Brom-2-(4-methylsulfanyl-phenylamino)-pyrimidin-4-yl)}amino]-2-methylbutan-2-ol (Verbindung 9.1), 192 mg (1,12 mmol) 5-Methyl-pyridin-2-sulfonamid, 265 mg (1,21 mmol) lodosobenzol und 266 mg (0,76 mmol) Eisen(III)acetylacetonat werden in einen Kolben eingewogen und mit 8 ml Acetonitril versetzt. Der Ansatz wird 62 Stunden bei Raumtemperatur gerührt und anschließend einrotiert. Der verbleibende Rückstand wird chromatographisch (Dichlormethan /Ethanol 8:2) gereinigt. Man erhält 100 mg (0,18 mmol; Ausbeute: 23%) des Produktes.

Alternativ kann das Endprodukt folgendermaßen hergestellt werden:
300 mg (0,76 mmol) (R)-3-[{5-Brom-2-(4-methylsulfanyl-phenylamino)-pyrimidin-4-yl)}amino]-2-methylbutan-2-ol (Verbindung 9.1), 260 mg (1,51 mmol) 5-Methyl-pyridin-2-sulfonamid, 121 mg (3,00 mmol) Magnesiumoxid, 374 mg (1,16 mmol) lodbenzol-diacetat und 33 mg (0,08 mmol) Rhodium(II)acetat Dimer werden in einen Kolben eingewogen und mit 10 ml Dichlormethan versetzt. Der Ansatz wird 19 Stunden bei Raumtemperatur gerührt und anschließend einrotiert. Der verbleibende Rückstand wird chromatographisch (Dichlormethan / Ethanol 9:1) gereinigt. Man erhält 42 mg (0,08 mmol; Ausbeute: 10%) des Produktes.

¹H-NMR (400 MHz, DMSO): 9.70 (s, 1H), 8.37 (br, 1H), 8.09 (s, 1H), 7.91 (m, 2H), 7.69 (m, 4H), 6.06 (d, 1H), 4.83 (s, 1H), 4.04 (m, 1H), 2.98 (s, 3H), 2.29 (s, 3H), 1.12 (m, 9H).
MS: 567 (ES+)

### Beispiel 11

### (RS)-S-{4-[(5-Brom-4-{[(R)-2-hydroxy-1,2-dimethylpropyl]amino}pyrimidin-2-yl)amino]phenyl}-N-[(4-chlor-3-pyridyl)sulfonyl]-S-methylsulfimid

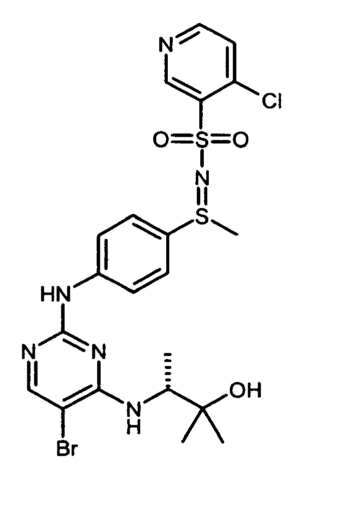

### Herstellung des Endproduktes

285 mg (0,76 mmol) (R)-3-[{5-Brom-2-(4-methylsulfanyl-phenylamino)-pyrimidin-4-yl)}amino]-2-methylbutan-2-ol (Verbindung 9.1), 207 mg (1,08 mmol) 4-Chlor-pyridin-3-sulfonamid, 252 mg (1,15 mmol) lodosobenzol und 253 mg (0,76 mmol) Eisen(III)acetylacetonat werden in einen Kolben eingewogen und mit 8 ml Acetonitril versetzt. Der Ansatz wird 144 Stunden bei Raumtemperatur gerührt und anschließend einrotiert. Der verbleibende Rückstand wird chromatographisch (Dichlormethan / Ethanol 8:2) gereinigt. Man erhält 38 mg (0,07 mmol; Ausbeute 9 %) des Produktes.

¹H-NMR (DMSO): 9.70 (s, 1H), 8.86 (m, 1H), 8.53 (m, 1H), 8.09 (s, 1H), 7.84 (m, 2H), 7.63 (m, 2H), 7.54 (m, 1H), 6.07 (d, 1H), 4.83 (s, 1H), 4.04 (m, 1H), 3.00 (s, 3H), 1.12 (m, 9H)

MS: 587 (ES+).

### Beispiel 12

### (RS)-S-{4-[(5-Brom-4-{[(R)-2-hydroxy-1,2-dimethylpropyl]amino}pyrimidin-2-yl)amino]phenyl}-N-[(4-methoxyphenyl)sulfonyl]-S-methylsulfimid

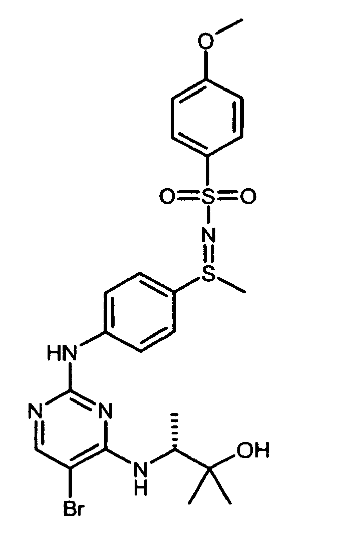

### Herstellung des Endproduktes

287 mg (0,76 mmol) (R)-3-[{5-Brom-2-(4-methylsulfanyl-phenylamino)-pyrimidin-4-yl)}amino]-2-methylbutan-2-ol (Verbindung 9.1), 202 mg (1,08 mmol) 4-Methoxybenzolsulfonamid, 254 mg (1,16 mmol) lodosobenzol und 255 mg (0,72 mmol) Eisen(III)acetylacetonat werden in einen Kolben eingewogen und mit 8 ml Acetonitril versetzt. Der Ansatz wird 90 Stunden bei Raumtemperatur gerührt und anschließend einrotiert. Der verbleibende Rückstand wird chromatographisch (Dichlormethan / Ethanol 8:2) gereinigt. Man erhält 23 mg (0,04 mmol; Ausbeute: 6 %) des Produktes.

¹H-NMR (DMSO): 9.69 (s, 1H), 8.08 (s, 1H), 7.87 (m, 2H), 7.59 (m, 4H), 6.92 (m, 2H), 6.07 (d, 1H), 4.84 (s, 1H), 4.03 (m, 1H), 3.72 (s, 3H), 2.87 (s, 3H), 1.12 (m, 9H).

MS: 582 (ES+).

### Beispiel 13

### (RS)-N-(Benzylsulfonyl)-S-{4-[(5-brom-4-{[(R)-2-hydroxy-1,2-dimethylpropyl]amino}pyrimidin-2-yl)amino]phenyl}-S-methylsulfimid

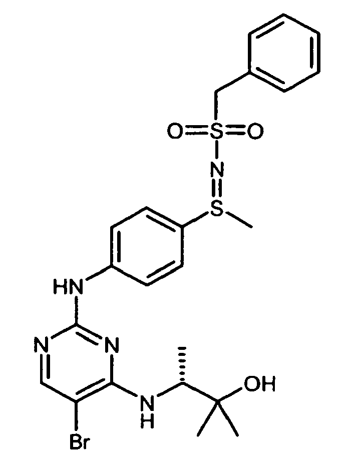

### Herstellung des Endproduktes

287 mg (0,72 mmol) (R)-3-[{5-Brom-2-(4-methylsulfanyl-phenylamino)-pyrimidin-4-yl)}amino]-2-methylbutan-2-ol (Verbindung 9.1), 185 mg (1,08 mmol) Phenylmethansulfonamid, 254 mg (1,16 mmol) lodosobenzol und 255 mg (0,72 mmol) Eisen(III)acetylacetonat werden in einen Kolben eingewogen und mit 8 ml Acetonitril versetzt. Der Ansatz wird 142 Stunden bei Raumtemperatur gerührt und anschließend einrotiert. Der verbleibende Rückstand wird chromatographisch (Dichlormethan / Ethanol 8:2) gereinigt. Man erhält 54 mg (0,10 mmol; Ausbeute: 13 %) des Produktes.

¹H-NMR (DMSO): 9.69 (s, 1H), 8.08 (s, 1H), 7.90 (m, 2H), 7.62 (m, 2H), 7.26 (m, 5H), 6.07 (d, 1H), 4.82 (s, 1H), 4.13 (s, 2H), 4.03 (m, 1H), 2.77 (s, 3H), 1.12 (m, 9H).

MS: 566 (ES+).

### Beispiel 14

### (RS)-S-{4-[(5-Brom-4-{[(R)-2-hydroxy-1,2-dimethylpropyl]amino}pyrimidin-2-yl)amino]phenyl}-S-methyl-N-[(trifluormethyl)sulfonyl]sulfimid

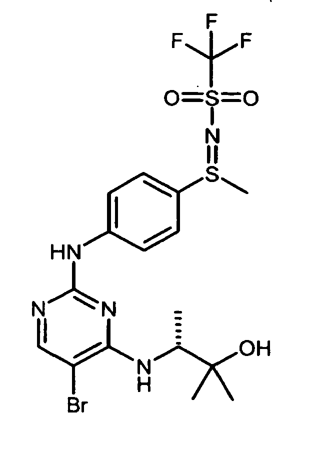

### Herstellung des Endproduktes

294 mg (0,74 mmol) (R)-3-[{5-Brom-2-(4-methylsulfanyl-phenylamino)-pyrimidin-4-yl)}amino]-2-methylbutan-2-ol (Verbindung 9.1), 165 mg (1,11 mmol) Trifluormethylsulfonamid, 260 mg (1,18 mmol) lodosobenzol und 261 mg (0,74 mmol) Eisen(III)acetylacetonat werden in einen Kolben eingewogen und mit 8 ml Acetonitril versetzt. Der Ansatz wird 142 Stunden bei Raumtemperatur gerührt und anschließend einrotiert. Der verbleibende Rückstand wird chromatographisch (Dichlormethan / Ethanol 8:2) gereinigt. Man erhält 12 mg (0,02 mmol; Ausbeute: 3%) des Produktes.

Alternativ kann das Endprodukt folgendermaßen hergestellt werden:
200 mg (0,50 mmol) (R)-3-[{5-Brom-2-(4-methylsulfanyl-phenylamino)-pyrimidin-4-yl)}amino]-2-methylbutan-2-ol (Verbindung 9.1) und 150 mg (1,01 mmol) Trifluormethylsulfonamid werden mit 7,2 ml Dichlormethan versetzt. Der Ansatz wird mit 101 mg (2,52 mmol) Magnesiumoxid, 22 mg (0,05 mmol) Rhodium(II)acetat Dimer und 324 mg (1,01 mmol) Iodbenzol-diacetat versetzt. Der Ansatz wird 2 Stunden bei Raumtemperatur gerührt und anschließend einrotiert. Der verbleibende Rückstand wird zunächst chromatographisch (Dichlormethan / Ethanol 9:1) gereinigt. Man erhält 28 mg (0,05 mmol; Ausbeute: 10%) des Produktes.

¹H-NMR (DMSO): 9.81 (s, 1H), 8.11 (s, 1H), 7.97 (m, 2H), 7.82 (m, 2H), 6.09 (d, 1H), 4.62 (br, 1H), 4.05 (m, 1H), 3.17 (s, 3H), 1.12 (m, 9H).
MS: 544 (ES+).

### Beispiel 15

### (RS)-S-{4-[(5-Brom-4-{[(R)-2-hydroxy-1,2-dimethylpropyl]amino}pyrimidin-2-yl)amino]phenyl}-S-methyl-N-(phenylsulfonyl)sulfimid

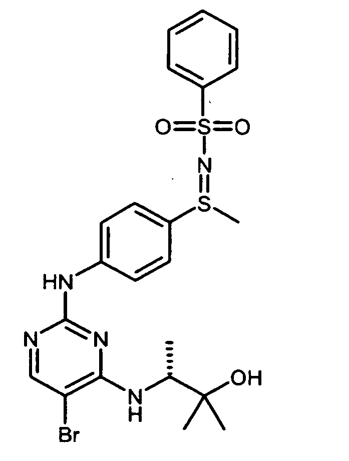

### Herstellung des Endproduktes

294 mg (0,74 mmol) (R)-3-[{5-Brom-2-(4-methylsulfanyl-phenylamino)-pyrimidin-4-yl)}amino]-2-methylbutan-2-ol (Verbindung 9.1), 174 mg (1,08 mmol) Phenylsulfonamid, 260 mg (1,18 mmol) Iodosobenzol und 261 mg (0,74 mmol) Eisen(III)acetylacetonat werden in einen Kolben eingewogen und mit 8 ml Acetonitril versetzt. Der Ansatz wird 91 Stunden bei Raumtemperatur gerührt und anschließend einrotiert. Der verbleibende Rückstand wird chromatographisch (Dichlormethan /Ethanol 8:2) gereinigt. Man erhält 64 mg (0,12 mmol; Ausbeute: 16%) des Produktes.

¹H-NMR (DMSO): 9.68 (s, 1H), 8.08 (s, 1H), 7.88 (m, 2H), 7.61 (m, 4H), 7.41 (m, 3H), 6.06 (d, 1H), 4.82 (s, 1H), 4.03 (m, 1H), 2.89 (s, 3H), 1.11 (m, 9H).
MS: 552 (ES+).

### Beispiel 16 (RS)-S-{4-[(5-Brom-4-{[(R)-2-hydroxy-1,2-dimethylpropyl]amino}pyrimidin-2-yl)amino]phenyl}-S-methyl-N-{[4-(trifluormethoxy)phenyl]sulfonyl}sulfimid

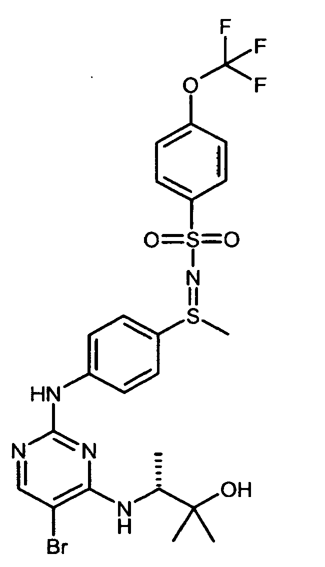

### Herstellung des Endproduktes

246 mg (0,62 mmol) (R)-3-[{5-Brom-2-(4-methylsulfanyl-phenylamino)-pyrimidin-4-yl)}amino]-2-methylbutan-2-ol (Verbindung 9.1), 224 mg (0,93 mmol) 4-Trifluormethoxy-benzolsulfonamid, 217 mg (1,00 mmol) lodosobenzol und 218 mg (0,62 mmol) Eisen(III)acetylacetonat werden in einen Kolben eingewogen und mit 7 ml Acetonitril versetzt. Der Ansatz wird 91 Stunden bei Raumtemperatur gerührt und anschließend einrotiert. Der verbleibende Rückstand wird chromatographisch (Dichlormethan / Ethanol 8:2) gereinigt. Man erhält 27 mg (0,12 mmol; Ausbeute: 7%) des Produktes.

¹H-NMR (DMSO): 9.73 (s, 1H), 8.13 (s, 1H), 7.89 (m, 2H), 7.78 (m, 2H), 7.63 (m, 2H), 7.39 (m, 2H), 6.11 (d, 1H), 4.87 (s, 1H), 4.07 (m, 1H), 2.99 (s, 3H), 1.11 (m, 9H).
MS: 636 (ES+).

### Beispiel 17

### (RS)-S-{4-[(5-Brom-4-{[(R)-2-hydroxy-1,2-dimethylpropyl]amino}pyrimidin-2-yl)amino]phenyl}-N-[(5-chlor-2-thienyl)sulfonyl]-S-methylsulfimid

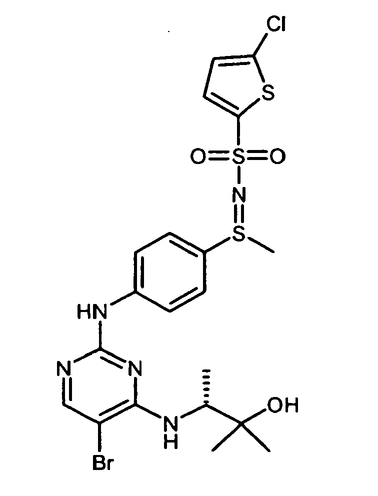

### Herstellung des Endproduktes

246 mg (0,62 mmol) (R)-3-[{5-Brom-2-(4-methylsulfanyl-phenylamino)-pyrimidin-4-yl)}amino]-2-methylbutan-2-ol (Verbindung 9.1), 184 mg (0,93 mmol) 5-Chlor-thiophen-2-sulfonamid, 218 mg (1,00 mmol) lodosobenzol und 218 mg (0,62 mmol) Eisen(III)acetylacetonat werden in einen Kolben eingewogen und mit 7 ml Acetonitril versetzt. Der Ansatz wird 91 Stunden bei Raumtemperatur gerührt und anschließend einrotiert. Der verbleibende Rückstand wird chromatographisch (Dichlormethan /Ethanol 8:2) gereinigt. Man erhält 6 mg (0,01 mmol; Ausbeute: 2%) des Produktes.

¹H-NMR (DMSO): 9.73 (s, 1H), 8.09 (s, 1H), 7.91 (m, 2H), 7.67 (m, 2H), 7.18 (m, 1H), 7.01 (m, 1H), 6.07 (d, 1H), 4.83 (s, 1H), 4.04 (m, 1H), 2.99 (s, 3H), 1.11 (m, 9H).
MS: 592 (ES+).

### Beispiel 18

### (RS)-S-{4-[(5-Brom-4{[(R)-2-hydroxy-1,2-dimethylpropyl]amino}pyrimidin-2-yl)amino]phenyl}-N-mesyl-S-methylsulfimid

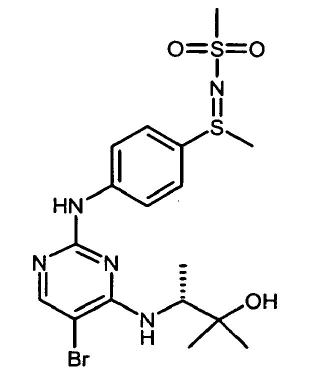

### Herstellung des Endproduktes

287 mg (0,72 mmol) (R)-3-[{5-Brom-2-(4-methylsulfanyl-phenylamino)-pyrimidin-4-yl)}amino]-2-methylbutan-2-ol (Verbindung 9.1), 103 mg (1,08 mmol) Methylsulfonamid, 254 mg (1,16 mmol) lodosobenzol und 255 mg (0,72 mmol) Eisen(III)acetylacetonat werden in einen Kolben eingewogen und mit 8 ml Acetonitril versetzt. Der Ansatz wird 88 Stunden bei Raumtemperatur gerührt und anschließend einrotiert. Der verbleibende Rückstand wird chromatographisch (Dichlormethan /Ethanol 8:2) gereinigt. Man erhält 12 mg (0,03 mmol; Ausbeute: 3%) des Produktes.

¹H-NMR (DMSO): 9.70 (s, 1H), 8.08 (s, 1H), 7.94 (m, 2H), 7.73 (m, 2H), 6.07 (d, 1H), 4.82 (s, 1H), 4.05 (m, 1H), 2.95 (s, 3H), 2.71 (s, 3H), 1.11 (m, 9H).
MS: 490 (ES+).

### Beispiel 19

### (RS)-N-{[5-(Acetylamino)-1,3,4-thiadiazol-2-yl]sulfonyl}-S-{4-[(5-brom-4-{[(R) -2-hydroxy-1,2-dimethylpropyl]amino}pyrimidin-2-yl)amino]phenyl}-S-methylsulfimid

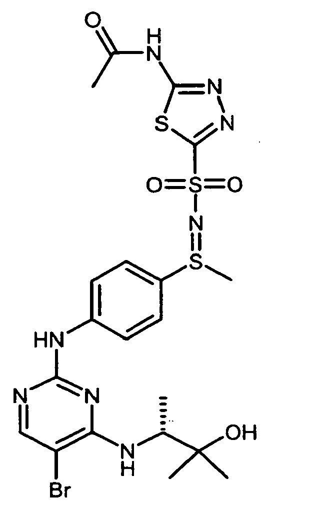

### Herstellung des Endproduktes

250 mg (0,63 mmol) (R)-3-[{5-Brom-2-(4-methylsulfanyl-phenylamino)-pyrimidin-4-yl)}amino]-2-methylbutan-2-ol (Verbindung 9.1), 210 mg (0,94 mmol) N-(5-Sulfamoyl-[1,3,4]thiadiazol-2-yl)-acetamid, 221 mg (1,01 mmol) lodosobenzol und 222 mg (0,63 mmol) Eisen(III)acetylacetonat werden in einen Kolben eingewogen und mit 6 ml Acetonitril versetzt. Der Ansatz wird 96 Stunden bei Raumtemperatur gerührt und anschließend einrotiert. Der verbleibende Rückstand wird chromatographisch (Dichlormethan / Ethanol 8:2) gereinigt. Man erhält 9 mg (0,02 mmol; Ausbeute: 2%) des Produktes.

¹H-NMR (DMSO): 12.84 (s, 1H), 9.81 (s, 1H), 8.15 (s, 1H), 7.97 (m, 2H), 7.77 (m, 2H), 6.17 (d, 1H), 4.05 (m, 1H), 3.12 (s, 3H), 2.20 (s, 3H), 1.11 (m, 9H).

### Beispiel 20

### (RS)-S-{4-[(5-Brom-4-{[(R)-2-hydroxy-1,2-dimethylpropyl]amino}pyrimidin-2-yl)amino]phenyl}-N-(ethylsulfonyl)-S-methylsulfimid

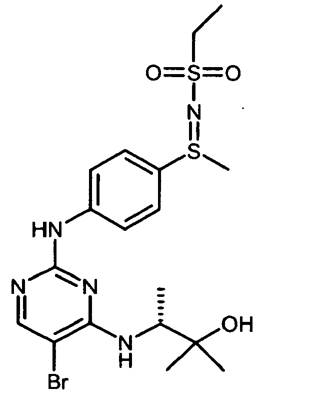

### Herstellung des Endproduktes

250 mg (0,63 mmol) (R)-3-[{5-Brom-2-(4-methylsulfanyl-phenylamino)-pyrimidin-4-yl)}amino]-2-methylbutan-2-ol (Verbindung 9.1), 103 mg (0,94 mmol) Ethylsulfonamid, 221 mg (1,01 mmol) lodosobenzol und 222 mg (0,63 mmol) Eisen(III)acetylacetonat werden in einen Kolben eingewogen und mit 6 ml Acetonitril versetzt. Der Ansatz wird 130 Stunden bei Raumtemperatur gerührt und anschließend einrotiert. Der verbleibende Rückstand wird chromatographisch (Dichlormethan / Ethanol 8:2) gereinigt. Man erhält 24 mg (0,05 mmol; Ausbeute: 8%) des Produktes.

¹H-NMR (DMSO): 9.74 (s, 1H), 8.13 (s, 1H), 7.97 (m, 2H), 7.77 (m, 2H), 6.13 (d, 1H), 4.85 (br, 1H), 4.09 (m, 1H), 3.00 (s, 3H), 2.82 (q, 2H), 1.11 (m, 12H).

### Beispiel 21

### (RS)-S-{4-[(5-Brom-4-{[(R)-2-hydroxy-1,2-dimethylpropyl]amino}pyrimidin-2-yl)amino]phenyl}-S-methyl-N-(propylsulfonyl)sulfimid

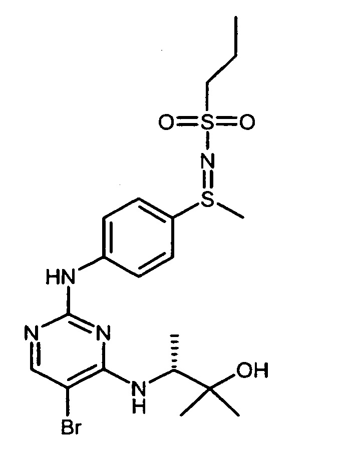

### Herstellung des Endproduktes

250 mg (0,63 mmol) (R)-3-[{5-Brom-2-(4-methylsulfanyl-phenylamino)-pyrimidin-4-yl)}amino]-2-methylbutan-2-ol (Verbindung 9.1), 116 mg (0,94 mmol) 1-Propylsulfonamid, 221 mg (1,01 mmol) lodosobenzol und 222 mg (0,63 mmol) Eisen(III)acetylacetonat werden in einen Kolben eingewogen und mit 6 ml Acetonitril versetzt. Der Ansatz wird 250 Stunden bei Raumtemperatur gerührt und anschließend einrotiert. Der verbleibende Rückstand wird chromatographisch (Dichlormethan / Ethanol 8:2) gereinigt. Man erhält 55 mg (0,11 mmol; Ausbeute: 17%) des Produktes.

¹H-NMR (DMSO): 9.85 (s, 1H), 8.12 (s, 1H), 7.91 (m, 2H), 7.75 (m, 2H), 6.27 (d, 1H), 4.04 (m, 1H), 2.95 (s, 3H), 2.75 (m, 2H), 1.56 (m, 2H), 1.11 (m, 9H), 0.85 (tr, 3H).

### Beispiel 22

### (RS)-S-{4-[(5-Brom-4-{[(R)-2-hydroxy-1,2-dimethylpropyl]amino}pyrimidin-2-yl)amino]phenyl}-N-(tert-butylsulfonyl)-S-methylsulfimid

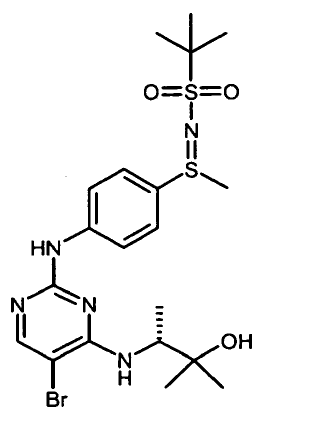

### Herstellung des Endproduktes

250 mg (0,63 mmol) (R)-3-[{5-Brom-2-(4-methylsulfanyl-phenylamino)-pyrimidin-4-yl)}amino]-2-methylbutan-2-ol (Verbindung 9.1), 129 mg (0,94 mmol) tert. Butylsulfonamid, 221 mg (1,01 mmol) lodosobenzol und 222 mg (0,63 mmol) Eisen(III)acetylacetonat werden in einen Kolben eingewogen und mit 6 ml Acetonitril versetzt. Der Ansatz wird 250 Stunden bei Raumtemperatur gerührt und anschließend einrotiert. Der verbleibende Rückstand wird chromatographisch (Dichlormethan / Ethanol 8:2) gereinigt. Man erhält 15 mg (0,03 mmol; Ausbeute: 4%) des Produktes.

¹H-NMR (DMSO): 9.74 (s, 1H), 8.09 (s, 1H), 7.92 (m, 2H), 7.74 (m, 2H), 6.11 (d, 1H), 4.04 (m, 1H), 2.94 (s, 3H), 1.11 (m, 18H).

### Verfahrensvariante 2 (Beispiele 23 bis 34)

### Beispiel 23

### (RS)-S-{4-[(4-{[2-(Acetylamino)ethyl]amino}-5-brompyrimidin-2-yl)amino]phenyl}-S-methyl-N-(tolylsulfonyl)sulfimid

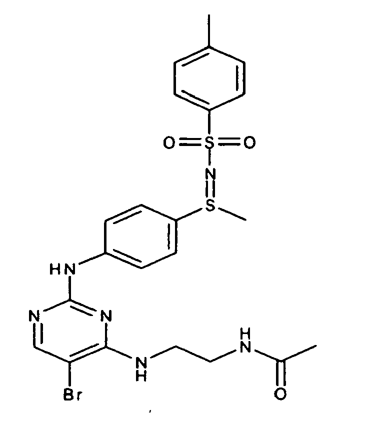

### 23a) Herstellung der Zwischenprodukte

### Verbindung 23.1

### N-[2-(5-Brom-2-chlor-pyrimidin-4-ylamino)-ethyl]-acetamid

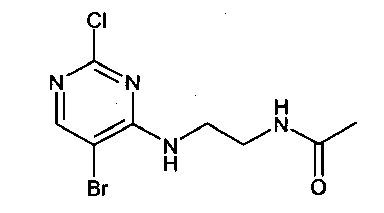

Eine Lösung von 300 mg (1,32 mmol) 5-Brom-2,4-dichlor-pyrimidin in 4,2 ml Acetonitril wird bei Raumtemperatur mit 0,14 ml (1,42 mmol) N-(2-Amino-ethyl)-acetamid und 0,22 ml (1,60 mmol) Triethylamin versetzt. Der Ansatz wird 24 Stunden bei Raumtemperatur gerührt und anschließend mit Essigester verdünnt. Man wäscht mit gesättigter NaCl-Lösung, 10%iger Zitronensäure-Lösung und gesättigter NaHCO₃-Lösung. Die organische Phase wird getrocknet (Na₂SO₄), filtriert und eingeengt. Der erhaltene Rückstand wird aus Essigester umkristallisiert. Man erhält 294 mg (1,00 mmol; Ausbeute: 75 %) des Produktes.

¹H-NMR (DMSO): 8.21 (s, 1H), 7.98 (tr, 1H), 7.74 (tr, 1H), 3.37 (m, 2H), 3.20 (m, 2H), 1.76 (s, 3H).

### Verbindung 23.2

### (RS)-S-Methyl-S-(4-nitrophenyl)-N-tosylsulfimid

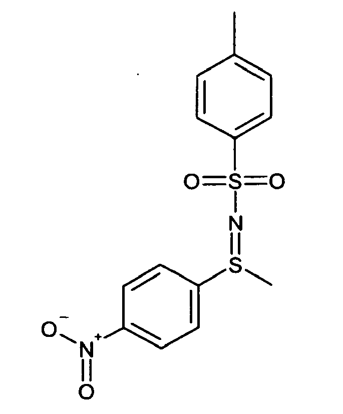

5,0 g (29,6 mmol) 1-Methylsulfanyl-4-nitro-benzol in 120 ml Acetonitril werden bei Raumtemperatur mit 10,0 g (35,3 mmol) Chloramin-T Trihydrat (Aldrich) versetzt. Der Ansatz wird 4 Stunden bei Raumtemperatur gerührt und anschließend mit Essigester verdünnt. Der Ansatz wird abgenutscht und der Filterkuchen mit Essigester nachgewaschen. Die vereinten organischen Phasen werden getrocknet (Na₂SO₄), filtriert und eingeengt. Der erhaltene Rückstand wird aus Methanol umkristallisiert. Man erhält 2,2 g (6,5 mmol; Ausbeute: 22 %) des Produktes.

¹H-NMR (DMSO): 8.39 (m, 2H), 8.03 (m, 2H), 7.57 (m, 2H), 7.21 (m, 2H), 3.01 (s, 3H), 2.27 (s, 3H).
MS: 338 (EI).

### Verbindung 23.3

### (RS)-S-(4-Aminophenyl)-S-methyl-N-tosylsulfimid

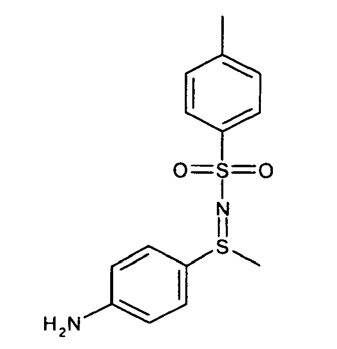

Eine Lösung von 500 mg (1,48 mmol) (RS)-S-Methyl-S-(4-nitrophenyl)-N-tosylsulfimid in 60 ml Ethanol wird mit 500 mg Raney Nickel (50%, wasserfeucht) versetzt und bei Raumtemperatur 2 Stunden unter einer Wasserstoffatmosphäre bei Normaldruck hydriert. Die Wasserstoffaufnahme beträgt 110 ml. Der Ansatz wird filtriert und eingeengt. Der erhaltene Rückstand wird chromatographisch (Dichlormethan / Ethanol 9:1) gereinigt. Man erhält 124 mg (0,40 mmol; Ausbeute: 27 %) des Produktes.

¹H-NMR (DMSO): 7.49 (m, 2H), 7.33 (m, 2H), 7.19 (m, 2H), 6.57 (m, 2H), 5.94 (s, 2H), 2.78 (s, 3H), 2.29 (s, 3H).
MS: 309 (ES).

### 23b) Herstellung des Endproduktes

Ein Ansatz von 65 mg (0,22 mmol) N-[2-(5-Brom-2-chlor-pyrimidin-4-ylamino)-ethyl]-acetamid und 62 mg (0,20 mmol) (RS)-S-(4-Aminophenyl)-S-methyl-N-tosylsulfimid in 3 ml Isopropanol wird mit 0,005 ml einer 4 molaren Lösung von Chlorwasserstoff in Dioxan versetzt und 18 Stunden bei 70°C gerührt. Der Ansatz wird mit weiteren 0,005 ml der 4 molaren Lösung von Chlorwasserstoff in Dioxan versetzt und weitere 72 Stunden bei 70°C gerührt. Der Ansatz wird einrotiert und der erhaltene Rückstand wird chromatographisch (Dichlormethan / Ethanol 8:2) gereinigt. Man erhält 28 mg (0,05 mmol; Ausbeute: 25 %) des Produktes.

¹H-NMR (DMSO): 9.64 (s, 1H), 8.08 (s, 1H), 7.88 (m, 2H), 7.59 (m, 2H), 7.51 (m, 2H), 7.19 (m, 2H), 6.58 (d, 1H), 4.30 (m, 1H), 2.87 (s, 3H), 2.28 (s, 3H), 1.22 (d, 6H).
MS: 522 (ES+).

### Beispiele 24 bis 32

### a) Herstellung von Zwischenprodukten

### Verbindung 24.1

### (RS)-S-Methyl-S-(4-nitrophenyl)-N-[(5-methyl-2-pyridyl)sulfonyl]sulfimid

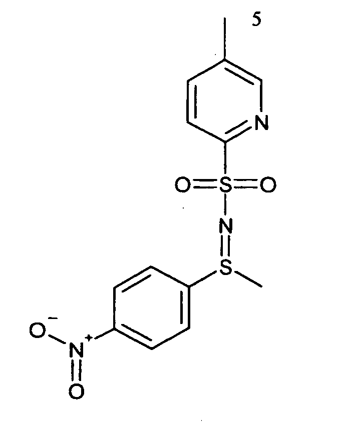

Zu einer Suspension von 300 mg (1,77 mmol) 1-Methylsulfanyl-4-nitro-benzol, 610 mg (3,55 mmol) 5-Methyl-2-pyridinsulfonamid, 285 mg (7,10 mmol) Magnesiumoxid und 78 mg (0,18 mmol) Rhodium(II)acetat Dimer in 12 ml Dichlormethan wird bei Raumtemperatur 879 mg (2,73 mmol) Jodosobenzoldiacetat gegeben. Der Ansatz wird 24 h gerührt und anschließend eingeengt. Der erhaltene Rückstand wird chromatographisch (Dichlormethan / Ethanol 95:5) gereinigt. Man erhält 326 mg (0,96 mmol; Ausbeute: 54 %) des Produktes.

¹H-NMR (DMSO): 8.41 (m, 2H), 8.31 (m, 1H), 8.12 (m, 2H), 7.76 (m, 1H), 7.70 (m, 1H), 3.11 (s, 3H), 2.29 (s, 3H).
MS: 340 (ES+).

### Verbindung 24.2

### (RS)-S-Methyl-S-(4-nitrophenyl)-N-[(4-chlor-3-pyridyl)sulfonyl]sulfimid

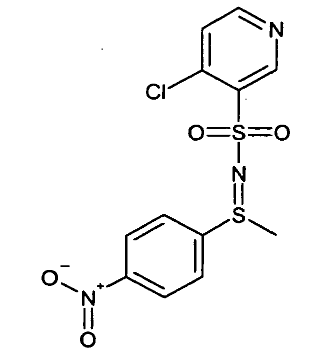

Zu einer Suspension von 300 mg (1,77 mmol) 1-Methylsulfanyl-4-nitro-benzol, 683 mg (3,55 mmol) 5-Chlor-3-pyridinsulfonamid, 285 mg (7,10 mmol) Magnesiumoxid und 78 mg (0,18 mmol) Rhodium(II)acetat Dimer in 12 ml Dichlormethan wird bei Raumtemperatur 879 mg (2,73 mmol) Jodosobenzoldiacetat gegeben. Der Ansatz wird 24 h gerührt und anschließend eingeengt. Der erhaltene Rückstand wird chromatographisch (Dichlormethan / Ethanol 95:5) gereinigt. Man erhält 294 mg (0,82 mmol; Ausbeute 46 %) des Produktes.

¹H-NMR (DMSO): 8.94 (s, 1H), 8.61 (m, 1H), 8.39 (m, 2H), 8.11 (m, 2H), 7.64 (m, 1H), 3.12 (s, 3H).
MS: 360 (ES+).

### Verbindung 24.3

### (RS)-S-Methyl-S-(4-nitrophenyl)-N-[(4-nitrophenyl)sulfonyl]sulfimid

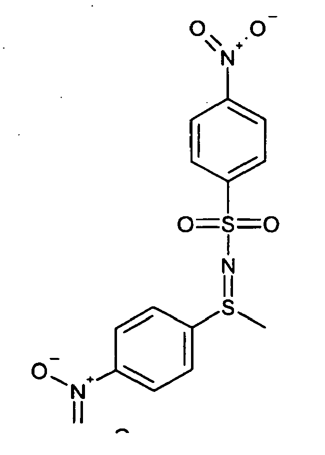

Zu einer Suspension von 300 mg (1,77 mmol) 1-Methylsulfanyl-4-nitro-benzol, 716 mg (3,55 mmol) 4-Nitrobenzolsulfonamid, 285 mg (7,10 mmol) Magnesiumoxid und 78 mg (0,18 mmol) Rhodium(II)acetat Dimer in 12 ml Dichlormethan wird bei Raumtemperatur 879 mg (2,73 mmol) Jodosobenzoldiacetat gegeben. Der Ansatz wird 24 h gerührt und anschließend eingeengt. Der erhaltene Rückstand wird chromatographisch (Dichlormethan / Ethanol 95:5) gereinigt. Man erhält 484 mg (1,31 mmol; Ausbeute: 74 %) des Produktes.

¹H-NMR (DMSO): 8.37 (m, 2H), 8.25 (m, 2H), 8.06 (m, 2H), 7.93 (m, 2H), 3.09 (s, 3H).
MS: 370 (ES+).

### Verbindung 24.4

### (RS)-S-(4-nitrophenyl)-S-methyl-N-mesylsulfimid

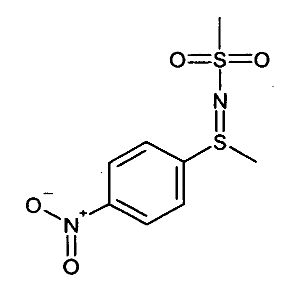

Zu einer Suspension von 300 mg (1,77 mmol) 1-Methylsulfanyl-4-nitro-benzol, 337 mg (3,55 mmol) Methansulfonamid, 285 mg (7,10 mmol) Magnesiumoxid und 78 mg (0,18 mmol) Rhodium(II)acetat Dimer in 12 ml Dichlormethan wird bei Raumtemperatur 879 mg (2,73 mmol) Jodosobenzoldiacetat gegeben. Der Ansatz wird 24 h gerührt und anschließend eingeengt. Der erhaltene Rückstand wird chromatographisch (Dichlormethan /Ethanol 95:5) gereinigt. Man erhält 133 mg (0,51 mmol; Ausbeute: 29 %) des Produktes.

¹H-NMR (DMSO): 8.43 (m, 2H), 8.14 (m, 2H), 3.06 (s, 3H), 2.86 (s, 3H).
MS: 263 (ES+).

### Verbindung 24.5

### (RS)-S-Methyl-S-(4-nitrophenyl)-N-[(4-methoxy-phenyl)sulfonyl]sulfimid

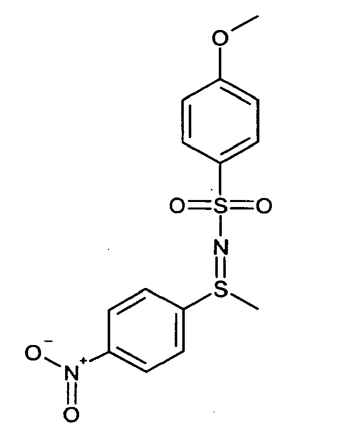

Zu einer Suspension von 300 mg (1,77 mmol) 1-Methylsulfanyl-4-nitro-benzol, 663 mg (3,55 mmol) 4-Methoxy-benzolsulfonamid, 285 mg (7,10 mmol) Magnesiumoxid und 78 mg (0,18 mmol) Rhodium(II)acetat Dimer in 12 ml Dichlormethan wird bei Raumtemperatur 879 mg (2,73 mmol) Jodosobenzoldiacetat gegeben. Der Ansatz wird 24 h gerührt und anschließend eingeengt. Der erhaltene Rückstand wird chromatographisch (Dichlormethan / Ethanol 95:5) gereinigt. Man erhält 119 mg (0,34 mmol; Ausbeute: 19 %) des Produktes.

¹H-NMR (DMSO): 8.36 (m, 2H), 8.04 (m, 2H), 7.61 (m, 2H), 6.94 (m, 2H), 3.74 (s, 3H), 3.01 (s, 3H).
MS: 355 (ES+).

### b) Herstellung der Endprodukte

Analog zu Beispiel 23 können aus den Zwischenprodukten 24.1 -24.5 beispielsweise folgende Endprodukte hergestellt werden:

| Beispiel | Struktur |
|---|---|
| 24 (identisch zu Beispiel 11) | 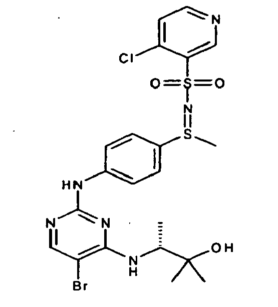 |
| 25 | 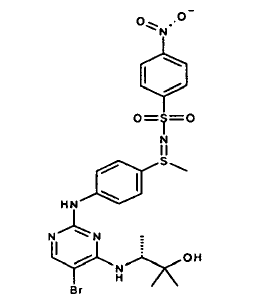 |
| 26 | 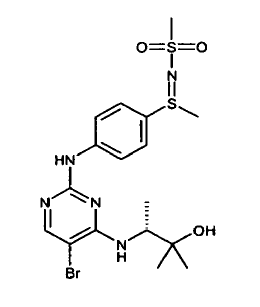 |
| 27 (identisch zu Beispiel 12) | 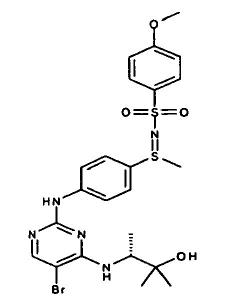 |
| 28 | 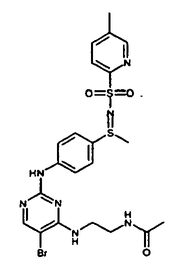 |
| 29 | 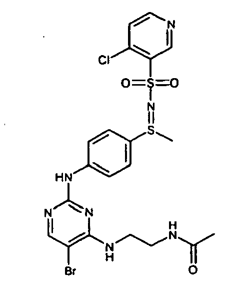 |
| 30 | 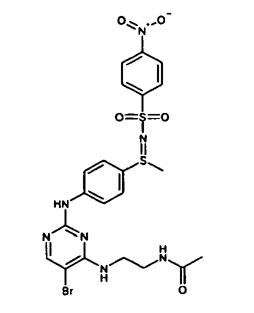 |
| 31 | 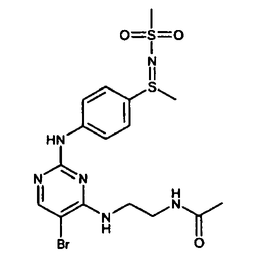 |
| 32 | 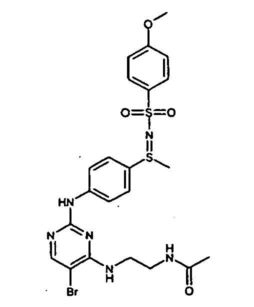 |

### Beispiel 33 (identisch zu Beispiel 14)

### (RS)-S-{4-[(5-Brom-4-{[(R)-2-hydroxy-1,2-dimethylpropyl]amino}pyrimidin-2-yl)amino]phenyl}-S-methyl-N-[(trifluormethyl)sulfonyl]sulfimid

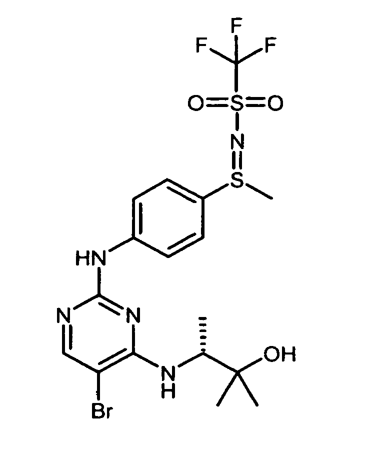

### 33a) Herstellung der Zwischenprodukte

### Verbindung 33.1 (RS)-S-Methyl-S-{4-[(trifluoracetyl)amino]phenyl}-N-[(trifluormethyl)sulfonyl]sulfimid

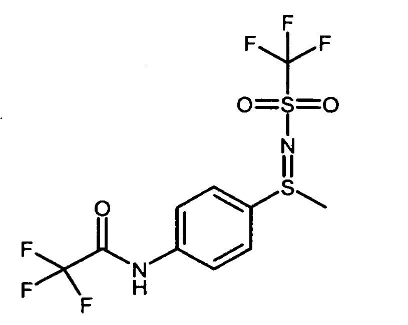

2,44 g (10,4 mmol) 2,2,2-Trifluor-N-(4-methylsulfanyl-phenyl)-acetamid und 2,80 g (18,8 mmol) Trifluormethylsulfonamid werden mit 145 ml Dichlormethan versetzt. Der Ansatz wird mit 2,09 g (51,9 mmol) Magnesiumoxid, 460 mg (1,04 mmol) Rhodium(II)acetat Dimer und 6,68 g (20,75 mmol) lodbenzol-diacetat versetzt. Der Ansatz wird 1 Stunde bei Raumtemperatur gerührt und anschließend filtriert und der Filterkuchen mit Dichlormethan gewaschen. Beim Einengen des Filtrates fallen Kristalle aus, die abgesaugt und mit wenig Dichlormethan gewaschen werden. Nach dem Trocknen erhält man 3,60 g (9,42 mmol; Ausbeute: 91 %) des Produktes.

¹H-NMR (DMSO): 11.65 (br, 1H); 7.93 (m, 4H), 3.21 (s, 3H).

### Verbindung 33.2 (RS)-S-(4-Aminophenyl)-S-methyl-N-[(trifluormethyl)sulfonyl]sulfimid

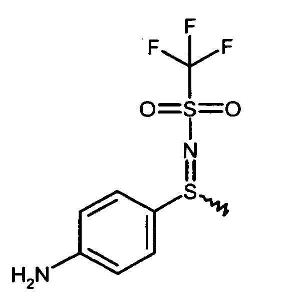

Eine Lösung von 1000 mg (2,62 mmol) (RS)-S-Methyl-S-{4-[(trifluoracetyl)amino]phenyl}-N-[(trifluormethyl)sulfonyl]sulfimid in 25 ml Methanol wird mit 361 mg (2,62 mmol) Kaliumcarbonat versetzt und 23 Stunden bei Raumtemperatur gerührt. Man versetzt mit weiteren 360 mg (2,62 mmol) Kaliumcarbonat und rührt weitere 22 Stunden bei Raumtemperatur. Der Ansatz wird mit Wasser verdünnt und gegen Essigester extrahiert (zweimal). Die vereinten organischen Phasen werden mit gesättigter NaCl Lösung gewaschen, über einem Whatman Filter filtriert und eingeengt. Der erhaltene Rückstand wird chromatographisch (Hexan / Essigester 1:4) gereinigt. Man erhält 193 mg (0,67 mmol; Ausbeute: 26%) des Produktes.

¹H-NMR (DMSO): 7.52 (m, 2H), 6.66 (m, 2H), 6.18 (s, 2H), 3.08 (s, 3H).

### 33b) Herstellung des Endproduktes

50 mg (0,17 mmol) (RS)-S-(4-Aminophenyl)-S-methyl-N-[(trifluormethyl)sulfonyl]sulfimid und 51 mg (0,17 mmol) (R)-3-[(5-Brom-2-chlorpyrimidin-4-yl)amino]-2-methylbutan-2-ol (vgl. WO2005037800) werden in 2 ml 1-Butanol 74 Stunden bei 90°C gerührt und anschließend einrotiert. Der verbleibende Rückstand wird chromatographisch (Dichlormethan / Ethanol 8:2) gereinigt. Man erhält 6 mg (0,01 mmol; Ausbeute: 6%) des Produktes.

### Beispiel 34

### (RS)-S-(4-{[4-{[(1R,2R)-2-Hydroxy-1-methylpropyl]amino}-5-(trifluormethyl)pyrimidin-2-yl]amino}phenyl)-S-methyl-N-[(trifluormethyl)sulfonyl]sulfimid

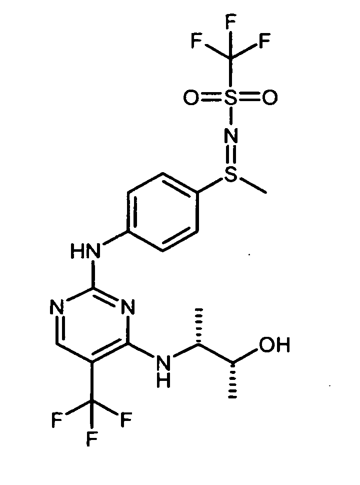

### 34a) Herstellung der Zwischenprodukte

### Verbindung 34.1

### (2R,3R)-3-(2-Chloro-5-trifluoromethyl-pyrimidin-4-ylamino)-butan-2-ol

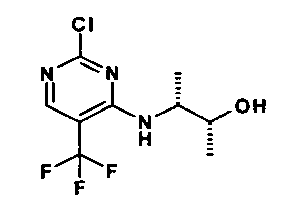

3,78 g (17,4 mmol) 2,4-Dichloro-5-trifluormethyl-pyrimidin und 2,19 g (17,4 mmol) (2R,3R)-3-Amino-butan-2-ol Hydrochlorid werden in 70 ml Acetonitril bei 0°C tropfenweise mit 4,8 ml (34,8 mmol) Triethylamin versetzt. Der Ansatz wird langsam auf Raumtemperatur erwärmt und anschließend 48 Stunden gerührt. Der Ansatz wird in halbkonzentrierte NaCl Lösung gegeben und mit Essigester extrahiert. Die vereinten organischen Phasen werden getrocknet (Na₂SO₄), filtriert und eingeengt. Der erhaltene Rückstand wird per HPLC gereinigt. Man erhält 1,45 g (5,4 mmol; 31 % Ausbeute) des Produktes.
- Säule:: XBridge C18 5µ
- Länge x ID:: 100x30 mm
- Eluenten:: A:H₂O B:Acetonitril
- Puffer:: A / 0,1 % TFA
- Gradient:: 60%A+40%B(2')_40->70%B(10')->99%B(0,5')
- Fluss:: 40,0 ml/min
- Detektion:: DAD (210-500 nm) TAC ; MS-ESI+ (125-800 m/z) TIC
- Temperatur:: Raumtemperatur
- RT in min:: 5,0-6,0

### 34b) Herstellung des Endproduktes

50 mg (0,19 mmol) (2R,3R)-3-(2-Chloro-5-trifluoromethyl-pyrimidin-4-ylamino)-butan-2-ol und 53 mg (0,94 mmol) (RS)-S-(4-Aminophenyl)-S-methyl-N-[(trifluormethyl)sulfonyl]sulfimid werden in 2 ml 1-Butanol 4 Stunden bei Raumtemperatur gerührt. Der Ansatz wird einrotiert und der verbleibende Rückstand chromatographisch (Dichlormethan / Ethanol 95:5) gereinigt. Man erhält 40 mg (0,19 mmol; Ausbeute: 42%) des Produktes.

¹H-NMR (DMSO): 10.20 (s, 1H), 8.32 (s, 1H), 8.06 (m, 2H), 7.90 (m, 2H), 6.14 (d, 1H), 5.11 (br, 1H), 4.16 (m, 1H), 3.80 (m, 1H), 3.23 (s, 3H), 1.25 (d, 3H), 1.09 (d, 3H).
MS: 520 (ES+)

### Assay 1

### Aurora-C Kinase Assay

Rekombinantes Aurora-C Protein wurde in transient-transfizierten HEK293 Zellen exprimiert und anschließend gereinigt. Als Kinase-Substrat wurde das biotinylierte Peptid mit der Aminosäuresequenz biotin-FMRLRRLSTKYRT verwendet, das bei der Fa. Jerini AG in Berlin gekauft wurde.
Aurora-C [5 nM im Testansatz, Testvolumen 5 µl] wurde für 90 min bei 22°C in Anwesenheit verschiedener Konzentrationen an Testsubstanzen (0 µM, sowie 10 Messpunkten innerhalb des Bereiches 0,001 - 20 µM in Doppelwerten) in Assaypuffer [25 mM HEPES pH7,4, 0.5 mM MnCl₂, 0,1 mM Na ortho-Vanadat, 2,0 mM Dithiothreitol, 0.05% Bovines Serumalbumin (BSA), 0.01% Triton X-100, 3 µM Adenosintrisphosphat (ATP), 0,67 nCi/µl gama-³³P-ATP, 2,0 µM Substratpeptid biotin-FMRLRRLSTKYRT, 1,0% Dimethylsulfoxid] inkubiert. Die Reaktion wurde durch Zugabe von 12.5 µl einer EDTA/Detektions-Lösung [16 mM EDTA, 40 mM ATP, 0.08% Triton X-100, 4 mg/ml PVT-Streptavidin-SPA-Beads (Fa. Amersham)] gestoppt. Nach 10 Minuten Inkubation wurden die SPA-Beads durch 10 minütige Zentrifugation bei 1000 x G pelletiert. Die Messung erfolgte in einem Topcount Szintillationsmessgerät der Firma PerkinElmer.
Die Messdaten wurden normalisiert auf 0% Inhibition (Enzymreaktion ohne Inhibitor) und 100% Inhibition (Enzymreaktion in Gegenwart von 0.1 µM Staurosporin (Fa. Sigma)). Die Bestimmung der IC50 Werte erfolgte mittels eines 4-Parameter Fits unter Benutzung firmeneigener Software.

### Assay 2

### CDK1/CycB Kinase Assay

Rekombinante CDK1- und CycB-GST-Fusionsproteine, gereinigt aus Bakulovirusinfizierten Insektenzellen (Sf9), wurden von ProQinase GmbH, Freiburg, gekauft. Das als Kinase-Substrat verwendet Histon IIIS ist über die Fa. Sigma käuflich zu erwerben.
CDK1/CycB (200 ng/Messpunkt) wurde für 10 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl2, 0,1 mM Na ortho-Vanada⁺, 1,0 mM Dithiothreitol, 0,5 µM ATP, 10 µg/Messpunkt Histon IIIS, 0,2 µCi/Messpunkt ³³P-gamma ATP, 0,05% NP40, 1,25% Dimethylsulfoxid] inkubiert. Die Reaktion wurde durch Zugabe von EDTA-Lösung (250 mM, pH8,0, 15 µl/Messpunkt) gestoppt.
Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLexTM A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt.

### Assay 3

### CDK2/CycE Kinase Assay

Rekombinante CDK2- und CycE-GST-Fusionsproteine, gereinigt aus Bakulovirusinfizierten Insektenzellen (Sf9), wurden von ProQinase GmbH, Freiburg, gekauft. Histon IIIS, das als Kinase-Substrat verwendet wurde, wurde bei der Fa. Sigma gekauft.
CDK2/CycE (50 ng/Messpunkt) wurde für 10 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM ATP, 10 µg/Messpunkt Histon IIIS, 0,2 µCi/Messpunkt ³³P-gamma ATP, 0,05% NP40, 1,25% Dimethylsulfoxid] inkubiert. Die Reaktion wurde durch Zugabe von EDTA-Lösung (250 mM, pH8,0, 15 µl/Messpunkt) gestoppt.
Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt.
Nach dem Trocknen der Filterstreifen für 1 Stunde bei 70°C wurden die Filterstreifen mit Szintillator-Streifen (MeltiLex^{™} A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt.

### Assay 4

### KDR Kinase Assay

Rekombinantes KDR Protein wurde in E. coli exprimiert und anschließend gereinigt. Als Kinase-Substrat wurde das biotinyliertes Peptid mit der Aminosäuresequenz biotin-DFGLARDMYDKEYYSVG verwendet, das bei der Fa. Biosynthan gekauft wurde.
KDR [Testvolumen 5 µl] wurde für 45 min bei 22°C in Anwesenheit verschiedener Konzentrationen an Testsubstanzen (0 µM, sowie 10 Messpunkten innerhalb des Bereiches 0,001 - 20 µM in Doppelwerten) in Assaypuffer [50 mM HEPES pH7,0, 25,0 mM MgCl₂, 1,0 mM MgCl₂, 0,1 mM Na ortho-Vanadat, 1,0 mM Dithiothreitol, 0.001 % NP40, 10 µM ATP, 0,03 µM Substratpeptid biotin- poly GluTyr, 1,0% Dimethylsulfoxid] inkubiert. Die Reaktion wurde durch Zugabe von 5 µl einer EDTA/Detektions-Lösung [50 mM HEPES pH7,5, 125 mM EDTA, 0.2% BSA, 0,1 µM Streptavidin-XLent (Fa. CisBio), 2 nM PT66-Eu (Fa. PerkinElmer)] gestoppt. Die Messung der Fluoreszenzemission bei 620 nm und 665 nm nach Anregung mit Licht der Wellenlänge 350 nm erfolgte in einem Rubystar HTRF-Messgerät der Firma BMG Labsystems.
Die Messdaten (Ratio aus Emission 665 geteilt durch Emission 620 multipliziert mit 10000) wurden normalisiert auf 0% Inhibition (Enzymreaktion ohne Inhibitor) und 100% Inhibition (alle Assaykomponenten außer Enzym). Die Bestimmung der IC50 Werte erfolgte mittels eines 4-Parameter Fits unter Benutzung firmeneigener Software.

### Assay 5

### MCF7 Proliferationsassay

Kultivierte humane MCF7 Brusttumorzellen (ATCC HTB-22) wurden in einer Dichte von 5000 Zellen/Meßpunkt in einer 96-well Multititerplatte in 200 µl Wachstumsmediums (RPMI1640, 10% Fötales Kälberserum, 2 mU/mL Insulin, 0,1 nM Östradiol) ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,1 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Meßpunkt einer 11 %igen GlutaraldehydLösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Meßpunkt einer 0,1%igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Meßpunkt einer 10%igen Essigsäure-Lösung gelöst, und die Extinktion wurde photometrisch bei einer Wellenlänge von 595 nm bestimmt. Die prozentuale Änderung des Zellwachstums wurde durch Normalisierung der Meßwerte auf die Extinktionwerte der Nullpunktplatte (=0%) und die Extinktion der unbehandelten (0 µM) Zellen (=100%) berechnet. Die Bestimmung der IC50 Werte erfolgte mittels eines 4-Parameter Fits unter Benutzung firmeneigener Software

### Beispiel 35

### Die Verbindungen der Beispiele 1 bis 9 und 11-23 wurden in den verschiedenen Kinase-Assays hinsichtlich ihrer inhibitorischen Wirkung getestet (Tab. 1)

**Tab.1**

| | Aurora C IC50 | CDK1/CycB IC50 | CDK2/CycE IC50 | KDR (VEGFR2) IC50 |
|---|---|---|---|---|
| Bsp. | [nM] | [nM] | [nM] | [nM] |
| 1 | 240 | >1000 | 900 | 37 |
| 2 | | >1000 | >1000 | 200 |
| 3 | 90 | >1000 | 1000 | 13 |
| 4 | | >1000 | >1000 | 170 |
| 5 | 4600 | >1000 | >1000 | 760 |
| 6 | | >1000 | >1000 | 120 |
| 7 | 680 | >1000 | >1000 | 43 |
| 8 | 360 | 620 | 99 | 22 |
| 9 | 360 | 150 | 28 | 36 |
| 11 | 370 | 90 | 14 | 44 |
| 12 | 480 | 160 | 39 | 38 |
| 13 | 660 | 140 | 28 | 15 |
| 14 | 990 | 120 | 39 | 70 |
| 15 | 570 | 110 | 21 | 37 |
| 16 | 1800 | 260 | 72 | 150 |
| 17 | nd | nd | nd | nd |
| 18 | 400 | 50 | 6 | 35 |
| 19 | 1100 | 98 | 25 | 320 |
| 20 | 250 | 31 | 10 | 35 |
| 21 | 390 | 36 | 12 | 49 |
| 22 | 390 | 46 | 22 | 44 |
| 23 | 36 | 990 | 1000 | 35 |

Aus der strukturellen Klasse der Sulfimid-substituierten Aminopyrimidine lassen sich sowohl selektive, als auch Multi-target Kinaseinhibitoren herstellen. Bsp. 7 stellt einen selektiven Inhibitor der VEGF-R2 Tyrosinkinase dar, während Bsp. 8 neben VEGF-R2 auch Aurora C kinase inhibiert, Bsp. 3 die VEGF-R2 und Aurora C inhibiert, und Bsp. 11 neben VEGF-R2 an den Serin/Threonin Kinasen Aurora C und CDK2 aktiv ist. Bsp. 9 zeigt Inhibition der VEGF-R2 und der CDK2 Kinase.

### Beispiel 36

Einige Verbindungen der Beispiele wurden im MCF7 Proliferationsassay hinsichtlich ihrer antiproliferativen Wirkung getestet (Tab. 2). Die getesteten Verbindungen zeigten eine potente Inhibition der Proliferation von MCF7 humanen Brusttumorzellen bei mikromolarer und sub-mikromolarer Konzentration. Hervorragende antiproliferative Aktivität zeigten insbesondere die Beispiele Nr. 9, 10, 13, 14, 15, 16, und 17.

**Tab.2**

| | MCF7 IC50 |
|---|---|
| Bsp. | [µM] |
| 1 | 2,2 |
| 2 | - |
| 3 | 0,34 |
| 4 | - |
| 5 | - |
| 6 | - |
| 7 | - |
| 8 | 0,9 |
| 9 | <0,1 |
| 11 | <0,1 |
| 12 | 0,2 |
| 13 | 0,11 |
| 14 | 0,11 |
| 15 | 0,11 |
| 16 | 0,17 |
| 17 | - |
| 18 | 0,17 |
| 19 | 1,8 |
| 20 | 0,35 |
| 21 | 0,31 |
| 22 | 0,33 |
| 23 | 2,2 |

## Patentansprüche

1. Verbindungen der allgemeinen Formel I, 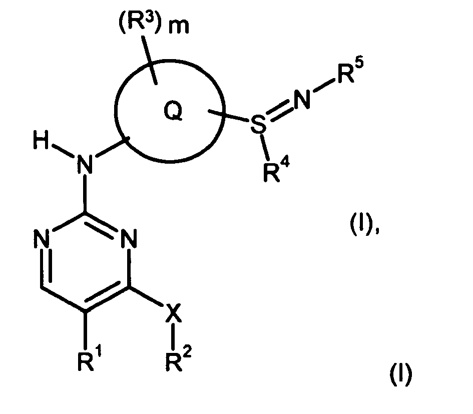 in der
R¹ für
(i) Wasserstoff, Halogen, Cyano, Nitro, -NR⁸R⁹, -NR⁷-C(O)-R¹², -NR⁷-C(O)-OR¹², -NR⁷-C(O)-NR⁸R⁹, -NR⁷-SO₂-R¹², -CF₃ oder -OCF₃, oder
(ii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, C₁-C₆-Alkoxy, -CF₃ und/oder -OCF₃ ein- oder mehrfach, gleich oder verschieden substituierten C₁-C₆-Alkyl-, C₂-C₆-Alkenyl-, C₁-C₆-Alkoxy- oder C₂-C₆-Alkinylrest, oder
(iii) einen gegebenenfalls mit Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, Halogen, -CF₃, C₁-C₆-Alkoxy, -OCF₃ und/oder C₁-C₆-Alkyl ein- oder mehrfach, gleich oder verschieden substituierten Phenyl- oder monocyclischen Heteroarylring steht,
R² für
(i) Wasserstoff oder
(ii) einen C₁-C₁₀-Alkyl-, C₂-C₁₀-Alkenyl- oder C₂-C₁₀₋Alkinylrest, einen C₃-C₇-Cycloalkyl-, Phenyl- oder Naphthylring, einen Heterocyclylring mit 3 bis 8 Ringatomen oder einen mono- oder bicyclischen Heteroarylring steht,
jeweils gegebenenfalls ein- oder mehrfach, gleich oder verschieden substituiert mit
a) Halogen, Hydroxy, -NR⁸R⁹, -NR⁷-C(O)-R¹², -NR⁷-C(O)-OR¹², -NR⁷-C(O)-NR⁸R⁹, -NR⁷-SO₂-R¹², Cyano, -C(O)R⁶,-O(CO)-R¹², -SO₂NR⁸R⁹, -SO₂-R¹², -S(O)(NR⁸)R¹², -(N)S(O)R¹³R¹⁴, -CF₃, -OCF₃, -N[(CO)-(C₁-C₆-Alkyl)]₂ und/oder
b) C₁-C₆-Alkoxy, C₁-C₆-Alkyl, C₂-C₆-Alkenyl, C₂-C₆-Alkinyl, C₃-C₈-Cycloalkyl, Phenyl, Naphthyl, Heterocyclyl mit 3 bis 8 Ringatomen und/oder einem monocyclischen oder bicyclischen Heteroaryl, jeweils gegebenenfalls selbst ein- oder mehrfach, gleich oder verschieden mit Halogen, Hydroxy, einem C₁-C₆-Alkyl, C₁-C₆-Alkoxy, -NR⁸R⁹, -C(O)OR¹⁶, -SO₂NR⁸R⁹, -CF₃ oder -OCF₃ substituiert,
R³ für
(i) Hydroxy, Halogen, Cyano, Nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₆-Alkyl- und/oder C₁-C₆- Alkoxyrest, und/oder
(iii) einen gegebenenfalls mit Halogen, Hydroxy, C₁-C₆-Alkoxy, -CF₃, -OCF₃, -NR⁸R⁹ und/oder C₁-C₆-Alkyl ein- oder mehrfach, gleich oder verschieden substituierten C₃-C₇-Cycloalkylring
steht,
m für 0-4 steht,
R⁴ für einen C₁-C₆-Alkyl-, C₂-C₆-Alkenyl-, C₂-C₆-Alkinylrest, einen C₃-C₇- Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert,
oder
R³ und R⁴ gemeinsam einen an Q annelierten 5 bis 7-gliedrigen Ring bilden, der gegebenenfalls ein- oder mehrfach, gleich oder verschieden mit Hydroxy, C₁-C₆-Alkyl, C₁-C₆-Alkoxy, Halogen oder -NR⁸R⁹ substituiert ist und gegebenenfalls zusätzlich zur Doppelbindung aus Q eine weitere Doppelbindung enthält, wenn der Ring 5-gliedrig ist
R⁵ für
-SO₂-(CH₂)ₙ-R¹² mit n gleich 0 oder 1,
-C(O)R¹², -C(O)OR¹², -C(O)NR⁸R⁹, -C(S)OR¹², -C(S)NR⁸R⁹ oder -R¹² steht,
oder
R⁴ und R⁵ gemeinsam einen 5 bis 7-gliedrigen Ring der Formel 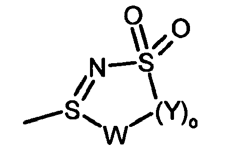 oder 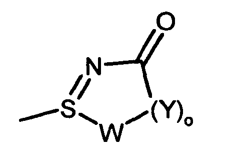 bilden,
in denen
W und Y jeweils unabhängig voneinander für eine gegebenenfalls ein- oder mehrfach, gleich oder verschieden mit Hydroxy, C₁-C₆- Alkyl, C₁-C₆-Alkoxy oder -NR⁸R⁹ substituierte -CH₂-Gruppe stehen, wobei
der C₁-C₆-Alkyl- und/oder C₁-C₆-Alkoxysubstituent gegebenenfalls selbst ein oder mehrfach, gleich oder verschieden mit Hydroxy, C₁-C₆-Alkoxy oder -NR⁸R⁹ substituiert ist und/oder gegebenenfalls zusätzlich zur Imid-Doppelbindung 1 oder 2 weitere
Doppelbindungen enthält
und
in denen
o 1-3 bedeutet
X für -O-, -S- oder -NR¹⁵- steht,
wobei,
R¹⁵ für
(i) Wasserstoff oder
(ii) einen C₁-C₆-Alkylrest, C₃-C₈-Cycloalkyl- oder Phenylring, einen Heterocyclylring mit 3 bis 8 Ringatomen oder einen monocyclischen Heteroarylring, oder
(iii) -C(O)-(C₁-C₆)-Alkyl, -C(O)-Phenyl, oder -C(O)-Benzyl steht, und (ii) und (iii) gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,
oder
wenn X für -NR¹⁵- steht, alternativ
X, R¹⁵ und R² gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆-Alkoxy, -C(O)R¹², -SO₂R¹², Halogen oder der Gruppe -NR⁸R⁹ substituiert ist, gegebenenfalls 1 bis 3 Doppelbindungen enthält und/oder gegebenenfalls durch eine oder mehrere -C(O)-Gruppen unterbrochen ist,
Q für einen Phenylring steht,
R⁶ für
(i) Wasserstoff oder Hydroxy, oder
(ii) einen C₁-C₆-Alkyl-, C₃-C₆-Alkenyl-, C₃-C₆-Alkinyl- oder C₁-C₆- Alkoxyrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆- Alkoxy und/oder -OCF₃ ein- oder mehrfach, gleich oder verschieden substituiert,
R⁷ für Wasserstoff oder einen C₁-C₆-Alkylrest steht,
R⁸ und R⁹ unabhängig voneinander für
(i) Wasserstoff und/oder
(ii) einen C₁-C₆-Alkylrest, C₂-C₆-Alkenyl-, C₃-C₈-Cycloalkyl- und/oder Phenylring, einen Heterocyclylring mit 3 bis 8 Ringatomen und/oder einen monocyclischen Heteroarylring, gegebenenfalls mit Hydroxy,
-NR¹⁰R¹¹, Cyano, Halogen, -CF₃, C₁-C₆-Alkoxy und/oder -OCF₃ ein- oder mehrfach, gleich oder verschieden substituiert sind,
oder
R⁸ und R⁹ gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, -NR¹⁰R¹¹, Cyano, Halogen, -CF₃, C₁-C₆-Alkoxy und/oder -OCF₃ ein- oder mehrfach, gleich oder verschieden substituiert sein kann,
R¹⁰ und R¹¹ unabhängig voneinander für Wasserstoff oder einen C₁-C₆-Alkylrest stehen, der gegebenenfalls mit Hydroxy, Cyano, Halogen, -CF₃, C₁- C₆-Alkoxy und/oder -OCF₃ ein- oder mehrfach, gleich oder verschieden substituiert ist.
R¹², R¹³, R¹⁴ unabhängig voneinander für -CF₃
oder
für einen C₁-C₆-Alkyl, C₂-C₆-Alkenyl- und/oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht, die gegebenenfalls jeweils selbst mit Hydroxy, Nitro, -NR⁸R⁹, -NH-C(O)-C₁-C₆-Alkyl ,Cyano, Halogen, -CF₃, C₁-C₆-Alkyl , C₁-C₆- Alkoxy und/oder -OCF₃ ein- oder mehrfach, gleich oder verschieden substituiert sind,
R¹⁶ für
(i) Wasserstoff oder
(ii) einen C₁-C₆-Alkyl-, C₃-C₆-Alkenyl-, C₃-C₆-Alkinylrest, einen C₃-C₇-Cycloalkyl- oder Phenylring, einen Heterocyclylring mit 3 bis 8 Ringatomen oder einen monocyclischen Heteroarylring steht, jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert,
sowie deren Salze, Diastereomere und Enantiomere.

2. Verbindungen der allgemeinen Formel I gemäß Anspruch 1, in der
R¹ für Halogen, -CF₃, -OCF₃, C₁-C₄-Alkyl oder Nitro steht,
R² für einen C₁-C₁₀-Alkyl-, C₂-C₁₀-Alkenyl- oder C₂-C₁₀-Alkinylrest, einen C₃-C₇-Cycloalkyl-, Phenyl- oder einen mono- oder bicyclischen Heteroarylring oder einen Heterocyclylring mit 3 bis 7 Ringatomen steht,
jeweils gegebenenfalls ein oder mehrfach, gleich oder verschieden substitutiert mit Hydroxy, -NR⁸R⁹, -NR⁷-C(O)-R¹² und/oder einem C₁-C₄-Alkylrest, der gegebenenfalls selbst ein- oder mehrfach substituiert ist mit Hydroxy
R³ für
(i) Hydroxy, Halogen, Cyano, Nitro, -CF₃, -OCF₃, , -NR⁸R⁹, -NR⁷-C(O)-R¹², -NR⁷-C(O)-OR¹², -NR⁷-C(O)-NR⁸R⁹, -NR⁷-SO₂-R¹², und/oder
(ii) einen gegebenenfalls mit Halogen, Hydroxy, C₁-C₆-Alkoxy, -CF₃, -OCF₃ oder -NR⁸R⁹ ein- oder mehrfach, gleich oder verschieden substituierten C₁-C₃-Alkyl- und/oder C₁-C₃- Alkoxyrest steht,
m für 0 oder 1 steht,
R⁴ für einen C₁-C₅-Alkylrest, einen C₃-C₆-Cycloalkyl- oder einen Phenylring steht,
jeweils gegebenenfalls selbst mit Hydroxy, -NR⁸R⁹, Cyano, Halogen, -CF₃, C₁-C₆-Alkoxy, -OCF₃ und/oder C₁-C₆-Alkyl ein- oder mehrfach, gleich oder verschieden substituiert
oder
R³ und R⁴ gemeinsam einen an Q annelierten 5-gliedrigen Ring bilden, der gegebenenfalls zusätzlich zur Doppelbindung aus Q eine weitere Doppelbindung enthält,
R⁵ für -SO₂-(CH₂)ₙ-R¹² mit n gleich 0 oder 1 steht,
wobei R¹² für CF₃
oder für einen C₁-C₄-Alkylrest, einen C₃-C₆-Cycloalkyl- oder Phenylring oder einen Heterocyclylring mit 3 bis 6 Ringatomen oder einen monocyclischen Heteroarylring steht, gegebenenfalls jeweils selbst mit Hydroxy, Nitro, -NR⁸R⁹, Cyano, Halogen, -CF₃, C₁-C₆-Alkyl , C₁-C₆-Alkoxy und/oder -OCF₃ ein- oder mehrfach, gleich oder verschieden substituiert,
oder
R⁴ und R⁵ gemeinsam einen 5-gliedrigen Ring der Formel (1) bilden, 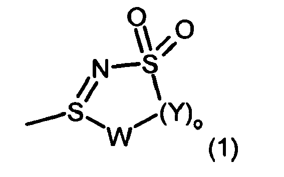 in der W und Y jeweils für eine -CH₂-Gruppe stehen und in der o 1 bedeutet.
X für -O-, -S- oder -NR¹⁵- steht,
wobei,
R¹⁵ für
(i) Wasserstoff oder
(ii) einen C₁-C₆-Alkylrest, C₃-C₈-Cycloalkyl- oder Phenylring, einen Heterocyclylring mit 3 bis 8 Ringatomen oder einen monocyclischen Heteroarylring, oder
(iii) -C(O)-(C₁-C₆)-Alkyl, -C(O)-Phenyl, oder -C(O)-Benzyl steht, und (ii) und (iii) gegebenenfalls mit Hydroxy, -NR¹⁰R¹¹, Cyano, Halogen, -CF₃, C₁-C₆-Alkoxy und/oder -OCF₃ ein- oder mehrfach, gleich oder verschieden substituiert sind,
oder
wenn X für -NR¹⁵- steht, alternativ
X, R¹⁵ und R² gemeinsam einen 3 bis 8 gliedrigen Ring bilden, der gegebenenfalls zusätzlich zum Stickstoffatom ein oder mehrere weitere Heteroatome enthält, gegebenenfalls ein- oder mehrfach, gleich oder verschieden mit Hydroxy, C₁-C₆-Alkyl, C₁-C₆- Alkoxy, -C(O)R¹², -SO₂R¹², Halogen oder der Gruppe -NR⁸R⁹ substituiert ist, gegebenenfalls 1 bis 3 Doppelbindungen enthält und/oder gegebenenfalls durch eine oder mehrere -C(O)- Gruppen unterbrochen ist,
Q für einen Phenylring steht,
R⁶ für einen C₂-C₅-Alkyl-, C₄-C₆-Alkenyl-, C₄-C₆-Alkinyl- oder C₂-C₅- Alkoxyrest, einen C₄-C₆-Cycloalkyl- oder Phenylring, einen Heterocyclylring mit 3 bis 5 Ringatomen oder einen monocyclischen Heteroarylring steht,
jeweils gegebenenfalls selbst mit Hydroxy, -NR⁸R⁹, Cyano, Halogen, -CF₃, C₁-C₆-Alkoxy und/oder -OCF₃ ein- oder mehrfach, gleich oder verschieden substituiert
R⁷ für Wasserstoff oder einen C₁-C₆-Alkylrest steht,
R⁸ und R⁹ jeweils unabhängig voneinander für Wasserstoff und/oder einen C₁-C₄-Alkylrest, C₃-C₆-Cycloalkyl- und/oder Phenylring und/oder einen monocyclischen Heteroarylring,
jeweils gegebenenfalls mit Hydroxy, -NR¹⁰R¹¹ oder C₁-C₆-Alkoxy ein- oder mehrfach, gleich oder verschieden substituiert,
oder
R⁸ und R⁹ gemeinsam mit dem Stickstoffatom einen 5- bis 7-gliedrigen Ring bilden, der gegebenenfalls zusätzlich zum Stickstoffatom 1 weiteres Heteroatom enthält und der mit Hydroxy ein- oder mehrfach substituiert sein kann,
R¹⁰ und R¹¹ unabhängig voneinander für Wasserstoff oder einen C₁-C₆-Alkylrest stehen, der gegebenenfalls mit Hydroxy ein- oder mehrfach, gleich oder verschieden substituiert ist.
R¹² für CF₃ oder
für einen C₁-C₆-Alkyl-, C₂-C₆-Alkenyl- oder C₂-C₆-Alkinylrest, einen C₃-C₇-Cycloalkyl- oder Phenylring , einen Heterocyclylring mit 3 bis 8 Ringatomen oder einen monocyclischen Heteroarylring steht, jeweils gegebenenfalls selbst mit Hydroxy, Halogen, Nitro, -NR⁸R⁹, C₁-C₆-Alkyl ,und/oder C₁-C₆-Alkoxy ein- oder mehrfach, gleich oder verschieden substituiert,
R¹³ und R¹⁴ unabhängig voneinander für einen C₁-C₆-Alkyllrest stehen, und
R¹⁶ für einen C₁-C₆-Alkylrest, einen C₃-C₇-Cycloalkyl- oder Phenylring, einen Heterocyclylring mit 3 bis 8 Ringatomen oder einen monocyclischen Heteroarylring steht,
sowie deren Salze, Diastereomere und Enantiomere.

3. Verbindungen der allgemeinen Formel (I) gemäß einem der Ansprüche 1 oder 2 in der
R¹ für Brom oder CF₃ steht,
sowie deren Salze, Diastereomere und Enantiomere.

4. Verbindungen der allgemeinen Formel (I) gemäß einem der Ansprüche 1 bis 3 4, in der
R² für einen C₁-C₆-Alkylrest, einen C₃-C₇-Cycloalkyl- oder Phenylring steht, jeweils gegebenenfalls ein oder mehrfach, gleich oder verschieden substituiert mit Hydroxy und/oder -NH-C(O)-C₁-C₆-Alkyl,
sowie deren Salze, Diastereomere und Enantiomere.

5. Verbindungen der allgemeinen Formel (I) gemäß einem der Ansprüche 1 bis 4 in der
X für -NR¹⁵- steht, wobei R¹⁵ für Wasserstoff steht,
sowie deren Salze, Diastereomere und Enantiomere.

6. Verbindungen der allgemeinen Formel (I) gemäß einem der Ansprüche 1 bis 5 in der
R³ für Hydroxy, Fluor, Chlor, Brom, Cyano, Nitro, -CF₃, Methyl oder Methoxy steht,
sowie deren Salze, Diastereomere und Enantiomere.

7. Verbindungen der allgemeinen Formel (I) gemäß einem der Ansprüche 1 bis 6 , in der m für 0 steht,
sowie deren Salze, Diastereomere und Enantiomere.

8. Verbindungen der allgemeinen Formel (I) gemäß einem der Ansprüche 1 bis 7. in der
R⁴ für einen C₁-C₄-Alkylrest oder einen C₃-C₅-Cycloalkylring steht,
jeweils gegebenenfalls_ selbst mit Hydroxy, -NR⁸R⁹ oder Halogen ein- oder mehrfach, gleich oder verschieden substituiert,
sowie deren Salze, Diastereomere und Enantiomere.

9. Verbindungen der allgemeinen Formel (I) gemäß einem der Ansprüche 1 bis 8, in der
R⁵ für -SO₂R¹² steht,
wobei
R¹² für einen C₁-C₆-Alkylrest, einen Phenyl- oder einen monocyclischen Heteroarylring steht, gegebenenfalls jeweils selbst mit Nitro, Halogen und/oder C₁-C₆-Alkyl ein- oder mehrfach, gleich oder verschieden substituiert,
sowie deren Salze, Diastereomere und Enantiomere.

10. Verbindungen der allgemeinen Formel (I) gemäß einem der Ansprüche 1 bis 8, in der
R⁵ für -SO₂-(CH₂)ₙ-R¹² mit n gleich 0 oder 1 steht,
wobei
R¹² für -CF₃
oder für einen C₁-C₆-Alkylrest, einen Phenyl- oder einen monocyclischen Heteroarylring steht,
die gegebenenfalls jeweils selbst mit Nitro, -NH-C(O)-C₁-C₆-Alkyl, Halogen, C₁-C₆-Alkyl , C₁-C₆-Alkoxy und/oder -OCF₃ ein- oder mehrfach, gleich oder verschieden substituiert sind,
sowie deren Salze, Diastereomere und Enantiomere.

11. Verbindungen der allgemeinen Formel (I) gemäß einem der Ansprüche 1 bis 10, in der
R⁶ für einen C₁-C₆-Alkyl-, einen C₁-C₆-Alkoxyrest oder einen C₃-C₇-Cycloalkylring steht, jeweils gegebenenfalls selbst mit Hydroxy, -NR⁸R⁹ und /oder C₁-C₆-Alkoxy ein- oder mehrfach, gleich oder verschieden substituiert, sowie deren Salze, Diastereomere und Enantiomere.

12. Verbindungen der allgemeinen Formel (I) gemäß einem der Ansprüche 1 bis 11, in der
R⁷ für Wasserstoff oder einen C₁-C₆-Alkylrest steht,
sowie deren Salze, Diastereomere und Enantiomere.

13. Verbindungen der allgemeinen Formel (I) gemäß einem der Ansprüche 1 bis 12 , in der
R⁸ und R⁹ Wasserstoff und/oder einen C₁-C₆-Alkylrest, einen C₃-C₆-Cycloalkyl-und/oder Phenylring,und/oder einen monocyclischen Heteroarylring,
oder
R⁸ und R⁹ gemeinsam mit dem Stickstoffatom einen 5- oder 6-gliedrigen Ring bilden, der gegebenenfalls zusätzlich zum Stickstoffatom 1 weiteres Heteroatom enthält,
sowie deren Salze, Diastereomere und Enantiomere.

14. Verbindungen der allgemeinen Formel (I) gemäß einem der Ansprüche 1 bis 13 in der
R¹⁰ und R¹¹ unabhängig voneinander für Wasserstoff oder eine Methylgruppe stehen,
sowie deren Salze, Diastereomere und Enantiomere.

15. Verbindungen der allgemeinen Formel (I) gemäß einem der Ansprüche 1 bis 14 in der
R¹² für einen C₁-C₆-Alkylrest, einen Phenyl- oder monocyclischen Heteroarylring steht,
jeweils gegebenenfalls selbst mit Hydroxy, Halogen, Nitro oder C₁-C₆-Alkyl ein-oder mehrfach, gleich oder verschieden substituiert,
sowie deren Salze, Diastereomere und Enantiomere.

16. Verbindungen der allgemeinen Formel (I) gemäß einem der Ansprüche 1 bis 15 in der
R¹³ und R¹⁴ unabhängig voneinander für einen C₁-C₆-Alkylrest stehen,
sowie deren Salze, Diastereomere und Enantiomere.

17. Verbindungen der allgemeinen Formel (I) gemäß einem der Ansprüche 1 bis 16 , in der
R¹⁶ für einen C₁-C₆-Alkylrest steht
sowie deren Salze, Diastereomere und Enantiomere.

18. Verbindungen gemäß der allgemeinen Formel (I) des Anspruches 1,
in der
R¹ für Wasserstoff, Halogen oder -CF₃ steht,
R² für einen C₁-C₁₀-Alkyrest-, einen C₃-C₇-Cycloalkyl- oder Phenylring steht, jeweils gegebenenfalls ein- oder mehrfach, gleich oder verschieden substituiert mit Hydroxy oder -NH-C(O)-C₁-C₆-Alkyl
m für 0 steht,
R⁴ für einen C₁-C₆-Alkylrest steht,
R⁵ für -SO₂-(CH₂)ₙ-R¹² mit n gleich 0 oder 1 steht,
X für -NH- steht,
Q für einen Phenylring steht,
R¹² für -CF₃
oder
für einen C₁-C₆-Alkylrest, einen Phenyl- oder einen monocyclischen Heteroarylring steht, die gegebenenfalls jeweils selbst mit Nitro, Halogen, -CF₃, C₁-C₆-Alkyl, -NH-C(O)-C₁-C₆-Alkyl , C₁-C₆-Alkoxy und/oder -OCF₃ ein- oder mehrfach, gleich oder verschieden substituiert sind,
sowie deren Salze, Diastereomere und Enantiomere.

19. Verbindungen gemäß Formel (I) des Anspruches 1,
in der
R¹ für Wasserstoff oder Halogen steht,
R² für einen C₁-C₁₀-Alkylrest, einen C₃-C₇-Cycloalkyl- oder Phenylring steht, jeweils gegebenenfalls ein- oder mehrfach, gleich oder verschieden substituiert mit Hydroxy , C₁-C₆-Alkylrest oder -NR⁷-C(O)-R¹²,
m für 0 steht,
R⁴ für einen C₁-C₆-Alkylrest steht,
R⁵ für -SO₂R¹² steht,
X für -NR¹⁵- steht, wobei R¹⁵ für Wasserstoff steht.
Q für einen Phenylring steht,
R¹² für einen C₁-C₆-Alkylrest, einen Phenyl- oder einen monocyclischen Heteroarylring steht,
jeweils gegebenenfalls selbst mit Nitro, Halogen oder C₁-C₆-Alkyl ein- oder mehrfach, gleich oder verschieden substituiert,
sowie deren Salze, Diastereomere und Enantiomere.

20. Verfahren zur Herstellung der Verbindungen gemäß einem der Ansprüche 1 bis 19 umfassend die Schritte
a) Umsetzung von 2-Chlor-pyrimidine der Formel (IV) mit Nucleophilen der Formel (III) zu Verbindungen der Formel (II) 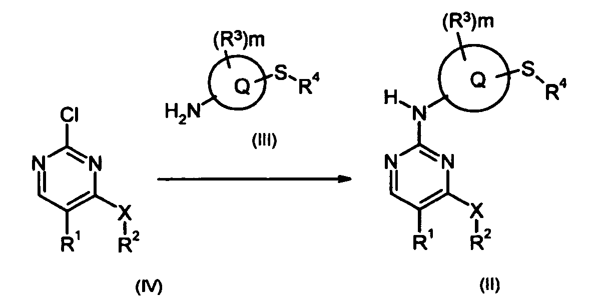
b) Iminierung der Thioether der Formel (II) unter Erhalt von Verbindungen der Formel (I) 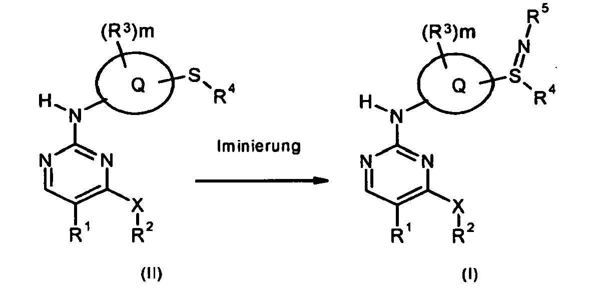 wobei Q, R¹, R², R³, R⁴, R⁵, X und m die in der allgemeinen Formel (I) gemäß der Ansprüche 1 bis 20 angegebenen Bedeutungen haben.

21. Verfahren zur Herstellung von Intermediaten der Formel (IV) durch Umsetzung von 2,4-Dichlor-pyrimidine der Formel (VI) mit Nucleophilen der Formel (V), 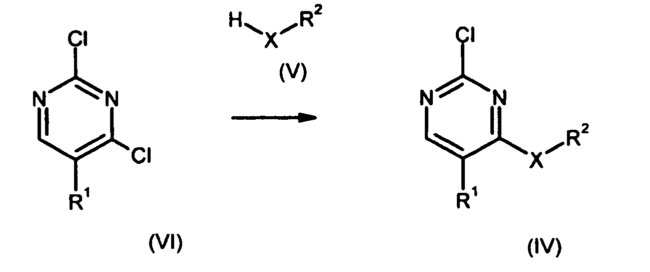 wobei R¹, R² und X die in der allgemeinen Formel (I) gemäß der Ansprüche 1 bis 19 angegebenen Bedeutungen haben.

22. Verfahren zur Herstellung der Verbindungen der Formel (I) gemäß einem der Ansprüche 1 bis 19 durch Umsetzung von 2-Chlor-pyrimidinen der Formel (IV) mit Nucleophilen der Formel (VII), 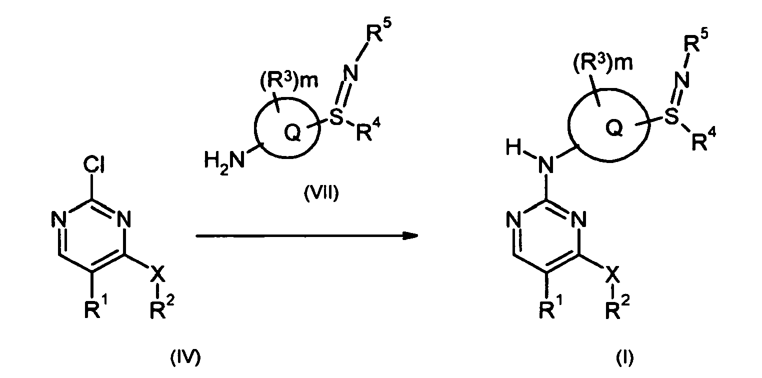 wobei Q, R¹, R², R³, R⁴, R⁵, X und m die in der allgemeinen Formel (I) gemäß der Ansprüche 1 bis 19 angegebenen Bedeutungen haben.

23. Verfahren zur Herstellung von Intermediaten der Formel (VII) umfassend die Schritte
a) Iminierung eines Thioether der Formel (IX) unter Erhalt von Sulfimiden der Formel (VIII) 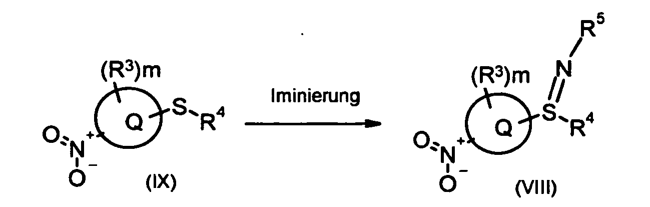
b) Reduktion der Nitro-Gruppe unter Erhalt der Intermediate der Formel (VII) 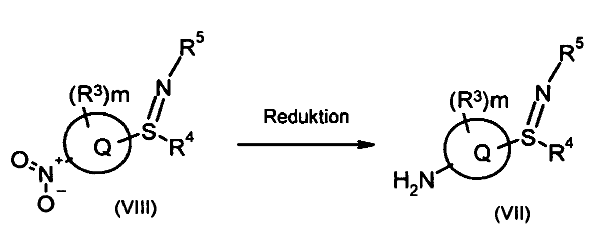
wobei Q, R³, R⁴ und R⁵ die in der allgemeinen Formel (I) gemäß der Ansprüche 1 bis 19 angegebenen Bedeutungen haben.

24. Verbindungen gemäß einem der Ansprüche 1 bis 19 zur Verwendung als Arzneimittel.

25. Verwendung einer Verbindung gemäß einem der Ansprüche 1 bis 19 zur Herstellung eines Medikamentes zur Behandlung von Krebs

26. Pharmazeutische Formulierung enthaltend eine Verbindung gemäß einem der Ansprüche 1 bis 19.

## Claims

1. Compounds of the general formula I, 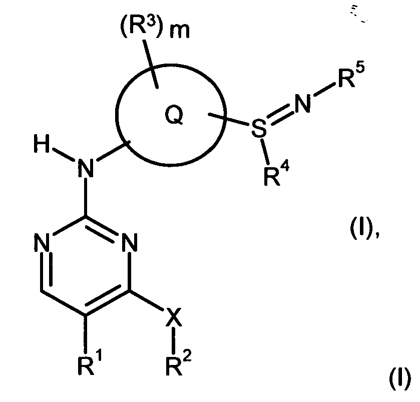 in which
R¹ is
(i) hydrogen, halogen, cyano, nitro, -NR⁸R⁹, -NR⁷-C(O)-R¹², -NR⁷-C(O)-OR¹², -NR⁷-C(O)-NR⁸R⁹, -NR⁷-SO₂-R¹²; -CF₃ or -OCF₃, or
(ii) a C₁-C₆-alkyl, C₂-C₆-alkenyl, C₁-C₆-alkoxy or C₂-C₆-alkynyl radical which is optionally substituted one or more times, identically or differently, by hydroxy, -NR⁸R⁹, -NR⁷-C(O)-R¹², -NR⁷-C(O)-OR¹², -NR⁷-C(O)-NR⁸R⁹, -NR⁷-SO₂-R¹², cyano, halogen, C₁-C₆-alkoxy, -CF₃ and/or -OCF₃, or
(iii) a phenyl or monocyclic heteroaryl ring which is optionally substituted one or more times, identically or differently, by hydroxy, -NR⁸R⁹, -NR⁷-C(O)-R¹², -NR⁷-C(O)-OR¹², -NR⁷-C(O)- NR⁸R⁹, -NR⁷-SO₂-R¹², cyano, halogen, -CF₃, C₁-C₆-alkoxy, -OCF₃ and/or C₁-C₆-alkyl,
R² is
(i) hydrogen or
(ii) a C₁-C₁₀-alkyl, C₂-C₁₀-alkenyl or C₂-C₁₀-alkynyl radical, a C₃-C₇- cycloalkyl, phenyl or naphthyl ring, a heterocyclyl ring having 3 to 8 ring atoms or a mono- or bicyclic heteroaryl ring, in each case optionally substituted one or more times, identically or differently, by
a) halogen, hydroxy, -NR⁸R⁹, -NR⁷-C(O)-R¹², -NR⁷-C(O)-OR¹², -NR⁷-C(O)-NR⁸R⁹, -NR⁷-SO₂-R¹², cyano, -C(O)R⁶, -O(CO)-R¹², -SO₂NR⁸R⁹, -SO₂-R¹², -S(O)(NR⁸)R¹², -(N)S(O)R¹³R¹⁴, -CF₃, -OCF₃, -N[(CO)-(C₁-C₆-alkyl)]₂ and/or
b) C₁-C₆-alkoxy, C₁-C₆-alkyl, C₂-C₆-alkenyl, C₂-C₆-alkynyl, C₃-C₈-cycloalkyl, phenyl, naphthyl, heterocyclyl having 3 to 8 ring atoms and/or a monocyclic or bicyclic heteroaryl, in each case optionally themselves substituted one or more times, identically or differently, by halogen, hydroxy, a C₁- C₆-alkyl, C₁-C₆-alkoxy, -NR⁸R⁹, -C(O)OR¹⁶, -SO₂NR⁸R⁹, -CF₃ or -OCF₃,
R³ is
(i) hydroxy, halogen, cyano, nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², and/or
(ii) a C₁-C₆-alkyl and/or C₁-C₆-alkoxy radical which is optionally substituted one or more times, identically or differently, by halogen, hydroxy, C₁-C₆-alkoxy, -CF₃, -OCF₃ or -NR⁸R⁹, and/or
(iii) a C₃-C₇-cycloalkyl ring which is optionally substituted one or more times, identically or differently, by halogen, hydroxy, C₁-C₆- alkoxy, -CF₃, -OCF₃, -NR⁸R⁹ and/or C₁-C₆-alkyl,
m is 0-4,
R⁴ is a C₁-C₆-alkyl, C₂-C₆-alkenyl, C₂-C₆-alkynyl radical, a C₃-C₇- cycloalkyl or phenyl ring, a heterocyclyl ring having 3 to 8 ring atoms or a monocyclic heteroaryl ring, in each case optionally themselves substituted one or more times, identically or differently, by hydroxy, -NR⁸R⁹, cyano, halogen, -CF₃, C₁-C₆-alkoxy, -OCF₃ and/or C₁-C₆- alkyl,
or
R³ and R⁴ together form a 5 to 7-membered ring which is fused to Q and which is optionally substituted one or more times, identically or differently, by hydroxy, C₁-C₆-alkyl, C₁-C₆-alkoxy, halogen or -NR⁸R⁹, and optionally comprises in addition to the double bond from Q a further double bond if the ring is 5-membered,
R⁵ is
-SO₂-(CH₂)ₙ-R¹² where n is 0 or 1,
-C(O)R¹², -C(O)OR¹², -C(O)NR⁸R⁹, -C(S)OR¹²,
-C(S)NR⁸R⁹ or -R¹²,
or
R⁴ and R⁵ together form a 5 to 7-membered ring of the formula 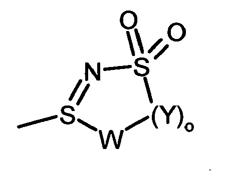 or 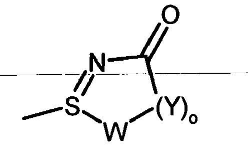 in which
W and Y are each independently of one another a -CH₂- group which is optionally substituted one or more times, identically or differently, by hydroxy, C₁-C₆-alkyl, C₁-C₆-alkoxy or -NR⁸R⁹, where the C₁-C₆-alkyl and/or C₁-C₆-alkoxy substituent is optionally itself substituted one or more times, identically or differently, by hydroxy, C₁-C₆-alkoxy or -NR⁸R⁹, and/or optionally comprises in addition to the imide double bond 1 or 2 further double bonds,
and
in which
o is 1-3
X is -O-, -S- or -NR¹⁵-,
where
R¹⁵ is
(i) hydrogen or
(ii) a C₁-C₆-alkyl radical, C₃-C₈-cycloalkyl or phenyl ring, a heterocyclyl ring having 3 to 8 ring atoms or a monocyclic heteroaryl ring, or
(iii) -C(O)-(C₁-C₆)-alkyl, -C(O)-phenyl, or -C(O)-benzyl and (ii) and (iii) are optionally substituted one or more times, identically or differently, by hydroxy, -NR¹⁰R¹¹, cyano, halogen, -CF₃, C₁-C₆-alkoxy and/or -OCF₃,
or
if X is -NR¹⁵-, alternatively
X, R¹⁵ and R² together form a 3 to 8 membered ring which optionally comprises in addition to the nitrogen atom one or more further heteroatoms, is optionally substituted one or more times, identically or differently, by hydroxy, C₁-C₆-alkyl, C₁-C₆-alkoxy, -C(O)R¹², -SO₂R¹², halogen or the group -NR⁸R⁹, optionally comprises 1 to 3 double bonds, and/or is optionally interrupted by one or more -C(O)- groups,
Q is a phenyl ring,
R⁶ is
(i) hydrogen or hydroxy, or
(ii) a C₁-C₆-alkyl, C₃-C₆-alkenyl, C₃-C₆-alkynyl or C₁-C₆-alkoxy radical, a C₃-C₇-cycloalkyl or phenyl ring, a heterocyclyl ring having 3 to 8 ring atoms or a monocyclic heteroaryl ring, in each case optionally themselves substituted one or more times, identically or differently, by hydroxy, -NR⁸R⁹, cyano, halogen, -CF₃, C₁-C₆-alkoxy and/or -OCF₃,
R⁷ is hydrogen or a C₁-C₆-alkyl radical,
R⁸ and R⁹ are independently of one another
(i) hydrogen and/or
(ii) a C₁-C₆-alkyl radical, C₂-C₆-alkenyl, C₃-C₈-cycloalkyl and/or phenyl ring, a heterocyclyl ring having 3 to 8 ring atoms and/or a monocyclic heteroaryl ring, optionally substituted one or more times, identically or differently, by hydroxy, -NR¹⁰R¹¹, cyano, halogen, -CF₃, C₁-C₆-alkoxy and/or -OCF₃,
or
R⁸ and R⁹ together with the nitrogen atom form a 5- to 7-membered ring which optionally comprises in addition to the nitrogen atom 1 or 2 further heteroatoms, and which may be substituted one or more times, identically or differently, by hydroxy, -NR¹⁰R¹¹, cyano, halogen, -CF₃, C₁-C₆-alkoxy and/or -OCF₃,
R¹⁰ and R¹¹ are independently of one another hydrogen or a C₁-C₆-alkyl radical which is optionally substituted one or more times, identically or differently, by hydroxy, cyano, halogen, -CF₃, C₁-C₆-alkoxy and/or -OCF₃,
R¹², R¹³, R¹⁴ are independently of one another -CF₃
or
a C₁-C₆-alkyl, C₂-C₆-alkenyl and/or C₂-C₆-alkynyl radical, a C₃-C₇- cycloalkyl or phenyl ring, a heterocyclyl ring having 3 to 8 ring atoms or a monocyclic heteroaryl ring,
which are optionally in each case themselves substituted one or more times, identically or differently, by hydroxy, nitro, -NR⁸R⁹, -NH-C(O)-C₁-C₆-alkyl, cyano, halogen, -CF₃, C₁-C₆-alkyl, C₁-C₆- alkoxy and/or -OCF₃,
R¹⁶ is
(i) hydrogen or
(ii) a C₁-C₆-alkyl, C₃-C₆-alkenyl, C₃-C₆-alkynyl radical, a C₃-C₇-cydoalkyl or phenyl ring, a heterocyclyl ring having 3 to 8 ring atoms or a monocyclic heteroaryl ring, in each case optionally themselves substituted one or more times, identically or differently, by hydroxy, -NR⁸R⁹, cyano, halogen, -CF₃, C₁-C₆- alkoxy and/or -OCF₃,
and the salts, diastereomers and enantiomers thereof.

2. Compounds of the general formula I according to Claim 1,
in which
R¹ is halogen, -CF₃, -OCF₃, C₁-C₄-alkyl or nitro,
R² is a C₁-C₁₀-alkyl, C₂-C₁₀-alkenyl or C₂-C₁₀-alkynyl radical, a C₃-C₇- cycloalkyl, phenyl or a mono- or bicyclic heteroaryl ring or a heterocyclyl ring having 3 to 7 ring atoms,
in each case optionally substituted one or more times, identically or differently, by hydroxy, -NR⁸R⁹, -NR⁷-C(O)-R¹² and/or a C₁-C₄-alkyl radical which is optionally itself substituted one or more times by hydroxy
R³ is
(i) hydroxy, halogen, cyano, nitro, -CF₃, -OCF₃, -NR⁸R⁹, -NR⁷-C(O)-R¹², -NR⁷-C(O)-OR¹², -NR⁷-C(O)-NR⁸R⁹, -NR⁷-SO₂-R¹², and/or
(ii) a C₁-C₃-alkyl and/or C₁-C₃-alkoxy radical which is optionally substituted one or more times, identically or differently, by halogen, hydroxy, C₁-C₆-alkoxy, -CF₃, -OCF₃ or -NR⁸R⁹,
m is 0 or 1,
R⁴ is a C₁-C₅-alkyl radical, a C₃-C₆-cycloalkyl or a phenyl ring, in each case optionally themselves substituted one or more times, identically or differently, by hydroxy, -NR⁸R⁹, cyano, halogen, -CF₃, C₁-C₆-alkoxy, -OCF₃ and/or C₁-C₆-alkyl,
or
R³ and R⁴ together form a 5-membered ring which is fused to Q and which optionally comprises in addition to the double bond from Q a further double bond,
R⁵ is -SO₂-(CH₂)ₙ-R¹² where n is 0 or 1,
where R¹² is CF₃
or is a C₁-C₄-alkyl radical, a C₃-C₆-cycloalkyl or phenyl ring or a heterocyclyl ring having 3 to 6 ring atoms or a monocyclic heteroaryl ring,
optionally in each case themselves substituted one or more times, identically or differently, by hydroxy, nitro, -NR⁸R⁹, cyano, halogen, -CF₃, C₁-C₆-alkyl, C₁-C₆-alkoxy and/or -OCF₃,
or
R⁴ and R⁵ together form a 5-membered ring of the formula (1) 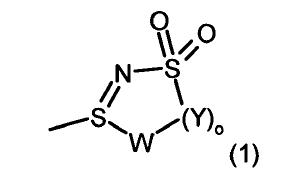 in which W and Y are each a -CH₂- group, and in which o is 1,
X is -O-, -S- or -NR¹⁵-,
where
R¹⁵ is
(i) hydrogen or
(ii) a C₁-C₆-alkyl radical, C₃-C₈-cycloalkyl or phenyl ring, a heterocyclyl ring having 3 to 8 ring atoms or a monocyclic heteroaryl ring, or
(iii) -C(O)-(C₁-C₆)-alkyl, -C(O)-phenyl, or -C(O)-benzyl, and (ii) and (iii) are optionally substituted one or more times, identically or differently, by hydroxy, -NR¹⁰R¹¹, cyano, halogen, -CF₃, C₁-C₆- alkoxy and/or -OCF₃,
or
if X is -NR¹⁵-, alternatively
X, R¹⁵ and R² together form a 3 to 8 membered ring which optionally comprises in addition to the nitrogen atom one or more further heteroatoms, is optionally substituted one or more times, identically or differently, by hydroxy, C₁-C₆-alkyl, C₁-C₆-alkoxy, -C(O)R¹², -SO₂R¹², halogen or the group -NR⁸R⁹, optionally comprises 1 to 3 double bonds, and/or is optionally interrupted by one or more -C(O)- groups,
Q is a phenyl ring,
R⁶ is a C₂-C₅-alkyl, C₄-C₆-alkenyl, C₄-C₆-alkynyl or C₂-C₅-alkoxy radical, a C₄-C₆-cycloalkyl or phenyl ring, a heterocyclyl ring having 3 to 5 ring atoms or a monocyclic heteroaryl ring, R⁷ in each case optionally themselves substituted one or more times, identically or differently, by hydroxy, -NR⁸R⁹, cyano, halogen, -CF₃, C₁-C₆-alkoxy and/or -OCF₃, is hydrogen or a C₁-C₆-alkyl radical,
R⁸ and R⁹ are each independently of one another hydrogen and/or a C₁-C₄- alkyl radical, C₃-C₆-cydoalkyl and/or phenyl ring, and/or a monocyclic heteroaryl ring,
in each case optionally substituted one or more times, identically or differently, by hydroxy, -NR¹⁰R¹¹ or C₁-C₆-alkoxy, or
R⁸ and R⁹ together with the nitrogen atom form a 5- to 7-membered ring which optionally comprises in addition to the nitrogen atom 1 further heteroatom, and which may be substituted one or more times by hydroxy,
R¹⁰ and R¹¹ are independently of one another hydrogen or a C₁-C₆-alkyl radical which is optionally substituted one or more times, identically or differently, by hydroxy,
R¹² is CF₃ or
is a C₁-C₆-alkyl, C₂-C₆-alkenyl or C₂-C₆-alkynyl radical, a C₃-C₇- cycloalkyl or phenyl ring, a heterocyclyl ring having 3 to 8 ring atoms or a monocyclic heteroaryl ring, in each case optionally themselves substituted one or more times, identically or differently, by hydroxy, halogen, nitro, -NR⁸R⁹, C₁-C₆- alkyl, and/or C₁-C₆-alkoxy,
R¹³ and R¹⁴ are independently of one another a C₁-C₆-alkyl radical, and
R¹⁶ is a C₁-C₆-alkyl radical, a C₃-C₇-cydoalkyl or phenyl ring, a heterocyclyl ring having 3 to 8 ring atoms or a monocyclic heteroaryl ring,
and the salts, diastereomers and enantiomers thereof.

3. Compounds of the general formula (I) according to either of Claims 1 or 2, in which
R¹ is bromine or CF₃,
and the salts, diastereomers and enantiomers thereof.

4. Compounds of the general formula (I) according to any of Claims 1 to 3, in which
R² is a C₁-C₆-alkyl radical, a C₃-C₇-cycloalkyl or phenyl ring, in each case optionally substituted one or more times, identically or differently, by hydroxy and/or -NH-C(O)-C₁-C₆-alkyl,
and the salts, diastereomers and enantiomers thereof.

5. Compounds of the general formula (I) according to any of Claims 1 to 4, in which
X is -NR¹⁵-, where R¹⁵ is hydrogen,
and the salts, diastereomers and enantiomers thereof.

6. Compounds of the general formula (I) according to any of Claims 1 to 5, in which
R³ is hydroxy, fluorine, chlorine, bromine, cyano, nitro, -CF₃, methyl or methoxy, and the salts, diastereomers and enantiomers thereof.

7. Compounds of the general formula (I) according to any of Claims 1 to 6, in which m is 0,
and the salts, diastereomers and enantiomers thereof.

8. Compounds of the general formula (I) according to any of Claims 1 to 7, in which
R⁴ is a C₁-C₄-alkyl radical or a C₃-C₅-cycloalkyl ring,
in each case optionally itself substituted one or more times, identically or differently, by hydroxy, -NR⁸R⁹ or halogen,
and the salts, diastereomers and enantiomers thereof.

9. Compounds of the general formula (I) according to any of Claims 1 to 8, in which
R⁵ is -SO₂R¹²,
where
R¹² is a C₁-C₆-alkyl radical, a phenyl or a monocyclic heteroaryl ring, optionally in each case themselves substituted one or more times, identically or differently, by nitro, halogen and/or C₁-C₆-alkyl,
and the salts, diastereomers and enantiomers thereof.

10. Compounds of the general formula (I) according to any of Claims 1 to 8, in which
R⁵ is -SO₂-(CH₂)ₙ-R¹² where n is 0 or 1,
where
R¹² is -CF₃
or is a C₁-C₆-alkyl radical, a phenyl or a monocyclic heteroaryl ring, which are optionally in each case themselves substituted one or more times, identically or differently, by nitro, -NH-C(O)-C₁-C₆-alkyl, halogen, C₁-C₆-alkyl, C₁-C₆-alkoxy and/or -OCF₃,
and the salts, diastereomers and enantiomers thereof.

11. Compounds of the general formula (I) according to any of Claims 1 to 10, in which
R⁶ is a C₁-C₆-alkyl, a C₁-C₆-alkoxy radical or a C₃-C₇-cycloalkyl ring, in each case optionally themselves substituted one or more times, identically or differently, by hydroxy, -NR⁸R⁹ and/or C₁-C₆-alkoxy, and the salts, diastereomers and enantiomers thereof.

12. Compounds of the general formula (I) according to any of Claims 1 to 11, in which
R⁷ is hydrogen or a C₁-C₆-alkyl radical,
and the salts, diastereomers and enantiomers thereof.

13. Compounds of the general formula (I) according to any of Claims 1 to 12, in which
R⁸ and R⁹ are hydrogen and/or a C₁-C₆-alkyl radical, a C₃-C₆-cydoalkyl and/or phenyl ring, and/or a monocyclic heteroaryl ring,
or
R⁸ and R⁹ form together with the nitrogen atom a 5- or 6-membered ring which optionally comprises in addition to the nitrogen atom 1 further heteroatom,
and the salts, diastereomers and enantiomers thereof.

14. Compounds of the general formula (I) according to any of Claims 1 to 13, in which
R¹⁰ and R¹¹ are independently of one another hydrogen or a methyl group,
and the salts, diastereomers and enantiomers thereof.

15. Compounds of the general formula (I) according to any of Claims 1 to 14, in which
R¹² is a C₁-C₆-alkyl radical, a phenyl or monocyclic heteroaryl ring,
in each case optionally themselves substituted one or more times, identically or differently, by hydroxy, halogen, nitro or C₁-C₆-alkyl,
and the salts, diastereomers and enantiomers thereof.

16. Compounds of the general formula (I) according to any of Claims 1 to 15, in which
R¹³ and R¹⁴ are independently of one another a C₁-C₆-alkyl radical,
and the salts, diastereomers and enantiomers thereof.

17. Compounds of the general formula (I) according to any of Claims 1 to 16, in which
R¹⁶ is a C₁-C₆-alkyl radical,
and the salts, diastereomers and enantiomers thereof.

18. Compounds according to the general formula (I) of Claim 1, in which
R¹ is hydrogen, halogen or -CF₃,
R² is a C₁-C₁₀-alkyl radical, a C₃-C₇-cydoalkyl or phenyl ring, in each case optionally substituted one or more times, identically or differently, by hydroxy or -NH-C(O)-C₁-C₆-alkyl
m is 0,
R⁴ is a C₁-C₆-alkyl radical,
R⁵ is -SO₂-(CH₂)ₙ-R¹² where n is 0 or 1,
X is -NH-,
Q is a phenyl ring,
R¹² is -CF₃
or
is a C₁-C₆-alkyl radical, a phenyl or a monocyclic heteroaryl ring, which are in each case optionally themselves substituted one or more times, identically or differently, by nitro, halogen, -CF₃, C₁-C₆- alkyl, -NH-C(O)-C₁-C₆-alkyl, C₁-C₆-alkoxy and/or -OCF₃,
and the salts, diastereomers and enantiomers thereof.

19. Compounds according to formula (I) of Claim 1,
in which
R¹ is hydrogen or halogen,
R² is a C₁-C₁₀-alkyl radical, a C₃-C₇-cycloalkyl or phenyl ring, in each case optionally substituted one or more times, identically or differently, by hydroxy, C₁-C₆-alkyl radical or -NR⁷-C(O)-R¹²,
m is 0,
R⁴ is a C₁-C₆-alkyl radical,
R⁵ is -SO₂R¹²,
X is -NR¹⁵-, where R¹⁵ is hydrogen,
Q is a phenyl ring,
R¹² is a C₁-C₆-alkyl radical, a phenyl or a monocyclic heteroaryl ring, in each case optionally themselves substituted one or more times, identically or differently, by nitro, halogen or C₁-C₆-alkyl,
and the salts, diastereomers and enantiomers thereof.

20. Process for preparing the compounds according to any of Claims 1 to 19, comprising the steps
a) reaction of 2-chloropyrimidines of the formula (IV) with nucleophiles of the formula (III) to give compounds of the formula (II) 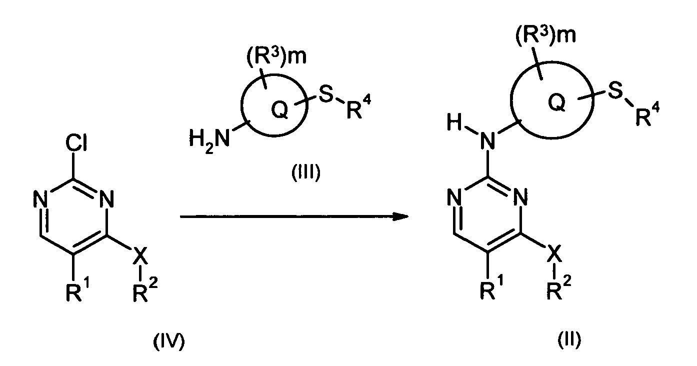
b) imination of the thioethers of the formula (II) to obtain compounds of the formula (I) 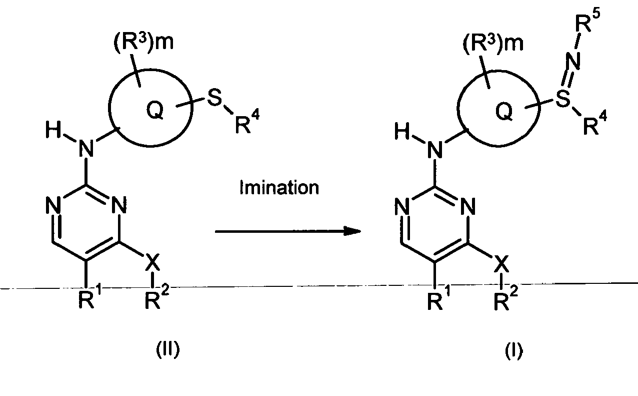
where Q, R¹, R², R³, R⁴, R⁵, X and m have the meanings indicated in the general formula (I) according to Claims 1 to 20.

21. Process for preparing intermediates of the formula (IV) by reacting 2,4-dichloropyrimidines of the formula (VI) with nucleophiles of the formula (V) 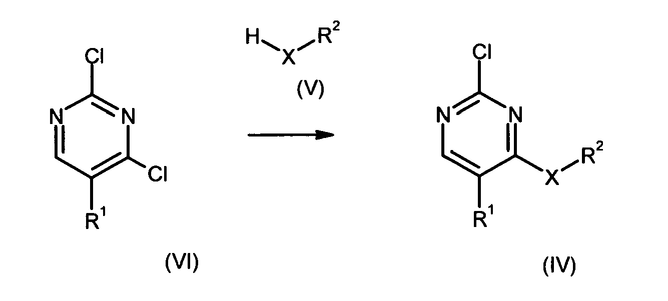 where R¹, R² and X have the meanings indicated in the general formula (I) according to Claims 1 to 19.

22. Process for preparing the compounds of the formula (I) according to any of Claims 1 to 19 by reacting 2-chloropyrimidines of the formula (IV) with nucleophiles of the formula (VII) 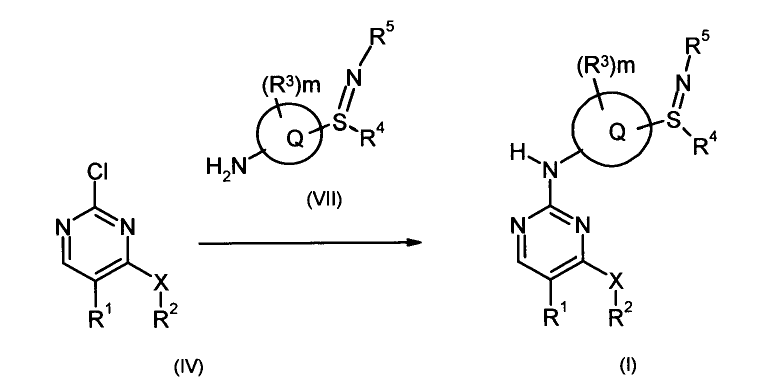 where Q, R¹, R², R³, R⁴, R⁵, X and m have the meanings indicated in the general formula (I) according to Claims 1 to 19.

23. Process for preparing intermediates of the formula (VII) comprising the steps
a) imination of a thioether of the formula (IX) to obtain sulphimides of the formula (VIII) 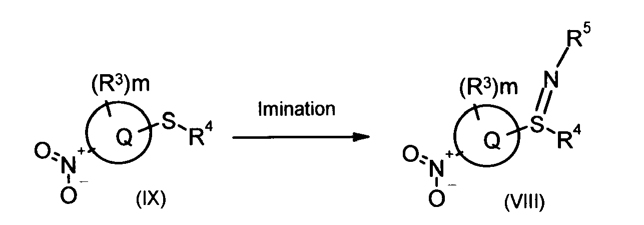
b) reduction of the nitro group to obtain the intermediates of the formula (VII) 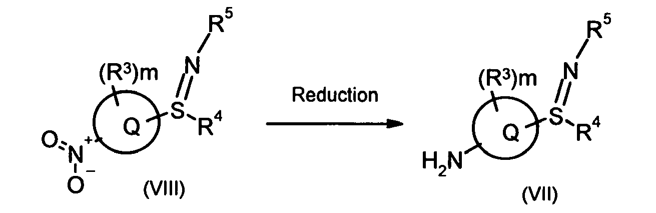
where Q, R³, R⁴ and R⁵ have the meanings indicated in the general formula (I) according to Claims 1 to 19.

24. Compounds according to any of Claims 1 to 19 for use as medicament.

25. Use of a compound according to any of Claims 1 to 19 for producing a medicament for the treatment of cancer.

26. Pharmaceutical formulation comprising a compound according to any of Claims 1 to 19.

## Revendications

1. Composés de formule générale I 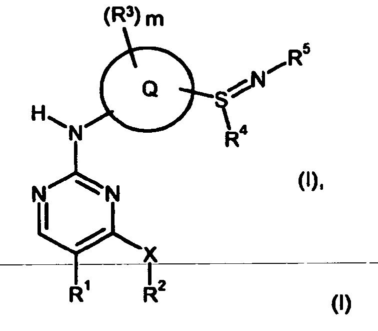 où
R¹ représente
(i) hydrogène, halogène, cyano, nitro, -NR⁸R⁹, -NR⁷-C(O)-R¹², -NR⁷-C(O)-OR¹², -NR⁷-C(O)-NR⁸R⁹, NR⁷-SO₂-R¹², -CF₃ ou -OCF₃, ou
(ii) un radical C₁-C₆-alkyle, C₂-C₆-alcényle, C₁-C₆- alcoxy ou C₂-C₆-alcynyle le cas échéant monosubstitué ou polysubstitué, de manière identique ou différente, par hydroxy, -NR⁸R⁹, -NR⁷-C(O)-R¹², -NR⁷-C(O)-OR¹², -NR⁷-C(O)-NR⁸R⁹, -NR⁷-SO₂-R¹², cyano, halogène, C₁-C₆-alcoxy, -CF₃ et/ou -OCF₃, ou
(iii) un cycle phényle ou hétéroaryle monocyclique, le cas échéant monosubstitué ou polysubstitué, de manière identique ou différente, par hydroxy, -NR⁸R⁹, -NR⁷-C(O)-R¹², -NR⁷-C(O)-OR¹², -NR⁷-C(O)-NR⁸R⁹, -NR⁷-SO₂-R¹², cyano, halogène, -CF₃, C₁-C₆-alcoxy, -OCF₃ et/ou C₁-C₆-alkyle,
R² représente
(i) hydrogène ou
(ii) un radical C₁-C₁₀-alkyle, C₂-C₁₀-alcényle ou C₂- C₁₀-alcynyle, un cycle C₃-C₇-cycloalkyle, phényle ou naphtyle, un cycle hétérocyclyle comprenant 3 à 8 atomes de cycle ou un cycle hétéroaryle monocyclique ou bicyclique,
à chaque fois le cas échéant monosubstitués ou polysubstitués, de manière identique ou différente, par
a) halogène, hydroxy, -NR⁸R⁹, -NR⁷-C(O)-R¹², -NR⁷-C(O)-OR¹², -NR⁷-C(O)-NR⁸R⁹, -NR⁷-SO₂- R¹², cyano, -C(O)R⁶, -O(CO)-R¹², -SO₂NR⁸R⁹, -SO₂-R¹², -S(O)(NR⁸)R¹², -(N)S(O)R¹³R¹⁴, -CF₃, -OCF₃, -N[(CO)-(C₁-C₆-alkyle)]₂ et/ou
b) C₁-C₆-alcoxy, C₁-C₆-alkyle, C₂-C₆- alcényle, C₂-C₆-alcynyle, C₃-C₈- cycloalkyle, phényle, naphtyle, hétérocyclyle comprenant 3 à 8 atomes de cycle et/ou hétéroaryle monocyclique ou bicyclique, à chaque fois le cas échéant monosubstitués ou polysubstitués eux- mêmes, de manière identique ou différente, par halogène, hydroxy, C₁-C₆- alkyle, C₁-C₆-alcoxy, -NR⁸R⁹, -C(O)OR¹⁶, -SO₂NR⁸R⁹, -CF₃ ou -OCF₃,
R³ représente
(i) hydroxy, halogène, cyano, nitro, -CF₃, -OCF₃, -C(O)NR⁸R⁹, -C(S)NR⁸R⁹, -NR⁸R⁹, -NR⁷-C(O)-R¹², -NR⁷-C(O)-OR¹², -NR⁷-C(O)-NR⁸R⁹, -NR⁷-SO₂-R¹², et/ou
(ii) un radical C₁-C₆-alkyle et/ou C₁-C₆-alcoxy, le cas échéant monosubstitué ou polysubstitué, de manière identique ou différente, par halogène, hydroxy, C₁-C₆-alcoxy, -CF₃, -OCF₃ ou -NR⁸R⁹, et/ou
(iii) un cycle C₃-C₇-cycloalkyle le cas échéant monosubstitué ou polysubstitué, de manière identique ou différente, par halogène, hydroxy, C₁-C₆-alcoxy, -CF₃, -OCF₃, -NR⁸R⁹ et/ou C₁-C₆-alkyle,
m vaut 0-4,
R⁴ représente un radical C₁-C₆-alkyle, C₂-C₆-alcényle, C₂-C₆-alcynyle, un cycle C₃-C₇-cycloalkyle ou phényle, un cycle hétérocyclyle comprenant 3 à 8 atomes de cycle ou un cycle hétéroaryle monocyclique, à chaque fois le cas échéant monosubstitués ou polysubstitués eux-mêmes, de manière identique ou différente, par hydroxy, -NR⁸R⁹, cyano, halogène, -CF₃, C₁-C₆-alcoxy, -OCF₃ et/ou C₁-C₆-alkyle,
ou
R³ et R⁴ forment ensemble un cycle de 5 à 7 chaînons condensé sur Q, qui est le cas échéant monosubstitué ou polysubstitué, de manière identique ou différente, par hydroxy, C₁-C₆-alkyle, C₁-C₆-alcoxy, halogène ou -NR⁸R⁹ et qui contient le cas échéant en plus de la double liaison de Q une autre double liaison, lorsque le cycle est à 5 chaînons
R⁵ représente
-SO₂-(CH₂)ₙ-R¹² avec n valant 0 ou 1, -C(O)R¹²,
-C(O)OR¹², -C(O)NR⁸R⁹, -C(S)OR¹², -C(S)NR⁸R⁹ ou -R¹²,
ou
R⁴ et R⁵ forment ensemble un cycle de 5 à 7 chaînons de formule 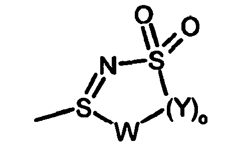 ou 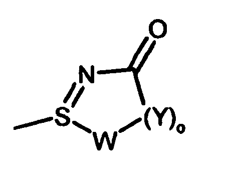 où
W et Y représentent à chaque fois indépendamment l'un de l'autre un groupe -CH₂- le cas échéant monosubstitué ou polysubstitué, de manière identique ou différente, par hydroxy, C₁-C₆-alkyle, C₁-C₆- alcoxy ou -NR⁸R⁹, où
le substituant C₁-C₆-alkyle et/ou C₁-C₆-alcoxy est le cas échéant monosubstitué ou polysubstitué lui- même, de manière identique ou différente, par hydroxy, C₁-C₆-alcoxy ou -NR⁸R⁹ et/ou contient le cas échéant, en plus de la double liaison imide, 1 ou 2 autres doubles liaisons
et
où
O vaut 1-3
X représente -O-, -S- ou -NR¹⁵-,
où
R¹⁵ représente
(i) hydrogène ou
(ii) un radical C₁-C₆-alkyle, un cycle C₃-C₈- cycloalkyle ou phényle, un cycle hétérocyclyle comprenant 3 à 8 atomes de cycle ou un cycle hétéroaryle monocyclique, ou
(iii) -C(O)-(C₁-C₆)-alkyle, -C(O)-phényle, ou -C(O)-benzyle, et (ii) et (iii) sont le cas échéant monosubstitués ou polysubstitués, de manière identique ou différente, par hydroxy, -NR¹⁰R¹¹, cyano, halogène, -CF₃, C₁-C₆-alcoxy et/ou -OCF₃,
ou
lorsque X représente -NR¹⁵-, en variante
X, R¹⁵ et R² forment ensemble un cycle de 3 à 8 chaînons, qui contient le cas échéant en plus de l'atome d'azote un ou plusieurs autres hétéroatomes, qui est le cas échéant monosubstitué ou polysubstitué, de manière identique ou différente, par hydroxy, C₁-C₆- alkyle, C₁-C₆-alcoxy, -C(O)R¹², -SO₂R¹², halogène ou le groupe -NR⁸R⁹, qui contient le cas échéant 1 à 3 doubles liaisons et/ou qui est le cas échéant interrompu par un ou plusieurs groupes -C(O)-,
Q représente un cycle phényle,
R⁶ représente
(i) hydrogène ou hydroxy, ou
(ii) un radical C₁-C₆-alkyle, C₃-C₆-alcényle, C₃-C₆- alcynyle ou C₁-C₆-alcoxy, un cycle C₃-C₇- cycloalkyle ou phényle, un cycle hétérocyclyle comprenant 3 à 8 atomes de cycle ou un cycle hétéroaryle monocyclique, à chaque fois le cas échéant monosubstitués ou polysubstitués eux-mêmes, de manière identique ou différente, par hydroxy, -NR⁸R⁹, cyano, halogène, -CF₃, C₁-C₆-alcoxy et/ou -OCF₃,
R⁷ représente hydrogène ou un radical C₁-C₆-alkyle,
R⁸ et R⁹ représentent indépendamment l'un de l'autre
(i) hydrogène et/ou
(ii) un radical C₁-C₆-alkyle, un cycle C₂-C₆- alcényle, C₃-C₈-cycloalkyle et/ou phényle, un cycle hétérocyclyle comprenant 3 à 8 atomes de cycle et/ou un cycle hétéroaryle monocyclique, le cas échéant monosubstitués ou polysubstitués, de manière identique ou différente par hydroxy, -NR¹⁰R¹¹, cyano, halogène, -CF₃, C₁-C₆-alcoxy et/ou -OCF₃,
ou
R⁸ et R⁹ forment ensemble avec l'atome d'azote un cycle de 5 à 7 chaînons, qui contient le cas échéant, en plus de l'atome d'azote, 1 ou 2 autres hétéroatomes et qui peut être monosubstitué ou polysubstitué, de manière identique ou différente, par hydroxy, -NR¹⁰R¹¹, cyano, halogène, -CF₃, C₁-C₆- alcoxy et/ou -OCF₃,
R¹⁰ et R¹¹ représentent, indépendamment l'un de l'autre, hydrogène ou un radical C₁-C₆-alkyle, qui est le cas échéant monosubstitué ou polysubstitué, de manière identique ou différente, par hydroxy, cyano, halogène, -CF₃, C₁-C₆-alcoxy et/ou -OCF₃,
R¹², R¹³, R¹⁴ représentent indépendamment l'un de l'autre -CF₃
ou
un radical C₁-C₆-alkyle, C₂-C₆-alcényle et/ou C₂-C₆- alcynyle, un cycle C₃-C₇-cycloalkyle ou phényle, un cycle hétérocyclyle comprenant 3 à 8 atomes de cycle ou un cycle hétéroaryle monocyclique, qui sont le cas échéant à chaque fois monosubstitués ou polysubstitués eux-mêmes, de manière identique ou différente, par hydroxy, nitro, -NR⁸R⁹, -NH- C(O)-C₁-C₆-alkyle, cyano, halogène, -CF₃, C₁-C₆- alkyle, C₁-C₆-alcoxy et/ou -OCF₃,
R¹⁶ représente
(i) hydrogène ou
(ii) un radical C₁-C₆-alkyle, C₃-C₆-alcényle, C₃-C₆- alcynyle, un cycle C₃-C₇-cycloalkyle ou phényle, un cycle hétérocyclyle comprenant 3 à 8 atomes de cycle ou un cycle hétéroaryle monocyclique, à chaque fois le cas échéant monosubstitués ou polysubstitués eux-mêmes, de manière identique ou différente, par hydroxy, -NR⁸R⁹, cyano, halogène, -CF₃, C₁-C₆- alcoxy et/ou -OCF₃,
ainsi que leurs sels, diastéréo-isomères et énantiomères.

2. Composés de formule générale I selon la revendication 1, où
R¹ représente halogène, -CF₃, -OCF₃, C₁-C₄-alkyle ou nitro,
R² représente un radical C₁-C₁₀-alkyle, C₂-C₁₀-alcényle ou C₂-C₁₀-alcynyle, un cycle C₃-C₇-cycloalkyle, phényle ou un cycle hétéroaryle monocyclique ou bicyclique ou un cycle hétérocyclyle comprenant 3 à 7 atomes de cycle,
à chaque fois le cas échéant monosubstitués ou polysubstitués, de manière identique ou différente, par hydroxy, -NR⁸R⁹, -NR⁷-C(O)-R¹² et/ou un radical C₁-C₄-alkyle, qui est le cas échéant monosubstitué ou polysubstitué lui-même par hydroxy
R³ représente
(i) hydroxy, halogène, cyano, nitro, -CF₃, -OCF₃, -NR⁸R⁹, -NR⁷-C(O)-R¹², -NR⁷-C(O)-OR¹², -NR⁷- C (O) -NR⁸R⁹, -NR⁷-SO₂-R¹², et/ou
(ii) un radical C₁-C₃-alkyle et/ou C₁-C₃-alcoxy, le cas échéant monosubstitué ou polysubstitué, de manière identique ou différente, par halogène, hydroxy, C₁-C₆-alcoxy, -CF₃, -OCF₃ ou -NR⁸R⁹,
m vaut 0 ou 1,
R⁴ représente un radical C₁-C₅-alkyle, un cycle C₃-C₆- cycloalkyle ou phényle,
à chaque fois le cas échéant monosubstitués ou polysubstitués eux-mêmes, de manière identique ou différente, par hydroxy, -NR⁸R⁹, cyano, halogène, -CF₃, C₁-C₆-alcoxy, -OCF₃ et/ou C₁-C₆-alkyle ou
R³ et R⁴ forment ensemble un cycle de 5 chaînons condensé sur Q, qui contient le cas échéant en plus de la double liaison de Q, une autre double liaison,
R⁵ représente -SO₂-(CH₂)ₙ-R¹² avec n valant 0 ou 1, où R¹² représente CF₃
ou un radical C₁-C₄-alkyle, un cycle C₃-C₆- cycloalkyle ou phényle ou un cycle hétérocyclyle comprenant 3 à 6 atomes de cycle ou un cycle hétéroaryle monocyclique,
à chaque fois le cas échéant monosubstitués ou polysubstitués eux-mêmes, de manière identique ou différente, par hydroxy, nitro, -NR⁸R⁹, cyano, halogène, 4-CF₃, C₁-C₆-alkyle, C₁-C₆-alcoxy, et/ou -OCF₃,
ou
R⁴ et R⁵ forment ensemble un cycle de 5 chaînons de formule (1) 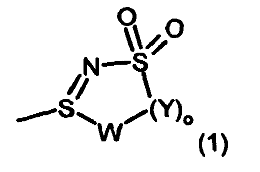 où W et Y représentent à chaque fois un groupe -CH₂- et où O vaut 1,
X représente -O-, -S- ou -NR¹⁵-,
où
R¹⁵ représente
(i) hydrogène ou
(ii) un radical C₁-C₆-alkyle, un cycle C₃-C₈- cycloalkyle ou phényle, un cycle hétérocyclyle comprenant 3 à 8 atomes de cycle ou un cycle hétéroaryle monocyclique, ou
(iii) -C(O)-(C₁-C₆)-alkyle, -C(O)-phényle, ou -C(O)-benzyle, et (ii) et (iii) sont le cas échéant monosubstitués ou polysubstitués, de manière identique ou différente, par hydroxy, -NR¹⁰R¹¹, cyano, halogène, -CF₃, C₁-C₆-alcoxy et/ou -OCF₃,
ou
lorsque X représente -NR¹⁵-, en variante
X, R¹⁵ et R² forment ensemble un cycle de 3 à 8 chaînons, qui contient le cas échéant en plus de l'atome d'azote un ou plusieurs autres hétéroatomes, qui est le cas échéant monosubstitué ou polysubstitué, de manière identique ou différente, par hydroxy, C₁-C₆- alkyle, C₁-C₆-alcoxy, -C(O)R¹², -SO₂R¹², halogène ou le groupe -NR⁸R⁹, qui contient le cas échéant 1 à 3 doubles liaisons et/ou qui est le cas échéant interrompu par un ou plusieurs groupes -C(O)-,
Q représente un cycle phényle,
R⁶ représente un radical C₂-C₅-alkyle, C₄-C₆-alcényle, C₄-C₆-alcynyle ou C₂-C₅-alcoxy, un cycle C₄-C₆- cycloalkyle ou phényle, un cycle hétérocyclyle comprenant 3 à 5 atomes de cycle ou un cycle hétéroaryle monocyclique, à chaque fois le cas échéant monosubstitués ou polysubstitués eux-mêmes, de manière identique ou différente, par hydroxy, -NR⁸R⁹, cyano, halogène, -CF₃, C₁-C₆-alcoxy et/ou -OCF₃
R⁷ représente hydrogène ou un radical C₁-C₆-alkyle,
R⁸ et R⁹ représentent à chaque fois indépendamment l'un de l'autre hydrogène et/ou un radical C₁-C₄-alkyle, un cycle C₃-C₆-cycloalkyle et/ou phényle et/ou un cycle hétéroaryle monocyclique, à chaque fois le cas échéant monosubstitués ou polysubstitués, de manière identique ou différente, par hydroxy, -NR¹⁰R¹¹ ou C₁-C₆-alcoxy, ou
R⁸ et R⁹ forment ensemble avec l'atome d'azote un cycle de 5 à 7 chaînons, qui contient le cas échéant, en plus de l'atome d'azote, 1 autre hétéroatome et qui peut être monosubstitué ou polysubstitué par hydroxy,
R¹⁰ et R¹¹ représentent, indépendamment l'un de l'autre, hydrogène ou un radical C₁-C₆-alkyle, qui est le cas échéant monosubstitué ou polysubstitué, de manière identique ou différente, par hydroxy,
R¹² représente CF₃ ou
un radical C₁-C₆-alkyle, C₂-C₆-alcényle ou C₂-C₆- alcynyle, un cycle C₃-C₇-cycloalkyle ou phényle, un cycle hétérocyclyle comprenant 3 à 8 atomes de cycle ou un cycle hétéroaryle monocyclique,
à chaque fois le cas échéant monosubstitués ou polysubstitués eux-mêmes, de manière identique ou différente, par hydroxy, halogène, nitro, -NR⁸R⁹, C₁-C₆-alkyle et/ou C₁-C₆-alcoxy,
R¹³ et représentent, indépendamment l'un de l'autre, un radical C₁-C₆-alkyle, et
R¹⁶ représente un radical C₁-C₆-alkyle, un cycle C₃-C₇- cycloalkyle ou phényle, un cycle hétérocyclyle comprenant 3 à 8 atomes de cycle ou un cycle hétéroaryle monocyclique,
ainsi que leurs sels, diastéréo-isomères et énantiomères.

3. Composés de formule générale (I) selon l'une quelconque des revendications 1 ou 2, où
R¹ représente brome ou CF₃,
ainsi que leurs sels, diastéréo-isomères et énantiomères.

4. Composés de formule générale (I) selon l'une quelconque des revendications 1 à 3, où
R² représente un radical C₁-C₆-alkyle, un cycle C₃-C₇- cycloalkyle ou phényle, à chaque fois le cas échéant monosubstitués ou polysubstitués, de manière identique ou différente, par hydroxy et/ou -NH-C(O)-C₁-C₆-alkyle,
ainsi que leurs sels, diastéréo-isomères et énantiomères.

5. Composés de formule générale (I) selon l'une quelconque des revendications 1 à 4, où
X représente -NR¹⁵-, où R¹⁵ représente hydrogène, ainsi que leurs sels, diastéréo-isomères et énantiomères.

6. Composés de formule générale (I) selon l'une quelconque des revendications 1 à 5, où
R³ représente hydroxy, fluor, chlore, brome, cyano, nitro, -CF₃, méthyle ou méthoxy,
ainsi que leurs sels, diastéréo-isomères et énantiomères.

7. Composés de formule générale (I) selon l'une quelconque des revendications 1 à 6, où m vaut 0, ainsi que leurs sels, diastéréo-isomères et énantiomères.

8. Composés de formule générale (I) selon l'une quelconque des revendications 1 à 7, où
R⁴ représente un radical C₁-C₄-alkyle ou un cycle C₃-C₅-cycloalkyle,
à chaque fois le cas échéant monosubstitués ou polysubstitués eux-mêmes, de manière identique ou différente, par hydroxy, -NR⁸R⁹ ou halogène, ainsi que leurs sels, diastéréo-isomères et énantiomères.

9. Composés de formule générale (I) selon l'une quelconque des revendications 1 à 8, où
R⁵ représente -SO₂R¹²,
où
R¹² représente un radical C₁-C₆-alkyle, un cycle phényle ou hétéroaryle monocyclique, le cas échéant monosubstitués ou polysubstitués eux- mêmes, de manière identique ou différente, par nitro, halogène et/ou C₁-C₆-alkyle,
ainsi que leurs sels, diastéréo-isomères et énantiomères.

10. Composés de formule générale (I) selon l'une quelconque des revendications 1 à 8, où
R⁵ représente -SO₂-(CH₂)ₙ-R¹² avec n valant 0 ou 1,
où
R¹² représente -CF₃ ou un radical C₁-C₆-alkyle, un cycle phényle ou hétéroaryle monocyclique, qui sont à chaque fois le cas échéant monosubstitués ou polysubstitués eux-mêmes, de manière identique ou différente, par nitro, -NH- C (O)-C₁-C₆-alkyle, halogène, C₁-C₆-alkyle, C₁-C₆- alcoxy et/ou -OCF₃,
ainsi que leurs sels, diastéréo-isomères et énantiomères.

11. Composés de formule générale (I) selon l'une quelconque des revendications 1 à 10, où R⁶ représente un radical C₁-C₆-alkyle, C₁-C₆-alcoxy ou
un cycle C₃-C₇-cycloalkyle, le cas échéant à chaque fois monosubstitués ou polysubstitués eux-mêmes, de manière identique ou différente, par hydroxy, -NR⁸R⁹ et/ou C₁-C₆-alcoxy,
ainsi que leurs sels, diastéréo-isomères et énantiomères.

12. Composés de formule générale (I) selon l'une quelconque des revendications 1 à 11, où
R⁷ représente hydrogène ou un radical C₁-C₆-alkyle, ainsi que leurs sels, diastéréo-isomères et énantiomères.

13. Composés de formule générale (I) selon l'une quelconque des revendications 1 à 12, où
R⁸ et R⁹ représentent hydrogène et/ou un radical C₁-C₆-alkyle, un cycle C₃-C₆-cycloalkyle et/ou phényle et/ou un cycle hétéroaryle monocyclique, ou
R⁸ et R⁹ forment ensemble avec l'atome d'azote un cycle de 5 ou 6 chaînons, qui contient le cas échéant en plus de l'atome d'azote 1 autre hétéroatome,
ainsi que leurs sels, diastéréo-isomères et énantiomères.

14. Composés de formule générale (I) selon l'une quelconque des revendications 1 à 13, où
R¹⁰ et R¹¹ représentent, indépendamment l'un de l'autre, hydrogène ou un groupe méthyle,
ainsi que leurs sels, diastéréo-isomères et énantiomères.

15. Composés de formule générale (I) selon l'une quelconque des revendications 1 à 14, où
R¹² représente un radical C₁-C₆-alkyle, un cycle phényle ou hétéroaryle monocyclique,
à chaque fois le cas échéant monosubstitués ou polysubstitués eux-mêmes, de manière identique ou différente, par hydroxy, halogène, nitro ou C₁-C₆-alkyle,
ainsi que leurs sels, diastéréo-isomères et énantiomères.

16. Composés de formule générale (I) selon l'une quelconque des revendications 1 à 15, où
R¹³ et R¹⁴ représentent, indépendamment l'un de l'autre, un radical C₁-C₆-alkyle,
ainsi que leurs sels, diastéréo-isomères et énantiomères.

17. Composés de formule générale (I) selon l'une quelconque des revendications 1 à 16, où
R¹⁶ représente un radical alkyle en C₁-C₆,
ainsi que leurs sels, diastéréo-isomères et énantiomères.

18. Composés de formule générale (I) selon la revendication 1, où
R¹ représente hydrogène, halogène ou -CF₃
R² représente un radical C₁-C₁₀-alkyle, un cycle C₃-C₇- cycloalkyle ou phényle, à chaque fois le cas échéant monosubstitués ou polysubstitués, de manière identique ou différente, par hydroxy ou -NH-C (O)-C₁-C₆-alkyle,
m vaut 0,
R⁴ représente un radical alkyle en C₁-C₆,
R⁵ représente -SO₂-(CH₂)ₙ-R¹² avec n valant 0 ou 1,
X représente -NH-,
Q représente un cycle phényle,
R¹² représente -CF₃
ou
représente un radical C₁-C₆-alkyle, un cycle phényle ou hétéroaryle monocyclique, qui sont le cas échéant à chaque fois monosubstitués ou polysubstitués eux-mêmes, de manière identique ou différente, par nitro, halogène, -CF₃, C₁-C₆- alkyle, -NH-C(O)-C₁-C₆-alkyle, C₁-C₆-alcoxy et/ou -OCF₃,
ainsi que leurs sels, diastéréo-isomères et énantiomères.

19. Composés de formule (I) selon la revendication 1, où
R¹ représente hydrogène ou halogène,
R² représente un radical C₁-C₁₀-alkyle, un cycle C₃-C₇- cycloalkyle ou phényle, à chaque fois le cas échéant monosubstitués ou polysubstitués, de manière identique ou différente, par hydroxy, un radical C₁-C₆-alkyle ou -NR⁷-C(O)-R¹²,
m vaut 0,
R⁴ représente un radical alkyle en C₁-C₆,
R⁵ représente -SO₂R¹²,
X représente -NR¹⁵-, où R¹⁵ représente hydrogène,
Q représente un cycle phényle,
R¹² représente un radical C₁-C₆-alkyle, un cycle phényle ou hétéroaryle monocyclique,
à chaque fois le cas échéant monosubstitués ou polysubstitués eux-mêmes, de manière identique ou différente, par nitro, halogène ou C₁-C₆-alkyle,
ainsi que leurs sels, diastéréo-isomères et énantiomères.

20. Procédé pour la préparation des composés selon l'une quelconque des revendications 1 à 19, comprenant les étapes
a) transformation de 2-chloropyrimidines de formule (IV) avec des nucléophiles de formule (III) en composés de formule (II) 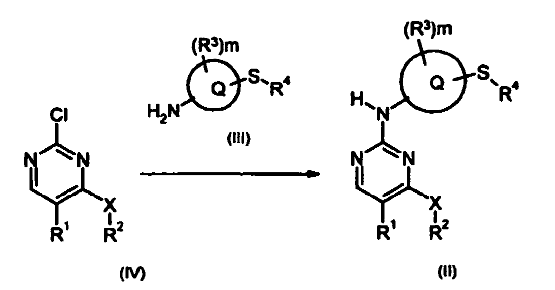
b) imination des thioéthers de formule (II) avec obtention de composés de formule (I) 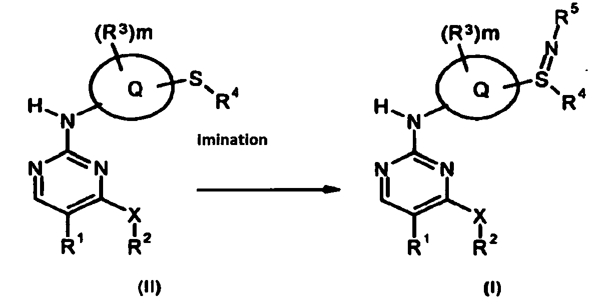
où Q, R¹, R², R³, R⁴, R⁵, X et m ont les significations indiquées dans la formule générale (I) selon les revendications 1 à 20.

21. Procédé pour la préparation d'intermédiaires de formule (IV) par transformation de 2,4-dichloropyrimidines de formule (VI) avec des nucléophiles de formule (V), 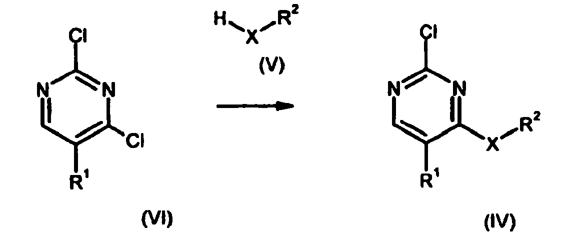 où R¹, R² et X ont les significations indiquées dans la formule générale (I) selon les revendications 1 à 19.

22. Procédé pour la préparation de composés de formule (I) selon l'une quelconque des revendications 1 à 19 par transformation de 2-chloropyrimidines de formule (IV) avec des nucléophiles de formule (VII) 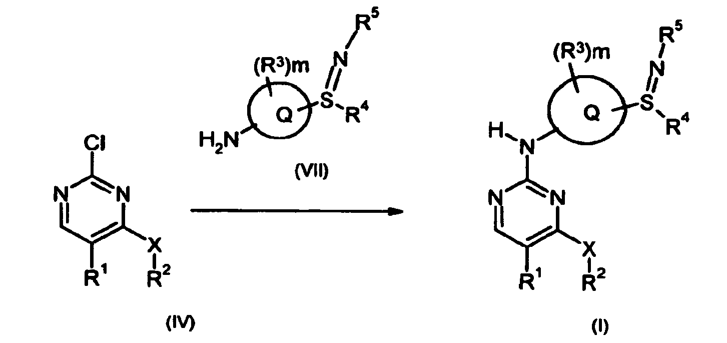 où Q, R¹, R², R³, R⁴, R⁵, X et m ont les significations indiquées dans la formule générale (I) selon les revendications 1 à 19.

23. Procédé pour la fabrication d'intermédiaires de formule (VII), comprenant les étapes de
a) imination d'un thioéther de formule (IX) avec obtention de sulfimides de formule (VIII) 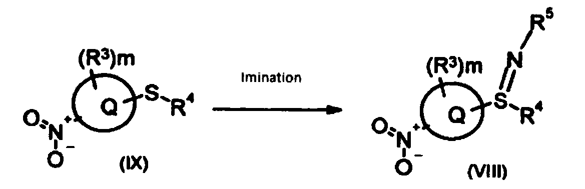
b) réduction du groupe nitro avec obtention des intermédiaires de formule (VII) 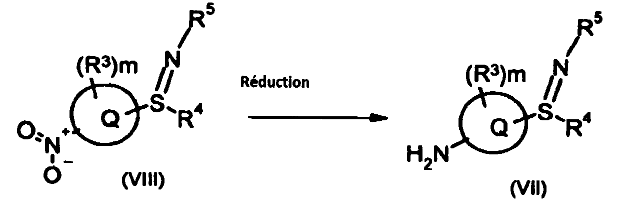
où Q, R³, R⁴ et R⁵ ont les significations indiquées dans la formule générale (I) selon les revendications 1 à 19.

24. Composés selon l'une quelconque des revendications 1 à 19 destinés à une utilisation comme médicament.

25. Utilisation d'un composé selon l'une quelconque des revendications 1 à 19 pour la préparation d'un médicament destiné au traitement du cancer.

26. Formulation pharmaceutique contenant un composé selon l'une quelconque des revendications 1 à 19.
